# EUROPEAN PATENT SPECIFICATION

(11) **EP 4 320 269 B1**
(45) Date of publication and mention of the grant of the patent: **11.11.2026**
(21) Application number: 22784117.8
(22) Date of filing: 08.04.2022
(51) Int. Cl.: C12Q 1/6827, C12Q 1/6886, C12Q 1/6883, C12Q 1/6869, C12Q 1/6809

(54) **CELL-FREE DNA METHYLATION AND NUCLEASE-MEDIATED FRAGMENTATION**
ZELLFREIE DNA-METHYLIERUNG UND NUKLEASEVERMITTELTE FRAGMENTIERUNG
MÉTHYLATION DE L'ADN ACELLULAIRE ET FRAGMENTATION MÉDIÉE PAR LES NUCLÉASES

(30) Priority: 08.04.2021 US 202163172542 P; 01.03.2022 US 202263315468 P
(43) Date of publication of application: 14.02.2024
(73) Proprietor: The Chinese University of Hong Kong, Shatin, New Territories (HK)
(72) Inventor: LO, Yuk-Ming Dennis, New Territories, Hong Kong (HK); CHIU, Rossa Wai Kwun, New Territories, Hong Kong (HK); CHAN, Kwan Chee, New Territories, Hong Kong (HK); JIANG, Peiyong, New Territories, Hong Kong (HK); CHAN, Wing Yan, New Territories, Hong Kong (HK); NI, Meng, New Territories, Hong Kong (HK); HAN, Diana Siao Cheng, New Territories, Hong Kong (HK); SIN, Tsz Kwan, New Territories, Hong Kong (HK)
(74) Representative: J A Kemp LLP
(86) International application number: PCT/CN2022/085695
(87) International publication number: WO 2022/214051

(56) References cited:
- WO-A1-2018/099418
- WO-A1-2019/006269
- WO-A1-2020/192680
- WO-A1-2021/121368
- WO-A2-2019/200404
- CN-A- 111 712 582
- US-A1- 2015 011 403
- US-A1- 2020 185 059
- REBECCA W. Y. CHAN ET AL: "Plasma DNA aberrations in systemic lupus erythematosus revealed by genomic and methylomic sequencing", PROCEEDINGS OF THE NATIONAL ACADEMY OF SCIENCES (PNAS), vol. 111, no. 49, 26 November 2014 (2014-11-26), pages E5302 - E5311, XP055495862, ISSN: 0027-8424, DOI: 10.1073/pnas.1421126111
- JOHANNES HARTL ET AL: "(PDF) Autoantibody-mediated impairment of DNASE1L3 activity in sporadic systemic lupus erythematosus", vol. 218, no. 5, 30 March 2021 (2021-03-30), US, XP093267548, ISSN: 0022-1007, Retrieved from the Internet <URL:https://www.researchgate.net/publication/349255561_Autoantibody-mediated_impairment_of_DNASE1L3_activity_in_sporadic_systemic_lupus_erythematosus#fullTextFileContent> DOI: 10.1084/jem.20201138
- HAN, D.S.C., MENG NI, REBECCA W.Y. CHAN, DANNY K.L. WONG, LINDA T. HIRAKI, STEFANO VOLPI, PEIYONG JIANG, KATHY O. LUI, K.C. ALLEN C: "Nuclease deficiencies alter plasma cell-free DNA methylation profiles", GENOME RESEARCH, vol. 31, no. 11, 1 September 2021 (2021-09-01), pages 2008 - 2021, XP055975867
- HAN DIANA S.C., NI MENG, CHAN REBECCA W.Y., CHAN VICKEN W.H., LUI KATHY O., CHIU ROSSA W.K., LO Y.M. DENNIS: "The Biology of Cell-free DNA Fragmentation and the Roles of DNASE1, DNASE1L3, and DFFB", THE AMERICAN JOURNAL OF HUMAN GENETICS, AMERICAN SOCIETY OF HUMAN GENETICS , CHICAGO , IL, US, vol. 106, no. 2, 6 February 2020 (2020-02-06), US , pages 202 - 214, XP055822533, ISSN: 0002-9297, DOI: 10.1016/j.ajhg.2020.01.008
- REBECCA W. Y. CHAN, PEIYONG JIANG, XIANLU PENG, LAI-SHAN TAM, GARY J. W. LIAO, EDMUND K. M. LI, PRISCILLA C. H. WONG, HAO SUN, K. : "Plasma DNA aberrations in systemic lupus erythematosus revealed by genomic and methylomic sequencing", PROCEEDINGS OF THE NATIONAL ACADEMY OF SCIENCES, NATIONAL ACADEMY OF SCIENCES, vol. 111, no. 49, 9 December 2014 (2014-12-09), pages E5302 - E5311, XP055495862, ISSN: 0027-8424, DOI: 10.1073/pnas.1421126111
- JIANG PEIYONG, XIE TINGTING, DING SPENCER C., ZHOU ZE, CHENG SUK HANG, CHAN REBECCA W.Y., LEE WING-SHAN, PENG WENLEI, WONG JOHN, W: "Detection and characterization of jagged ends of double-stranded DNA in plasma", GENOME RESEARCH, COLD SPRING HARBOR LABORATORY PRESS, US, vol. 30, no. 8, 14 August 2020 (2020-08-14), US , pages 1144 - 1153, XP055902244, ISSN: 1088-9051, DOI: 10.1101/gr.261396.120

## Description

### BACKGROUND

Many exciting diagnostic and prognostic applications using cell-free DNA (cfDNA) have been developed for noninvasive prenatal testing and cancer liquid biopsies (Chiu et al., Proc Ntl Acad Sci USA. 2008; 105:20458-20463; Chan et al., N Engl J Med, 2017;377:513-522). Plasma cfDNA is essentially a mixture of short DNA molecules with a modal size of 166 bp that are released from different tissues in the body, including but not limited to, hematopoietic tissues, brain, liver, lung, colon, pancreas and so on (Sun et al., Proc Natl Acad Sci USA. 2015; 112:E5503-12; Lehmann-Werman et al., Proc Natl Acad Sci USA. 2016; 113: E1826-34; Moss et al., Nat Commun. 2018; 9: 5068).

Exploiting the unique pattern of methylation in multiple cell types, cfDNA has been interrogated at differentially methylated regions to determine the tissue-of-origin of cfDNA molecules, where the increase of cfDNA from specific tissues can allow for the site of pathology to be localized (Sun et al., Proc Natl Acad Sci USA. 2015; 112:E5503-12; Guo et al., Nat Genet. 2017; 49:635-642). For example, genome-wide analysis of DNA methylation differences between cancer and normal cells has been utilized for cancer detection (Chan et al., Proc Natl Acad Sci USA. 2013; 110:18761-18768; Kang et al., Genome Bio. 2017:18).

While cfDNA methylation is a promising marker for cancer and tissue-of-origin testing, the field has only just begun to explore the biology behind the fragmentation of cfDNA. In this regard, the fragmentation of DNA into cfDNA has been found to be non-random and to reflect the underlying position of nucleosomes (Sun et al., Genome Res. 2019;29:418-427; Snyder et al., Cell. 2016; 164:57-68; Ivanov et al., BMC Genomics. 2015; 16:S1; Chandrananda et al., BMC Med Genomics. 2015;8:29). By studying the fragmentomics of cfDNA, we have previously shown that different nuclease deficiencies affect cfDNA fragment ends and size profiles (Serpas et al., Proc Natl Acad Sci USA. 2019; 116:641-649; Han et al., Am J Hum Genet. 2020; 106:202-214; Chan et al., Am J Hum Genet. 2020; 1-13). The fragmentomic profile of cfDNA has been revealed as an emerging biomarker for cancer (Jiang et al., Cancer Discov. 2020; 10:664-673).
WO 2019/200404 A2, WO 2019/006269 A1, and WO 2018/099418 A1 disclose background art to the invention.

### BRIEF SUMMARY

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes. Some embodiments of the present disclosure describes practical implementation of cfDNA methylation measurements for determining nuclease-mediated cfDNA fragmentation, which can be used for determining a level of cancer and a fractional concentration of cfDNA in a sample. Certain levels of nuclease activity may be correlated with certain levels of methylation in certain regions

As examples, methylation levels in certain genomic regions can be analyzed to classify nuclease activity. The relative abundance of fragments covering sites that are hypomethylated or hypermethylated can be used to determine a level of a condition (e.g., a disease or disorder) in a subject, a classification of nuclease activity, or a fractional concentration of clinically-relevant DNA molecules in a sample. The classification of whether a gene exhibits a genetic disorder or the efficacy of a treatment can also be determined using methylation levels at certain sites.

DNA fragments from a subject with a condition may have a greater tendency to be within certain regions (e.g., open chromatin regions). The number of copy number aberrations within these regions compared to copy number aberrations including those outside these regions can be used to determine whether a subject has a condition.

In other examples, methylation of cfDNA in a biological sample can also be used to provide information on the sample itself. Analyzing fragments from sites that are hypomethylated or hypermethylated in a reference genome can be used to estimate the fractional concentration of clinically-relevant DNA molecules in a biological sample.

In addition to using linear cfDNA, cfDNA from extrachromosomal circular DNA (eccDNA) can be used to analyze a biological sample. The size distribution of cfDNA fragments from eccDNA can be used to determine a classification of whether a gene exhibits a genetic disorder, a classification of nuclease activity, or an efficacy of treatment. A parameter value based on the amount of eccDNA in a sample can be used to determine whether a gene exhibits a genetic disorder. Embodiments described herein also include methods for determining a quantity in a mixture of cell-free DNA fragments from eccDNA.

These and other embodiments of the disclosure are described in detail below. For example, other embodiments are directed to systems, devices, and computer readable media associated with methods described herein.

A better understanding of the nature and advantages of embodiments of the present invention may be gained with reference to the following detailed description and the accompanying drawings.

### TERMS

*A "tissue"* corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (mother vs. fetus) or to healthy cells vs. tumor cells. "Reference tissues" can correspond to tissues used to determine tissue-specific methylation levels. Multiple samples of a same tissue type from different individuals may be used to determine a tissue-specific methylation level for that tissue type.

*A "biological sample"* refers to any sample that is taken from a subject (*e.g.,* a human (or other animal), such as a pregnant woman, a person with cancer or other disorder, or a person suspected of having cancer or other disorder, an organ transplant recipient or a subject suspected of having a disease process involving an organ (e.g., the heart in myocardial infarction, or the brain in stroke, or the hematopoietic system in anemia) and contains one or more nucleic acid molecule(s) of interest. The biological sample can be a bodily fluid, such as blood, plasma, serum, urine, vaginal fluid, fluid from a hydrocele (e.g. of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g. thyroid, breast), intraocular fluids (e.g. the aqueous humor), etc. Stool samples can also be used. In various embodiments, the majority of DNA in a biological sample that has been enriched for cell-free DNA (e.g., a plasma sample obtained via a centrifugation protocol) can be cell-free, e.g., greater than 50%, 60%, 70%, 80%, 90%, 95%, or 99% of the DNA can be cell-free. The centrifugation protocol can include, for example, 3,000 g x 10 minutes, obtaining the fluid part, and re-centrifuging at for example, 30,000 g for another 10 minutes to remove residual cells. As part of an analysis of a biological sample, a statistically significant number of cell-free DNA molecules can be analyzed (e.g., to provide an accurate measurement) for a biological sample. In some embodiments, at least 1,000 cell-free DNA molecules are analyzed. In other embodiments, at least 10,000 or 50,000 or 100,000 or 500,000 or 1,000,000 or 5,000,000 cell-free DNA molecules, or more, can be analyzed. At least a same number of sequence reads can be analyzed.

*A "sequence read"* refers to a string of nucleotides sequenced from any part or all of a nucleic acid molecule. For example, a sequence read may be a short string of nucleotides (e.g., 20-150 nucleotides) sequenced from a nucleic acid fragment, a short string of nucleotides at one or both ends of a nucleic acid fragment, or the sequencing of the entire nucleic acid fragment that exists in the biological sample. A sequence read may be obtained in a variety of ways, e.g., using sequencing techniques or using probes, e.g., in hybridization arrays or capture probes as may be used in microarrays, or amplification techniques, such as the polymerase chain reaction (PCR) or linear amplification using a single primer or isothermal amplification. As part of an analysis of a biological sample, at least 1,000 sequence reads can be analyzed. As other examples, at least 10,000 or 50,000 or 100,000 or 500,000 or 1,000,000 or 5,000,000 sequence reads, or more, can be analyzed. An amount of sequence reads can be used as a proxy for the number of DNA fragments. To determine the number of DNA fragments from the amount of sequence reads, a calculation may be performed to account for paired-end sequencing and/or bias of sequencing techniques.

A sequence read can include an *"ending sequence"* associated with an end of a fragment. The ending sequence can correspond to the outermost N bases of the fragment, e.g., 1-30 bases at the end of the fragment. If a sequence read corresponds to an entire fragment, then the sequence read can include two ending sequences. When paired-end sequencing provides two sequence reads that correspond to the ends of the fragments, each sequence read can include one ending sequence.

An *"ending position"* or *"end position"* (or just *"end*) can refer to the genomic coordinate or genomic identity or nucleotide identity of the outermost base, i.e. at the extremities, of a cell-free DNA molecule, e.g. plasma DNA molecule. The end position can correspond to either end of a DNA molecule. In this manner, if one refers to a start and end of a DNA molecule, both would correspond to an ending position. In practice, one end position is the genomic coordinate or the nucleotide identity of the outermost base on one extremity of a cell-free DNA molecule that is detected or determined by an analytical method, such as but not limited to massively parallel sequencing or next-generation sequencing, single molecule sequencing, double- or single-stranded DNA sequencing library preparation protocols, polymerase chain reaction (PCR), or microarray. Such *in vitro* techniques may alter the true *in vivo* physical end(s) of the cell-free DNA molecules. Thus, each detectable end may represent the biologically true end or the end is one or more nucleotides inwards or one or more nucleotides extended from the original end of the molecule e.g. 5' blunting and 3' filling of overhangs of non-blunt-ended double stranded DNA molecules by the Klenow fragment. The genomic identity or genomic coordinate of the end position could be derived from results of alignment of sequence reads to a human reference genome, e.g. hg19. It could be derived from a catalog of indices or codes that represent the original coordinates of the human genome. It could refer to a position or nucleotide identity on a cell-free DNA molecule that is read by but not limited to target-specific probes, mini-sequencing, DNA amplification.

*A "preferred end"* (or *"recurrent ending position*") refers to an end that is more highly represented or prevalent (e.g., as measured by a rate) in a biological sample having a physiological (e.g. pregnancy) or pathological (disease) state (e.g. cancer) than a biological sample not having such a state or than at different time points or stages of the same pathological or physiological state, e.g., before or after treatment. A preferred end therefore has an increased likelihood or probability for being detected in the relevant physiological or pathological state relative to other states. The increased probability can be compared between the pathological state and a non-pathological state, for example in patients with and without a cancer and quantified as likelihood ratio or relative probability. The likelihood ratio can be determined based on the probability of detecting at least a threshold number of preferred ends in the tested sample or based on the probability of detecting the preferred ends in patients with such a condition than patients without such a condition. Examples for the thresholds of likelihood ratios include but not limited to 1.1, 1.2, 1.3, 1.4, 1.5, 1.6, 1.8, 2.0, 2.5, 3.0, 3.5, 4.0, 4.5, 5, 6, 8, 10, 20, 40, 60, 80 and 100. Such likelihood ratios can be measured by comparing relative abundance values of samples with and without the relevant state. Because the probability of detecting a preferred end in a relevant physiological or disease state is higher, such preferred ending positions would be seen in more than one individual with that same physiological or disease state. With the increased probability, more than one cell-free DNA molecule can be detected as ending on a same preferred ending position, even when the number of cell-free DNA molecules analyzed is far less than the size of the genome. Thus, the preferred or recurrent ending positions are also referred to as the "frequent ending positions." In some embodiments, a quantitative threshold may be used to require that ends be detected at least multiple times (e.g., 3, 4, 5, 6, 7, 8, 9, 10, 15, 20, or 50) within the same sample or same sample aliquot to be considered as a preferred end. A relevant physiological state may include a state when a person is healthy, disease-free, or free from a disease of interest. Similarly, a *"preferred ending window"* corresponds to a contiguous set of preferred ending positions.

A *"rate"* of DNA molecules ending on a position relates to how frequently a DNA molecule ends on the position. Such a rate can be referred to as an *"end density."* The rate may be based on a number of DNA molecules that end on the position normalized against a number of DNA molecules analyzed. The normalization can also be based on the average, median, or total number of ends in the surrounding region. The surrounding region used for normalization may include, but is not limited to, 500, 1000, 3000, 5000, etc. bp upstream and/or downstream of the position.

The term *"alleles"* refers to alternative DNA sequences at the same physical genomic locus, which may or may not result in different phenotypic traits. In any particular diploid organism, with two copies of each chromosome (except the sex chromosomes in a male human subject), the genotype for each gene comprises the pair of alleles present at that locus, which are the same in homozygotes and different in heterozygotes. A population or species of organisms typically include multiple alleles at each locus among various individuals. A genomic locus where more than one allele is found in the population is termed a polymorphic site. Allelic variation at a locus is measurable as the number of alleles (i.e., the degree of polymorphism) present, or the proportion of heterozygotes (i.e., the heterozygosity rate) in the population. As used herein, the term *"polymorphism"* refers to any inter-individual variation in the human genome, regardless of its frequency. Examples of such variations include, but are not limited to, single nucleotide polymorphism, simple tandem repeat polymorphisms, insertion-deletion polymorphisms, mutations (which may be disease causing) and copy number variations. The term *"haplotype"* as used herein refers to a combination of alleles at multiple loci that are transmitted together on the same chromosome or chromosomal region. A haplotype may refer to as few as one pair of loci or to a chromosomal region, or to an entire chromosome or chromosome arm.

*A "relative frequency"* (also referred to just as "frequency") may refer to a proportion (e.g., a percentage, fraction, or concentration). In particular, a relative frequency of a particular end motif (e.g., CCGA or just a single base) can provide a proportion of cell-free DNA fragments in a sample that are associated with the end motif CCGA, e.g., by having an ending sequence of CCGA.

An *"aggregate value"* may refer to a collective property, e.g., of relative frequencies of a set of ending positions. Examples include a mean, a median, a sum of relative frequencies, a variation among the relative frequencies (e.g., standard deviation (SD), the coefficient of variation (CV), interquartile range (IQR) or a certain percentile cutoff (e.g. 95^{th} or 99^{th} percentile) among different relative frequencies), or a difference (e.g., a distance) from a reference pattern of relative frequencies, as may be implemented in clustering.

*A "calibration sample"* can correspond to a biological sample whose desired measured value (e.g., nuclease activity, classification of a genetic disorder, or other desired property) is known or determined via a calibration method, e.g., using other measurement techniques such as clotting measurements for effective dosage or ELISA for measuring nuclease quantity or assays quantifying the rate of DNA digestion by nucleases for measuring nuclease activity (an example method can involve fluorometric or spectrophotometric measurement of DNA quantity before and after, or in real-time, the addition of a nuclease-containing sample; another example is using radial enzyme diffusion methods). A calibration sample can have separate measured values (e.g., an amount of fragments with a particular end motif or with a particular size) can be determined to which the desired measure value can be correlated.

*A "calibration data point"* includes a *"calibration value"* (e.g., an amount of fragments with a particular end motif or with a particular size) and a measured or known value that is desired to be determined for other test samples. The calibration value can be determined from various types of data measured from DNA molecules of the sample, (e.g., an amount of fragments with an end motif or with a particular size). The calibration value corresponds to a parameter that correlates to the desired property, e.g., classification of a genetic disorder, nuclease activity, or efficacy of anticoagulant dosage. For example, a calibration value can be determined from measured values as determined for a calibration sample, for which the desired property is known. The calibration data points may be defined in a variety of ways, e.g., as discrete points or as a calibration function (also called a calibration curve or calibration surface). The calibration function could be derived from additional mathematical transformation of the calibration data points.

*A "site"* (also called a *"genomic site*") corresponds to a single site, which may be a single base position or a group of correlated base positions, e.g., a CpG site, TSS site, Dnase hypersensitivity site, or larger group of correlated base positions. A "locus" may correspond to a region that includes multiple sites. A locus can include just one site, which would make the locus equivalent to a site in that context.

*A "separation value"* corresponds to a difference or a ratio involving two values, e.g., two fractional contributions or two methylation levels. The separation value could be a simple difference or ratio. As examples, a direct ratio of x/y is a separation value, as well as x/(x+y). The separation value can include other factors, e.g., multiplicative factors. As other examples, a difference or ratio of functions of the values can be used, e.g., a difference or ratio of the natural logarithms (ln) of the two values. A separation value can include a difference and a ratio.

*A "separation value"* and an *"aggregate value"* (e.g., of relative frequencies) are two examples of a parameter (also called a metric) that provides a measure of a sample that varies between different classifications (states), and thus can be used to determine different classifications. An aggregate value can be a separation value, e.g., when a difference is taken between a set of relative frequencies of a sample and a reference set of relative frequencies, as may be done in clustering.

*A "relative abundance"* is a type of separation value that relates an amount (one value) of cell-free DNA molecules ending within one window of genomic position to an amount (other value) of cell-free DNA molecules ending within another window of genomic positions. The two windows may overlap, but would be of different sizes. In other implementations, the two windows would not overlap. Further, the windows may be of a width of one nucleotide, and therefore be equivalent to one genomic position. An end density is a type of relative abundance.

The term *"classification"* as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1).

The terms *"cutoff"* and *"threshold"* refer to predetermined numbers used in an operation. For example, a cutoff size can refer to a size above which fragments are excluded. A threshold value may be a value above or below which a particular classification applies. Either of these terms can be used in either of these contexts. A cutoff or threshold may be "a reference value" or derived from a reference value that is representative of a particular classification or discriminates between two or more classifications. Such a reference value can be determined in various ways, as will be appreciated by the skilled person. For example, metrics can be determined for two different cohorts of subjects with different known classifications, and a reference value can be selected as representative of one classification (e.g., a mean) or a value that is between two clusters of the metrics (e.g., chosen to obtain a desired sensitivity and specificity). As another example, a reference value can be determined based on statistical simulations of samples. A particular value for a cutoff, threshold, reference, etc. can be determined based on a desired accuracy (e.g., a sensitivity and specificity).

*A "level of pathology"* (or level of a disorder) can refer to the amount, degree, or severity of pathology associated with an organism. An example is a cellular disorder in expressing a nuclease. Another example of pathology is a rejection of a transplanted organ. Other example pathologies can include autoimmune attack (e.g., lupus nephritis damaging the kidney or multiple sclerosis), inflammatory diseases (e.g., hepatitis), fibrotic processes (e.g. cirrhosis), fatty infiltration (e.g. fatty liver diseases), degenerative processes (e.g. Alzheimer's disease) and ischemic tissue damage (e.g., myocardial infarction or stroke). A heathy state of a subject can be considered a classification of no pathology. The pathology can be cancer.

The term *"level of cancer"* can refer to whether cancer exists (i.e., presence or absence), a stage of a cancer, a size of tumor, whether there is metastasis, the total tumor burden of the body, the cancer's response to treatment, and/or other measure of a severity of a cancer (e.g. recurrence of cancer). The level of cancer may be a number or other indicia, such as symbols, alphabet letters, and colors. The level may be zero. The level of cancer may also include premalignant or precancerous conditions (states). The level of cancer can be used in various ways. For example, screening can check if cancer is present in someone who is not previously known to have cancer. Assessment can investigate someone who has been diagnosed with cancer to monitor the progress of cancer over time, study the effectiveness of therapies or to determine the prognosis. In one embodiment, the prognosis can be expressed as the chance of a patient dying of cancer, or the chance of the cancer progressing after a specific duration or time, or the chance or extent of cancer metastasizing. Detection can mean 'screening' or can mean checking if someone, with suggestive features of cancer (e.g. symptoms or other positive tests), has cancer.

The *"methylation index"* or *"methylation status"* for each genomic site (e.g., a CpG site) can refer to the proportion of DNA fragments (e.g., as determined from sequence reads or probes) showing methylation at the site over the total number of reads covering that site. A "read" can correspond to information (e.g., methylation status at a site) obtained from a DNA fragment. A read can be obtained using reagents (e.g. primers or probes) that preferentially hybridize to DNA fragments of a particular methylation status. Typically, such reagents are applied after treatment with a process that differentially modifies or differentially recognizes DNA molecules depending of their methylation status, e.g. bisulfite conversion, or methylation-sensitive restriction enzyme, or methylation binding proteins, or anti-methylcytosine antibodies, or single molecule sequencing techniques that recognize methylcytosines and hydroxymethylcytosines.

The *"methylation density"* of a region can refer to the number of reads at sites within the region showing methylation divided by the total number of reads covering the sites in the region. The sites may have specific characteristics, e.g., being CpG sites. Thus, the "CpG methylation density" of a region can refer to the number of reads showing CpG methylation divided by the total number of reads covering CpG sites in the region (e.g., a particular CpG site, CpG sites within a CpG island, or a larger region). For example, the methylation density for each 100-kb bin in the human genome can be determined from the total number of cytosines not converted after bisulfite treatment (which corresponds to methylated cytosine) at CpG sites as a proportion of all CpG sites covered by sequence reads mapped to the 100-kb region. This analysis can also be performed for other bin sizes, e.g. 500 bp, 5 kb, 10 kb, 50-kb or 1-Mb, etc. A region could be the entire genome or a chromosome or part of a chromosome (e.g. a chromosomal arm). The methylation index of a CpG site is the same as the methylation density for a region when the region only includes that CpG site. The "proportion of methylated cytosines" can refer the number of cytosine sites, "C's", that are shown to be methylated (for example unconverted after bisulfite conversion) over the total number of analyzed cytosine residues, i.e. including cytosines outside of the CpG context, in the region. The methylation index, methylation density and proportion of methylated cytosines are examples of "methylation levels." Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by the Pacific Biosciences single molecule real time analysis (Tse et al. Proc Natl Acad Sci U S A 2021; 118: e2019768118)

The term *"hypomethylation"* can refer to a site or set of sites (e.g., a region) that has below a specified value for a methylation level, e.g., at or below 80%, 75%, 70%, 65%, or 60% for the methylation level. The term *"hypermethylation"* can refer to a site or set of sites (e.g., a region) that has above a specified value for a methylation level, e.g., at or above 80%, 75%, 70%, 65%, or 60% for the methylation level.

The name of a gene is typically written in italics. A human gene is typically also written in all capital letters. A mouse gene may not be capitalized after the first letter. The protein is conventionally written in all capital letters and without italics. As examples, a mouse may have the *Dnase1l3* gene and the DNASE1L3 protein, while a human may have the *DNASE1L3* gene and the DNASE1L3 protein.

The term "about" or "approximately" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term "about" or "approximately" can mean within an order of magnitude, within 5-fold, and more preferably within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to ±10%. The term "about" can refer to ±5%.

### BRIEF DESCRIPTION OF THE DRAWINGS

**FIG. 1** shows that cfDNA from *Dnasell3-deficient* mice is hypomethylated and cfDNA from *Dnase1*-deficient mice is hypermethylated on a genome-wide level according to embodiments of the present invention. The overall CpG methylation percentage was calculated from all sequenced fragments in plasma cfDNA and buffy coat genomic DNA of each sample. Wilcoxon signed-rank test was performed on paired plasma and buffy coat samples. Wilcoxon rank-sum test was performed between wild-type and *Dnase1l3-*deficient samples.

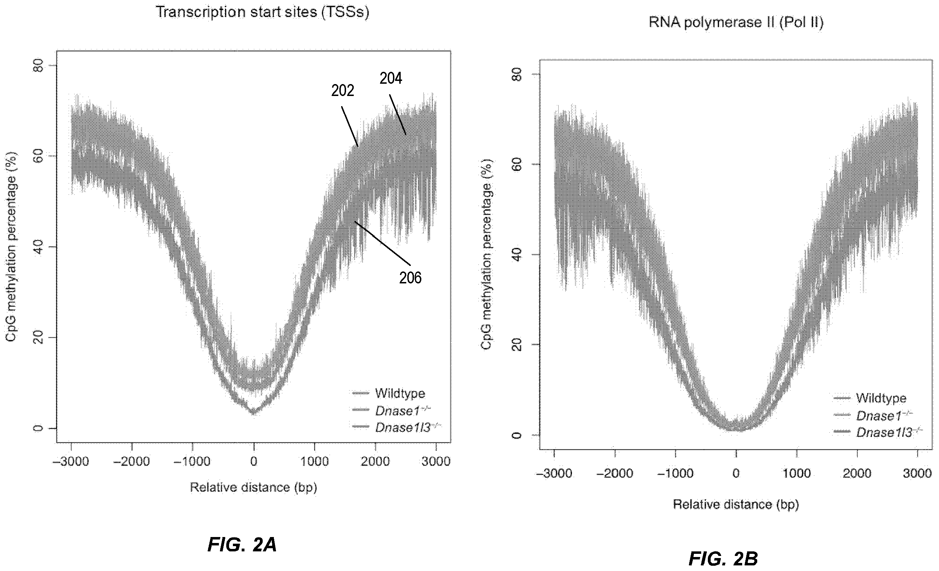

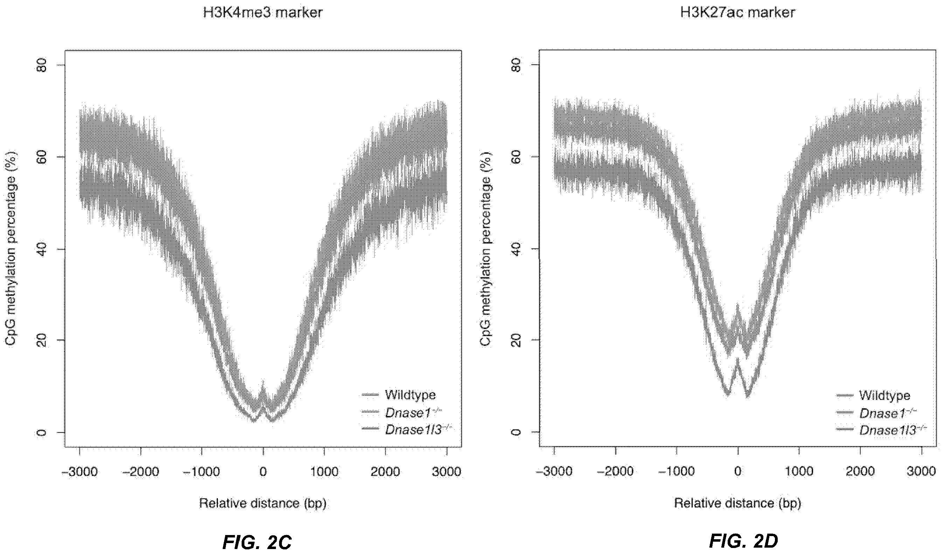

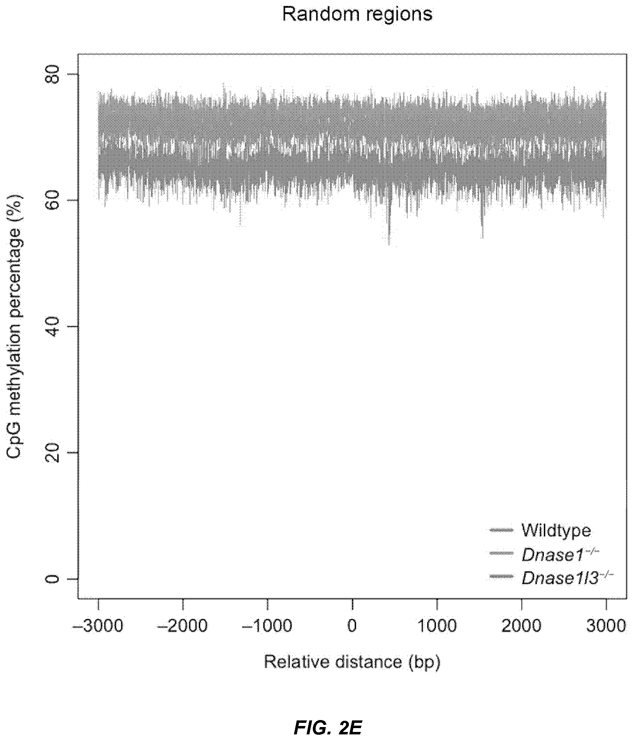

**FIGS. 2A, 2B****,** **2C, 2D****,** and **2E** show the median CpG methylation percentage relative to certain sites according to embodiments of the present invention. The median CpG methylation percentage of cfDNA from each genotype in transcription start sites (FIG. 2A), RNA polymerase II sites (FIG. 2B), H3K4me3 marker regions (FIG. 2C), H3K27ac marker regions (FIG. 2D), and random regions (FIG. 2E) are shown. The CpG methylation percentage of all fragments in each sample was calculated over each of these aggregated regions and the median of each genotype is shown in a ± 3000 bp window. cfDNA from WT mice is in green, *Dnase1l3*-deficient mice is in red, and *Dnase1*-deficient mice in blue.
**FIG. 3** shows cfDNA size profile of WT, *Dnasell3-deficient* mice, and *Dnase1-*deficient mice. The median cfDNA size profile of each genotype was plotted using all sample fragments. cfDNA from WT mice is in green, *Dnase1l3*-deficient mice is in red, and *Dnase1-*deficient mice in blue.
**FIGS. 4A****,** **4B****,** and **4C** show the size profile of unmethylated and methylated fragments according to embodiments of the present invention. The size profile of 0% methylated fragments (orange) and 100% methylated fragments (purple) are compared within cfDNA of wildtype (FIG. 4A), *Dnase1l3*-deficient (FIG. 4B), and *Dnase1*-deficient mice (FIG. 4C).
**FIGS. 5A** **and** **5B** show thesize profile of cfDNA from different genotypes using only 0% methylated fragments (FIG. 5A), or only 100% methylated fragments (FIG. 5B) according to embodiments of the present invention.
**FIGS. 6A, 6B****,** **6C, 6D****,** **6E,** and **6F** show the normalized end density at relative distances from various sites for different genotypes according to embodiments of the present invention.
**FIG. 7** shows the size profile of fragments in OCRs and CpG islands (CGIs) according to embodiments of the present invention. The median cfDNA size profile of fragments inside OCRs and CGIs is shown. cfDNA from wildtype mice is in green, *Dnase1l3*-deficient mice is in red, and *Dnase1-*deficient mice in blue. The cfDNA size profile of all wildtype fragments is shown as a comparison in gray.
**FIG. 8** shows the proportion of fragments in OCRs and CGI regions for different genotypes according to embodiments of the present invention. cfDNA from *Dnase1l3*-deficient mice had significantly increased proportion of OCR and CGI fragments. Wilcoxon rank-sum test was performed between wildtype and *Dnase1l3*-deficient mouse samples.
**FIG. 9** shows CpG methylation percentages after OCR and CGI fragments were bioinformatically excluded in masking analysis according to embodiments of the present invention. The CpG methylation percentage increased after these fragments were masked. The relative hypomethylation of cfDNA from *Dnase1l3*-deficient mice is substantially diminished after masking, but remains significantly different from the cfDNA methylation of wildtype mice. Wilcoxon rank-sum test was performed between wildtype and *Dnase1l3*-deficient samples.
**FIGS. 10A-10C** are circos plots showing genome-wide CpG methylation percentages before (outer ring) and after (inner ring) masking OCR and CGI fragments according to embodiments of the present invention. Each dot represents the CpG methylation percentage in a 1 Mb bin of the mouse autosome and colored in blue if ≥ 70% and in red if < 70%. Circos plots are shown for wildtype (FIG. 10A), *Dnase1l3*-deficient (FIG. 10B), and *Dnase1-*deficient mice (FIG. 10C).
**FIG. 11** is a graph of the median CpG methylation percentage for all fragments of a particular size for different genotypes according to embodiments of the present invention.
**FIG. 12** is a graph of the proportion of OCR and CGI fragments within each fragment size for different genotypes according to embodiments of the present invention.
**FIG. 13** is a graph of the CpG methylation percentage for each fragment size after masking OCR and CGI fragments according to embodiments of the present invention.
**FIGS. 14A, 14B****,** and **14C** show CpG methylation percentage with each fragment size before and after masking OCR and CGI fragments in wildtype (FIG. 14A), *Dnase1l3*-deficient (FIG. 14B), and *Dnase1-*deficient mice (FIG. 14C) according to embodiments of the present invention.
**FIGS. 15A** and **15B** show plasma cfDNA methylation percentage of the putatively unmethylated CpG (FIG. 15A) and putatively methylated CpG (FIG. 15B) in wildtype, *Dnase1l3-*deficient, and *Dnase1*-deficient mice according to embodiments of the present invention.

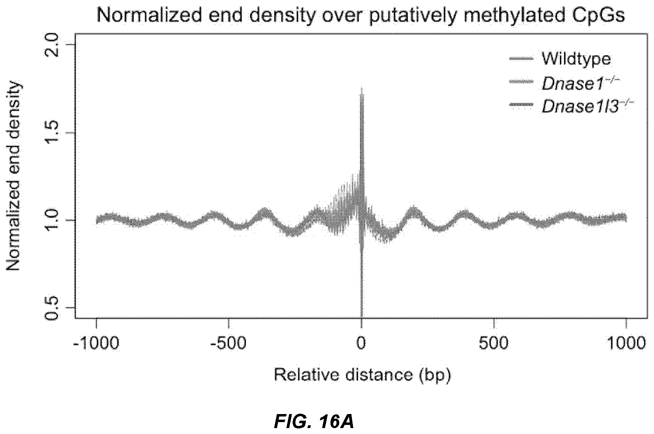

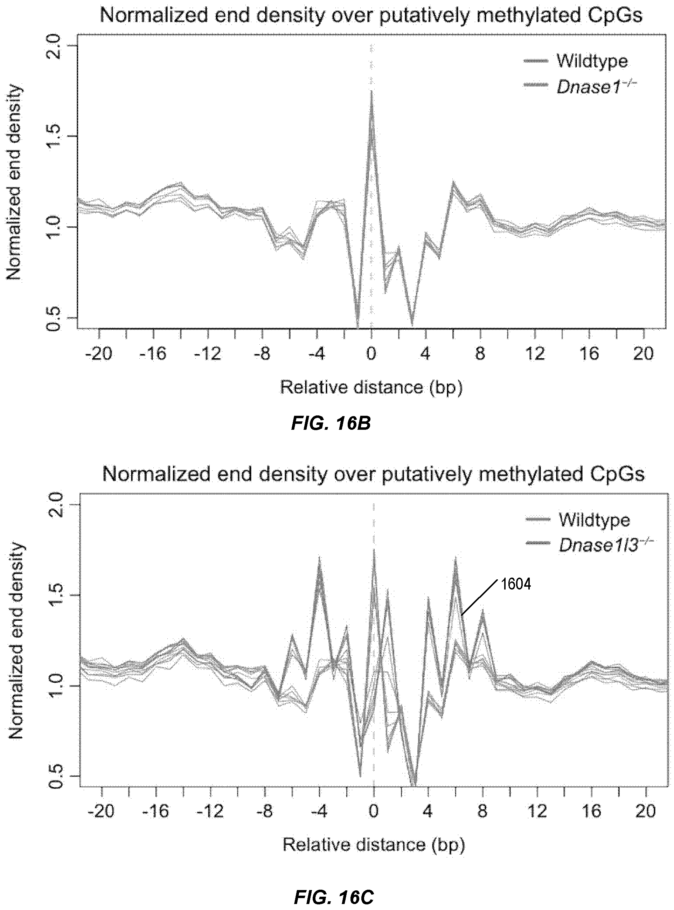

**FIGS. 16A****,** **16B,** and **16C** show normalized end densities over putatively methylated CpGs according to embodiments of the present invention. Putatively methylated CpGs were identified, and the plasma cfDNA end density over these CpGs were normalized by the median end counts of the ± 1000 bp region. A ± 1000 bp window (FIG. 16A) and a ± 20 bp window (FIG. 16B, FIG. 16C) is shown. The identified C is placed at position 0. Comparisons between the normalized end density of all wildtype samples (green) and all *Dnase1*-deficient mice samples (blue) is shown (FIG. 16B). Comparisons between the normalized end density of all wildtype samples (green) and all *Dnase1l3*-deficient mice samples (red) is shown (FIG. 16C).

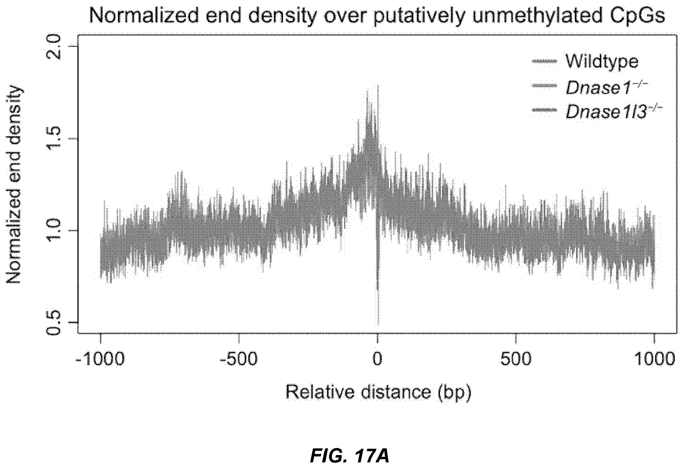

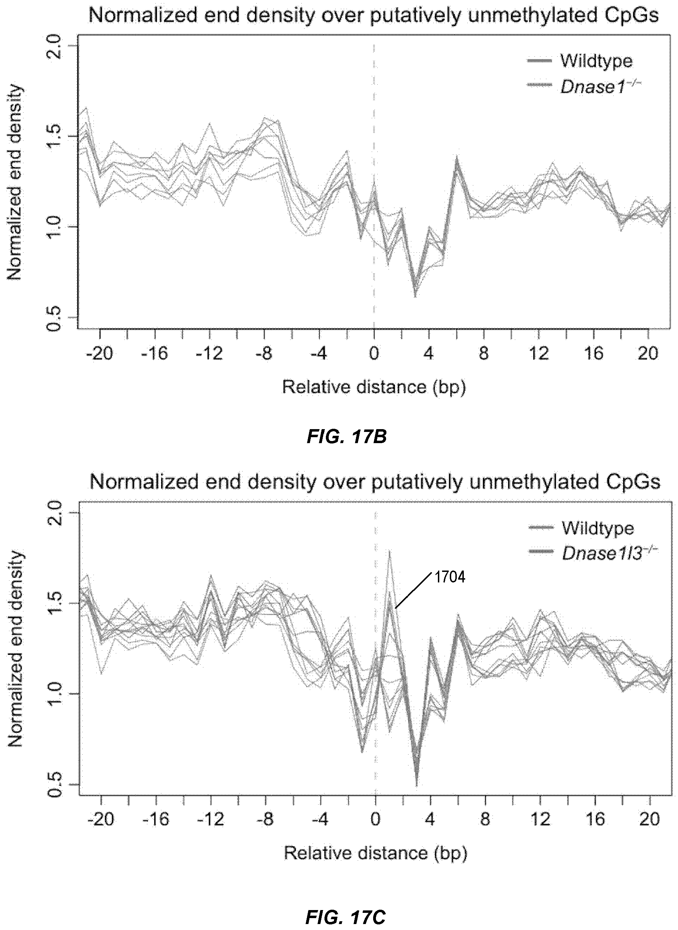

**FIGS. 17A****,** **17B**, and **17C** show normalized end densities over putatively unmethylated CpGs according to embodiments of the present invention. Putatively unmethylated CpGs were identified, and the plasma cfDNA end density over these CpGs were normalized by the median end counts of the ± 1000 bp region. A ± 1000 bp window (FIG. 17A) and a ± 20 bp window (FIG. 17B, 17C) is shown. The identified C is placed at position 0. Comparisons between the normalized end density of all wildtype samples (green) and all *Dnase1*-deficient mice samples (blue) is shown (FIG. 17B). Comparisons between the normalized end density of all wildtype samples (green) and all *Dnase1l3*-deficient mice samples (red) is shown (FIG. 17C).
**FIG. 18** shows CpG methylation percentage of the human plasma (orange) and buffy coat (purple) samples are shown according to embodiments of the present invention. H2, H4, and V11 have the homozygous frameshift c.290_291delCA (p.Thr97Ilefs*2) mutation, and H1 is the heterozygous parent of H2 and H4. The median value of 8 control samples is shown.
**FIGS. 19A** and **19B** show the CpG methylation percentage of fragments from each sample calculated over the aggregated TSS regions (FIG. 19A) and random regions (FIG. 19B). The median of each sample type is shown in a ± 3000 bp window.
**FIGS. 20A** and **20B** show the median cfDNA size profile of each subject type plotted using only 0% methylated fragments (FIG. 20A) or only 100% methylated fragments (FIG. 20B) according to embodiments of the present invention.
**FIGS. 21A** and **21B** show the median normalized end density for each sample type in a ± 1000 bp window over the aggregated TSS regions (FIG. 21A) and random regions (FIG. 21B).
**FIG. 22** shows the proportion of fragments in OCR and CGI regions according to embodiments of the present invention. OCRs are defined as the regions ± 500 bp around the center of TSS, PoI II, H3K4me3, and H3K27ac regions. cfDNA from *DNASE1L3-*deficient subjects had significantly increased proportion of OCR and CGI fragments. Wilcoxon rank-sum test was performed between control and *DNASE1L3*-deficient subjects.
**FIGS. 23A****,** **23B****,** and **23C** shows Circos plots from normal (FIG. 23A) and *DNASE1L3*-deficient patients (FIGS. 23B and 23C) showing genome-wide CpG methylation percentages before (outer ring) and after (inner ring) masking OCR and CGI fragments according to embodiments of the present invention. Each dot represents the CpG methylation percentage in a 1 Mb bin of the mouse autosome and colored in blue if ≥ 70% and in red if < 70%. Red dots are closer to the center than blue dots.
**FIG. 24** shows normalized end density over putatively methylated CpGs in a ± 20 bp window according to embodiments of the present invention. The identified C is placed at position 0. cfDNA from control samples is in light green, the heterozygous *DNASE1L3* parent is in dark green, and *DNASE1L3*-deficient subjects is in red.
**FIG. 25** illustrates deduced activities of DNASE1 and DNASE1L3 according to embodiments of the present invention.
**FIG. 26** shows cfDNA CpG methylation from control individuals (CTR), chronic hepatitis B carriers (HBV), and patients with hepatocellular carcinoma (HCC) according to embodiments of the present invention. The overall CpG methylation percentage was calculated from all bisulfite sequenced fragments in plasma cfDNA of each sample. cfDNA from patients with HCC is relatively hypomethylated on a genome-wide level, but the difference is not statistically significant using the Wilcoxon rank-sum test (P value = 0.14).

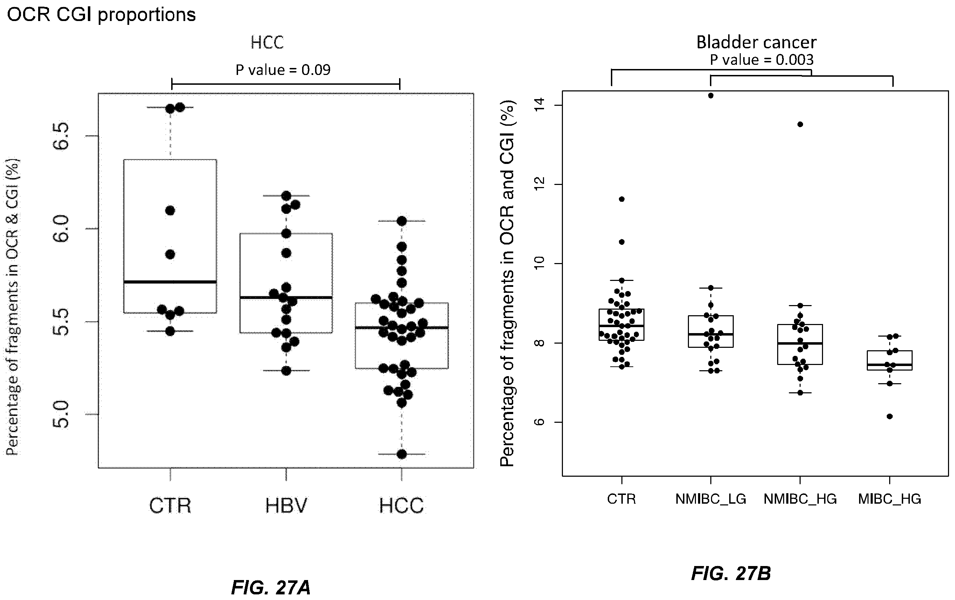

**FIGS. 27A** and **27B** show percentage of fragments in OCR and CGI regions according to embodiments of the present invention. The regions ± 500 bp around the center of TSS, H3K4me3, and H3K27ac regions were merged with CGI regions. FIG. 27A shows the proportion of fragments in these OCRs and CGI regions from control individuals (CTR), chronic hepatitis B carriers (HBV), and patients with hepatocellular carcinoma (HCC) are shown in the figure on the left. cfDNA from HCC patients had significantly decreased proportion of OCR and CGI fragments from controls (P value = 0.009, Wilcoxon rank-sum test). FIG. 27B shows the proportion of fragments in these OCRs and CGI regions from control individuals (CTR), patients with low-grade non-muscle-invasive bladder cancer (NMIBC_LG), high-grade non-muscle-invasive bladder cancer (NIMBC_HG), and high-grade muscle-invasive bladder cancer (MIBC_HG). cfDNA from patients with all three types of bladder cancer had significantly decreased proportions of OCR and CGI fragments from controls (P value =0.003 Wilcoxon rank-sum test).
**FIG. 28** shows percentage of fragments in OCR and CGI regions according to embodiments of the present invention. The regions ± 500 bp around the center of TSS, H3K4me3, and H3K27ac regions were merged with CGI regions. The proportions of fetal-specific and maternal-specific fragments in these OCRs and CGI regions are shown. There are significantly fewer fetal-specific fragments in these OCR and CGI regions than maternal-specific fragments (P value = 9.2 E-06, Wilcoxon rank-sum test).
**FIG. 29** is a table showing another set of open chromatin regions that includes transcription start sites (TSS), CCCTC-binding factor (CTCF) sites, DNase1 hypersensitivity sites (DNaseI), and H3K27ac, H3K4me3, and H3K4me1 histone markers.

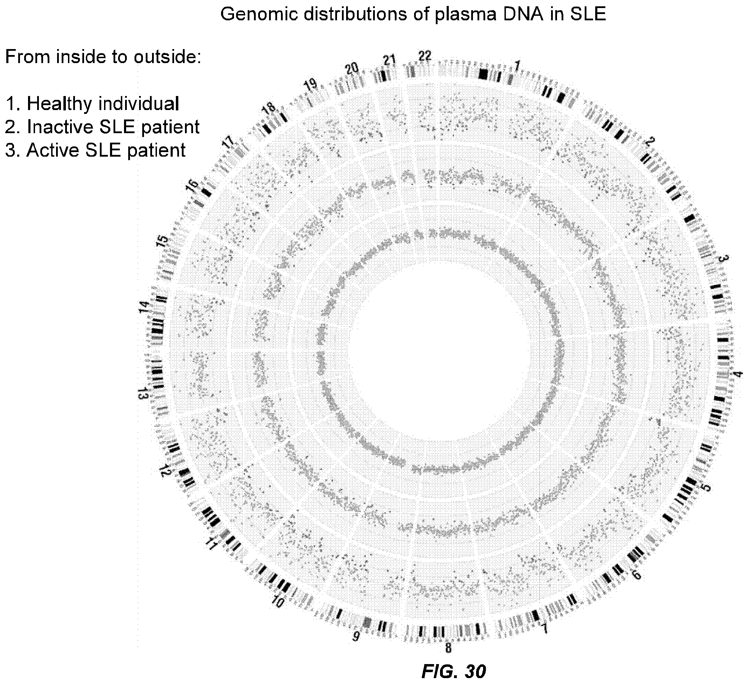

**FIG. 30** is a circos plot showing the genomic representation in 1 Mb bins across the whole genome in a healthy individual (inner ring), inactive SLE patient (middle ring), and an active SLE patient (outer ring) according to embodiments of the present invention. Each dot represents the genomic representation of 1 Mb bin, and colored in red if -3 SD from the mean genomic representation in the group of healthy controls, and colored in green if +3 SD from the mean genomic representation in the group of healthy controls. Active SLE patients have cfDNA with widely divergent genomic distributions.
**FIG. 31** is a schematic illustration of the measured genomic representation (MGR) calculation according to embodiments of the present invention.
**FIG. 32** is a genomic representation after bioinformatically removing open chromatin regions according to embodiments of the present invention. Open chromatin regions were removed in silico and measured genomic representations (MGR) were calculated as illustrated in FIG. 31.
**FIG. 33** is a circo plot of MGR in a patient (S112) with active SLE before in silico removal of the open chromatin regions (inner ring) and after in silico removal of the open chromatin regions (outer ring) according to embodiments of the present invention. As illustrated, the copy number aberrations are normalized due to the in silico removal of the open chromatin regions.
**FIG. 34** is a circo plot of MGR in an HCC patient before in silico removal of the open chromatin regions (inner ring) and after in silico removal of the open chromatin regions (outer ring) according to embodiments of the present invention. There is no substantial change before and after masking the OCR regions in the example HCC case.

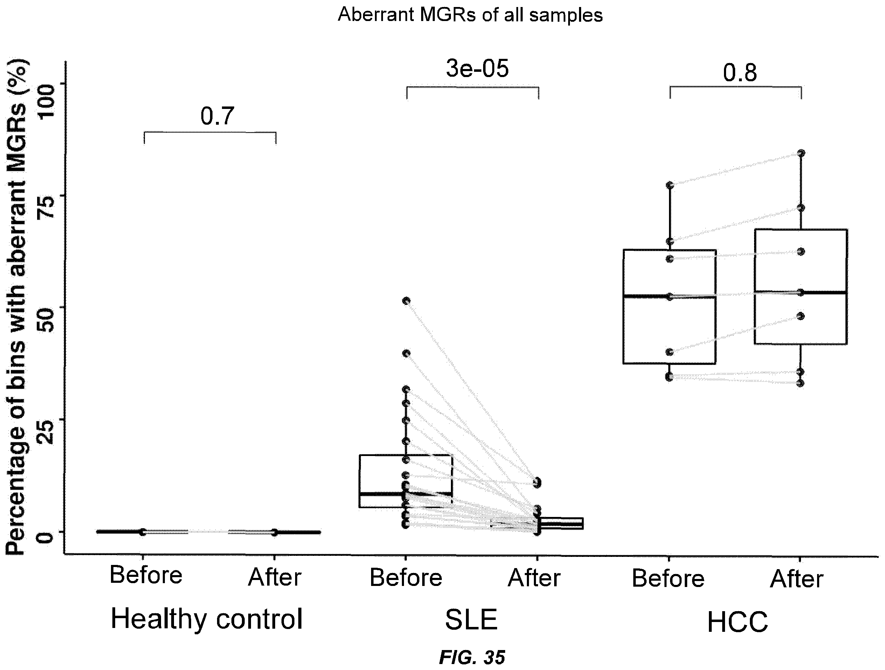

**FIG. 35** is a boxplot representation of MGRs in samples from an SLE patient, an HCC patient, and a healthy individual before and after in silico removal of open chromatin regions according to embodiments of the present invention. The percentage of bins with aberrant MGRs (more than +3 SD or less than -3 SD) is similar before and after bioinformatically masking the OCRs in healthy controls and patients with HCC. On the other hand, it is significantly different in SLE patients after masking the OCRs. Wilcoxon rank-sum testing was used. These results indicate that masking open chromatin regions in the analysis of MGR aberrations may be used to reduce false positive results.

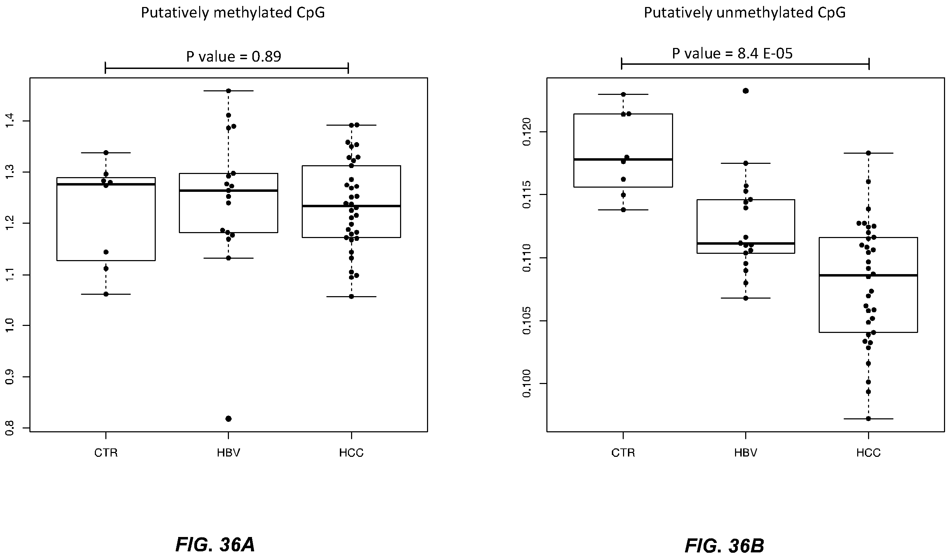

**FIGS. 36A** and **36B** show the percentage of fragments overlapping with at least 1 base of the putatively methylated CpG (FIG. 36A) or the putatively unmethylated CpG (FIG. 36B) in control individuals (CTR), chronic hepatitis B carriers (HBV), and patients with hepatocellular carcinoma (HCC) according to embodiments of the present invention. The coverage of the putatively methylated CpG is not significantly different in CTR vs HCC (P value 0.89, Wilcoxon rank sum test), while the coverage of the putatively unmethylated CpG is significantly lower in HCC versus CTR (P value 8.4 E-05, Wilcoxon rank sum test). Putatively methylated CpG sites (putatively hyper methylated or putatively hypomethylated CPG sites) were distinguished by analysing a downloaded dataset of tissue samples to identify CpG sites that were consistently methylated or unmethylated, e.g., those that had a methylation level above (hypermethylated) or below (hypomethylated) a specified value.

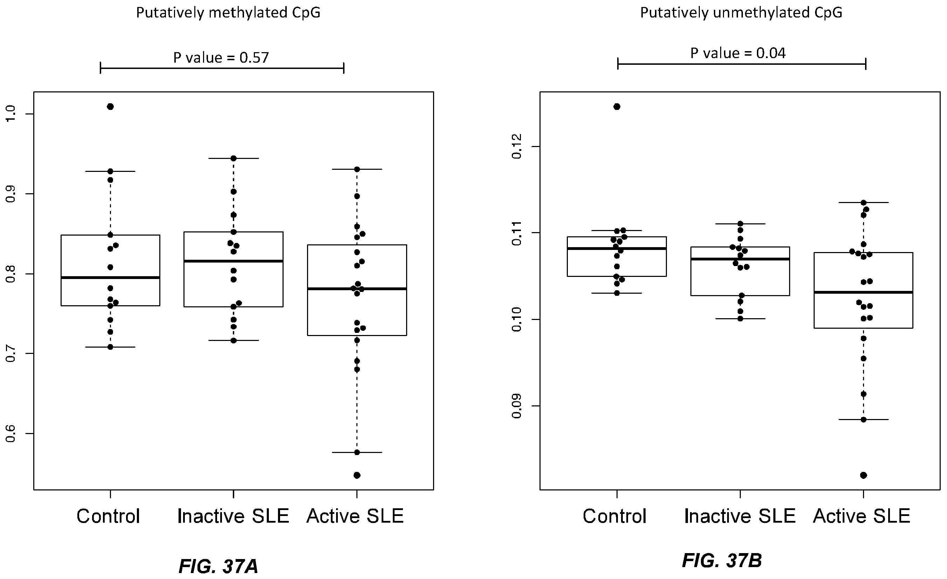

**FIGS. 37A** and **37B** show the percentage of fragments overlapping with at least 1 base of the putatively methylated CpG (FIG. 37A) or the putatively unmethylated CpG (FIG. 37B) in control individuals (Control), patients with inactive SLE, and patients with active SLE according to embodiments of the present invention. The coverage of the putatively methylated CpG is not significantly different in Control versus active SLE (P value 0.57, Wilcoxon rank sum test), while the coverage of the putatively unmethylated CpG is significantly lower in active SLE vs Control (P value 0.04, Wilcoxon rank sum test).

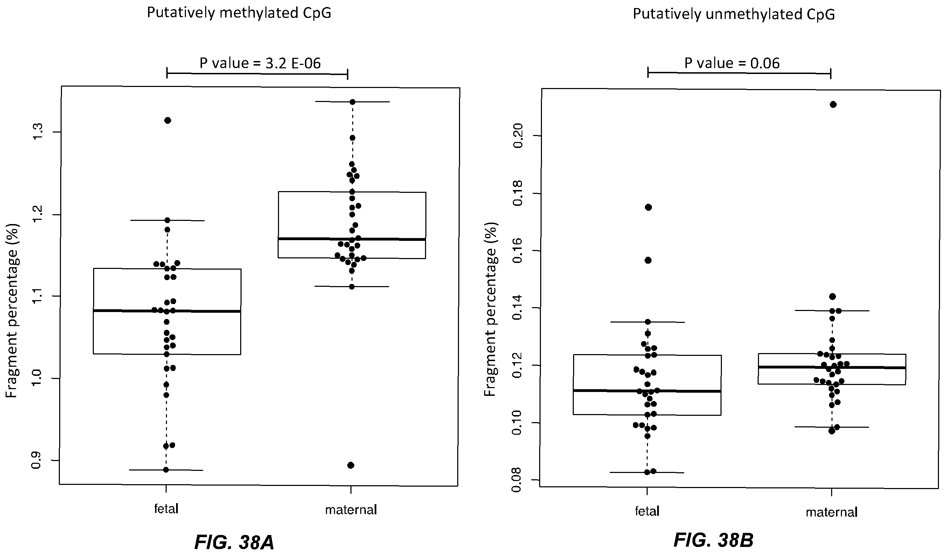

**FIGS. 38A** and **38B** show the percentage of fragments overlapping with at least 1 base of the putatively methylated CpG (FIG. 38A) or the putatively unmethylated CpG (FIG. 38B) in fetal-specific fragments and maternal-specific fragments according to embodiments of the present invention. The coverage of the putatively methylated CpG is significantly lower in fetal-specific fragments compared with maternal-specific fragments (P value 3.2 E-06, Wilcoxon rank sum test). The coverage of the putatively unmethylated CpG is not significantly lower in in fetal-specific fragments compared with maternal-specific fragments (P value 0.06, Wilcoxon rank sum test).
**FIG. 39** is a flowchart illustrating a method for detecting a genetic disorder for a gene associated with a nuclease using a biological sample of a subject including cell-free DNA according to embodiments of the present disclosure.
**FIG. 40** is a flowchart illustrating a method for determining an efficacy of a treatment of a subject having blood disorder according to embodiments of the present disclosure.
**FIG. 41** is a flowchart illustrating a method for monitoring an activity of a nuclease using a biological sample of a subject including cell-free DNA according to embodiments of the present disclosure.
**FIG. 42** is a flowchart illustrating a method for analyzing a biological sample of a subject including cell-free DNA according to embodiments of the present disclosure.
**FIG. 43** is a flowchart illustrating a method for monitoring activity of a nuclease using a biological sample of a subject including cell-free DNA according to embodiments of the present disclosure.
**FIG. 44** is a flowchart illustrating a method for estimating a fractional concentration of clinically-relevant DNA molecules in a biological sample of a subject according to embodiments of the present disclosure.
**FIG. 45** is a flowchart illustrating a method for analyzing a biological sample of a subject including cell-free DNA according to embodiments of the present disclosure.
**FIG. 46** illustrates an experimental design to study effects of nucleases on plasma extrachromosomal circular DNA (eccDNA), according to embodiments of the present invention.
**FIG. 47** shows eccDNA counts distribution in wild type mice and mice lacking DNASE1 or DNASE1L3, according to embodiments of the present invention.
**FIGS. 48A-48C** show eccDNA size frequencies in three groups of mice, according to embodiments of the present invention.
**FIG. 48D** shows area under the curve (AUC) ratios corresponding to the eccDNA size frequencies in the three groups of mice, according to embodiments of the present invention.
**FIG. 48E** illustrates an example for determining an AUC value for a first peak clusters and a second peak cluster in a eccDNA size frequency graph.
**FIG. 48F** shows AUC value for the first peak cluster and the second peak cluster corresponding to FIGS. 48A-48C for each group of mice, according to embodiments of the present invention.
**FIGS. 49A-49C** show size distribution eccDNA obtained from liver in three groups of mice using tagmentation, according to embodiments of the present invention.
**FIGS. 49D-49F** show size distribution of eccDNA obtained from buffy coat in three groups of mice using tagmentation, according to embodiments of the present invention.
**FIG. 49G** shows AUC ratio of the two peaks in eccDNA size distribution of the three mice groups corresponding to FIGS. 49A-49C, according to embodiments of the present invention.
**FIG. 49H** shows AUC ratio of the two peaks in eccDNA size distribution of the three mice groups corresponding to FIGS. 49D-49F, according to embodiments of the present invention.
**FIGS. 50A-50C** show size distribution eccDNA obtained from liver in three groups of mice using rolling circle amplification (RCA), according to embodiments of the present invention.
**FIGS. 50D-50F** show size distribution of eccDNA obtained from buffy coat in three groups of mice using RCA, according to embodiments of the present invention.
**FIG. 50G** shows AUC ratio of the two peaks in eccDNA size distribution of the three mice groups corresponding to FIGS. 49A-49C, according to embodiments of the present invention.
**FIG. 50H** shows AUC ratio of the two peaks in eccDNA size distribution of the three mice groups corresponding to FIGS. 49D-49F, according to embodiments of the present invention.
**FIG. 51** shows information of mouse pregnancy samples and their respective eccDNA fetal fractions in maternal plasma, according to embodiments of the present invention.
**FIGS. 52A-52C** shows the mean size distributions of total plasma eccDNA in the three groups of pregnant mice, according to embodiments of the present invention.
**FIG. 52D** shows the mean size distributions of the maternal eccDNA, according to embodiments of the present invention.
**FIG. 52E** shows the mean size distributions of the fetal eccDNA, according to embodiments of the present invention.
**FIG. 53A** shows the size profile of eccDNA pooled from plasma samples collected from 4 healthy human subjects, according to embodiments of the present invention.
**FIG. 53B** shows the size profile of eccDNA pooled from 4 plasma samples collected from 3 human subjects with loss-of-function mutations of *DNASE1L3,* according to embodiments of the present invention.
**FIG. 53C** shows the comparison of the AUC ratios between healthy controls and *DNASE1L3-*mutated subjects (shown in FIGS. 53A and 53B), according to embodiments of the present invention.
**FIGS. 54A-54D** show the size profile of eccDNA plasma samples collected from 4 healthy human subjects separately, according to embodiments of the present invention.
**FIGS. 54E** and **54F** shows the size profile of eccDNA in plasma samples collected from the first human subject with loss-of-function mutations of *DNASE1L3* pre and post hemodialysis, according to embodiments of the present invention.
**FIGS. 54G** and **54H** show the size profile of eccDNA in plasma samples collected from two different human subject with loss-of-function mutations of *DNASE1L3,* according to embodiments of the present invention.
**FIG. 55** is a flowchart illustrating a method for using size of eccDNA to detect a genetic disorder for a gene associated with a nuclease using a biological sample of a subject including cell-free eccDNA, according to embodiments of the present invention.
**FIG. 56** illustrates two different approaches for sample preparation and tissue eccDNA identification, according to embodiments of the present invention.
**FIG. 57** is a flowchart illustrating a method for determining an efficacy of a treatment of a subject having blood disorder according to embodiments of the present invention.
**FIG. 58** is a flowchart illustrating a method for monitoring an activity of a nuclease using a biological sample of a subject including eccDNA, according to embodiments of the present invention.
**FIG. 59** is a flowchart illustrating a method for using an amount of eccDNA to detect a genetic disorder for a gene associated with a nuclease using a biological sample of a subject including cell-free DNA, according to embodiments of the present invention.
**FIG. 60** is a flowchart illustrating a method for analyzing a biological sample to quantify the amount of eccDNA according to embodiments of the present disclosure.
**FIG. 61** shows an example technique for eccDNA identification according to embodiments of the present disclosure.
**FIGS. 62A** and **62B** show a schematic approach for junction searching approach according to embodiments of the present disclosure.
**FIG. 63** is a table showing fragment counts obtained from sequencing according to embodiments of the present invention.
**FIG. 64** Are graphs of the plasma cfDNA coverage of each sample over the deleted regions according to embodiments of the present invention.
**FIG. 65** illustrates a measurement system according to an embodiment of the present invention.
**FIG. 66** shows a block diagram of an example computer system usable with systems and methods according to embodiments of the present invention.

### DETAILED DESCRIPTION

Cell-free DNA (cfDNA) is a powerful, non-invasive biomarker for cancer and prenatal testing and circulates in plasma (as well as other cell-free samples) as short fragments. Cell-free DNA includes both linear DNA and extrachromosomal circular DNA (eccDNA). eccDNA in plasma includes DNA from a subset of mitochondrial genomes of certain tissues. However, the cfDNA has not been used to understand nuclease activity in individuals. Different nuclease activities can indicate different levels of disease and different tissue types. Additionally, the effect of nuclease activity on DNA fragmentation and methylation had not previously been accounted for in analyzing cfDNA. In this disclosure, we apply relationships between nuclease activity, cfDNA methylation, and size profile in analyzing biological samples.

Different nuclease deficiencies may affect the apparent methylation level of plasma cfDNA on a genome-wide level. DNASE1L3 and DNASE1 in mice were studied as examples of nucleases. Different nuclease activity affected hypomethylation/hypermethation levels of fragments in certain genomic regions, for example, transcriptional start sites (TSSs). The relative abundance of fragments covering sites that are hypomethylated or hypermethylated can be used to determine a level of a condition (e.g., a disease or disorder) in a subject, a classification of nuclease activity, or a fractional concentration of clinically-relevant DNA molecules in a sample. The relative abundance may be determined based on the number of fragments at certain sites compared to the number of fragments at other sites. For example, more fragments from CpG sites that are hypomethylated may indicate that a condition exists.

A greater number of fragments from certain regions may indicate a level of a condition. Samples from a subject having a condition may have more fragments in certain regions or higher or lower methylation in certain regions. The regions may include open chromatin regions (OCRs), CpG islands (CGIs), or near TSSs. A number of copy number aberrations within certain regions may be used to determine whether a subject has a condition.

The fractional concentration of clinically-relevant DNA can be determined by analyzing fragments from sites that are hypomethylated or hypermethylated. For example, fetal DNA have fewer fragments from methylated CpG sites or from OCRs and CGIs.

Different sizes of cfDNA may be associated with different methylation levels depending on nuclease activity. Certain size fragments may be relatively hypermethylated, while other size fragments may be relatively hypomethylated. Thus, different genomic regions may not be represented evenly in the different sizes of cfDNA with different nuclease activity conditions.

eccDNA can be used to analyzed a biological sample. The size distribution of cfDNA fragments from eccDNA can be used to determine a classification of whether a gene exhibits a genetic disorder, a classification of nuclease activity, or an efficacy of treatment. Certain nuclease deficiencies may result in more longer cfDNA fragments. A parameter value based on the amount of eccDNA in a sample can be used to determine whether a gene exhibits a genetic disorder. Embodiments described herein also include methods for determining a quantity in a mixture of cell-free DNA fragments from eccDNA.

### I. NUCLEASE EFFECT ON CELL-FREE DNA METHYLATION

The effect of nucleases on cell-free DNA methylation is described. Cell-free DNA from organisms with nuclease deficiencies were analyzed. Changes in methylation and size profile were observed, including at certain genomic sites or genomic regions. Samples from mice and humans deficient in certain nucleases were studied. Results from example nucleases can be applied to other nucleases based on the cleaving and other characteristics of the other nucleases. Size profile, methylation amount, normalized end density were seen to vary based on nuclease deficiency, including at or near certain genomic sites.

### A. Experimental design and results

In an example analysis, we performed whole genome bisulfite sequencing of pooled plasma cfDNA and buffy coat genomic DNA from mice deficient in either DNASE1L3 or DNASE1 and their wildtype counterparts to compare their cfDNA profiles (including size and methylation profiles). Similar results were found with samples from human subjects.

In the analysis of cfDNA from nuclease-deficient subjects, the overall CpG methylation percentage of each sample was determined. The method used to determine CpG methylation in this analysis was bisulfite sequencing. Other methods may involve direct electrochemical detection, single-molecule real-time detection, methylated DNA immunoprecipitation, microarray analysis, methylation-specific PCR, or matrix-assisted laser desorption ionization time-of-flight mass spectroscopy.

In bisulfite sequencing, bisulfite is used to convert cytosine into uracil, leaving methylated cytosines (Cs) intact. Subsequent PCR amplification of the modified DNA using methylation-specific and non-specific primer pairs replaces all uracil nucleotides with thymine (T). This produces methylation-specific single-nucleotide alterations that can be identified with sequencing and alignment against a reference sequence. The methylation percentage of a given cytosine in the reference genome is calculated by the sequenced number of C/(C+T) at that given cytosine. The overall methylation of a sample can be calculated using the sequenced portion of all fragments (i.e., read 1 and read 2) and determining the counts of Cs and Ts at each reference C. The methylation percentage may be limited to the Cs in CpG dinucleotides or may be a C followed by any other nucleotides (CHs, where H may be either adenosine, thymine, or cytosine).

Bisulfite sequencing can return a methylation status for each genomic site. The methylation status can be used to determine a methylation density of a region. A site or a region can be determined to be hypomethylated or hypermethylated based on the methylation density. The methylation analysis can be used along with fragment size analysis to determine characteristics of the sample or the subject having a nuclease deficiency.

### 1. Changes in plasma methylation for nuclease-deficient mice

The overall methylation percentage of CpG sites was studied across different genotypes for nuclease deficiency and for different sample types. Additionally, CpG methylation percentage was measured for different genomic regions.

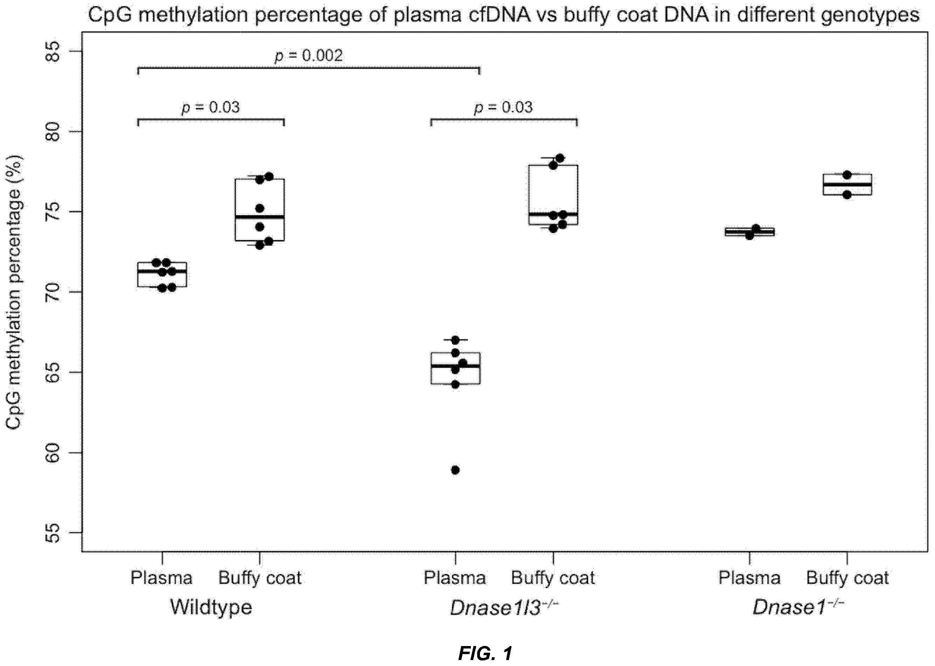

**FIG. 1** shows the overall CpG methylation percentage calculated from all reads in each sample. The X-axis shows the genotype (wildtype [WT], *Dnase1l3*^{*-*/}*⁻, Dnase1*^{*-*/}*⁻)* of the mice and the sample type (plasma, buffy coat). The Y-axis shows the percentage of CpG sites that are methylated. Among all genotypes, the CpG methylation percentage of plasma cfDNA was lower than that of their corresponding genomic DNA extracted from the buffy coat (WT plasma median percentage: 71.3% vs. WT buffy coat median percentage: 74.7%, Wilcoxon signed-rank test, p = 0.03; *Dnase1l3*-deficient plasma median percentage: 65.4% vs. *Dnase1l3*-deficient buffy coat median percentage: 74.8%, Wilcoxon signed-rank test, p = 0.03; *Dnasel-deficient* plasma median percentage: 73.8% vs. *Dnasel-deficient* buffy coat median percentage: 76.7%).

Comparing between the nuclease genotypes, plasma cfDNA from *Dnase1l3*-deficient mice was strikingly more hypomethylated than plasma cfDNA from WT mice (Wilcoxon rank-sum test, p = 0.002). On the other hand, plasma cfDNA from *Dnase1*-deficient mice was relatively hypermethylated. In contrast to the differing methylation levels in plasma cfDNA, the CpG methylation percentages of genomic DNA between WT, *Dnase1l3*-deficient mice, and *Dnase1-*deficient mice were not appreciably different from each other. Altogether, these data suggested that while the methylation levels of DNA inside the buffy coat cells of different genotypes were largely unaffected by DNASE1L3 or DNASE1 deficiency, the apparent methylation of plasma cfDNA was affected by the absence of either one of these nucleases.

**FIG. 2A** shows the median CpG methylation percentage of cfDNA from each genotype in transcriptional start sites (TSSs). The X-axis shows the relative distance in base pairs from a transcriptional start site (TSS). The Y-axis shows the median CpG methylation percentage. The different color lines show results for different genotypes. Blue line 202 shows *Dnase1*^{*-*/*-*}genotype results. Green line 204 shows wildtype genotype results. Redline 206 shows *Dnase1l3*^{*-*/-} genotype results. Blue line 202 is generally higher than green line 204, which is generally higher than red line 206. The order of the colored lines is the same for all of FIGS. 2A-2E. The TSSs of all genes were downloaded from UCSC. The TSS regions were aggregated with the nucleotide marking the TSS placed at position 0. The CpG methylation percentage is lowest for all genotypes at the center of these regions. The cfDNA of *Dnase1l3*-deficient mice is hypomethylated compared to cfDNA of wildtype at all relative distances in these regions while the cfDNA of *Dnase1*-deficient mice is slightly hypermethylated.

**FIG. 2B** shows the median CpG methylation percentage of cfDNA from each genotype around RNA polymerase II binding sites (Pol II). The Pol II binding site of all genes were downloaded from the Mouse ENCODE project. The Pol II regions were aggregated with the center of these regions placed at position 0. The CpG methylation percentage is lowest for all genotypes at the center of these regions. The cfDNA of *Dnase1l3*-deficient mice is hypomethylated compared to cfDNA of wildtype at all relative distances in these regions while the cfDNA of *Dnase1*-deficient mice is slightly hypermethylated.

**FIGS. 2C-2D** shows the median CpG methylation percentage of cfDNA from each genotype around H3K4me3 and H3K27ac regions. H3K4me3 and H3K27ac are modifications that are markers for active enhancers and active promoters, respectively. The H3K4me3 and H3K27ac regions were downloaded from the Mouse ENCODE project. The H3K4me3 and H3K27ac regions were aggregated with the center of these regions placed at position 0. The CpG methylation percentage is lowest for all genotypes at the center of these regions. The cfDNA of *Dnase1l3-*deficient mice is hypomethylated compared to cfDNA of wildtype at all relative distances in these regions while the cfDNA of *Dnase1-*deficient mice is slightly hypermethylated.

**FIG. 2E** shows the median CpG methylation percentage of cfDNA from each genotype around random regions in the genome. 10,000 random non-overlapping regions of 10,000 bp length were randomly selected across the whole genome by BEDTools (v2.27.1) (Quinlan et al., Bioinformatic. 2010;26:841-842). The apparent hypomethylation of plasma cfDNA from *Dnase1l3-deficient* mice and the apparent slight hypermethylation of plasma cfDNA from *Dnase1-deficient* mice were present in the random regions. As random regions would reflect the whole genome that is >97% heterochromatin, the apparent hypomethylation of cfDNA from *Dnase1l3*-deficient mice and apparent hypermethylation of cfDNA from *Dnase1*-deficient mice appeared to be independent of open or closed chromatin states and to affect the whole genome.

FIGS. 2A-2E all show that *Dnase1l3*-deficient mice are hypomethylated around certain sites compared to wildtype and that *Dnase1-*deficient mice are hypermethylated around certain sites compared to wildtype. These figures showed that different nuclease deficiencies can result in different methylation levels.

### 2. Effects of nuclease deficiency and methylation on the cfDNA size profile

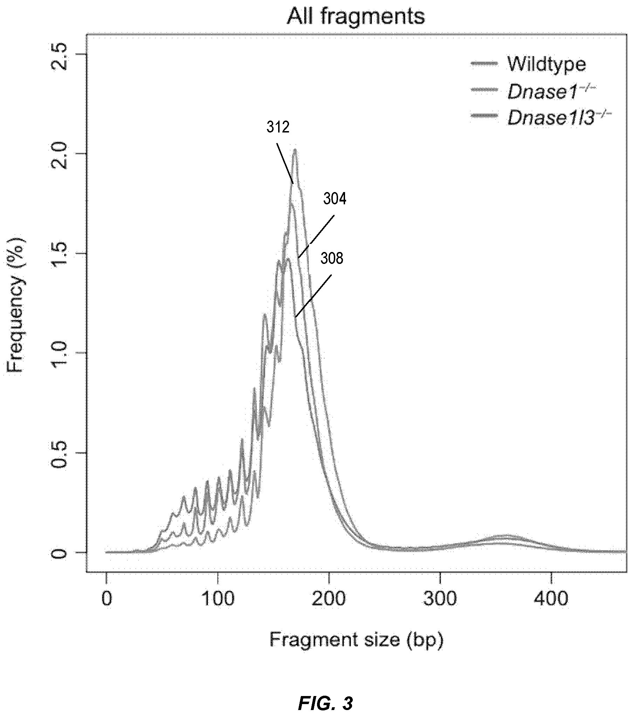

The effect of these different nucleases on the plasma cfDNA size profile has previously been characterized, and the median size profile of each genotype is shown in **FIG. 3** (Serpas et al., 2019; Cheng et al., 2018). FIG. 3 shows the fragment size in base pairs on the X-axis, and the frequency of the fragment size on the Y-axis. In comparison to cfDNA from WT mice (green line 304) with a modal size of 167 bp, cfDNA from *Dnase1l3*-deficient mice (red line 308) exhibited an increase in short ≤ 150 bp fragments, a modal size of 164 bp with a decrease in 166 bp fragments, and a slight increase in fragments ≥ 250 bp, consistent with our previous findings (Serpas et al., 2019). On the other hand, there was a subtler difference in size profiles when comparing cfDNA from *Dnase1*-deficient mice (blue line 312) to cfDNA from WT mice (green). There was a slight reduction in the short ≤ 150 bp fragments, an increase in the frequency of 166 bp fragments, and a modal size of 171 bp. Previously, we had reported that the size profile of cfDNA from *Dnase1*-deficient mice did not obviously differ from the size profile of cfDNA from WT mice (Cheng et al., 2018). While the difference had not been obvious, in retrospect, the subtle differences in the size profiles that we noted here with the benefit of more samples were also present then (Cheng et al., 2018). Thus, compared to WT mice, *Dnase1l3*-deficient mice had a shorter cfDNA size profile and *Dnase1*-deficient mice had a slightly longer cfDNA size profile.

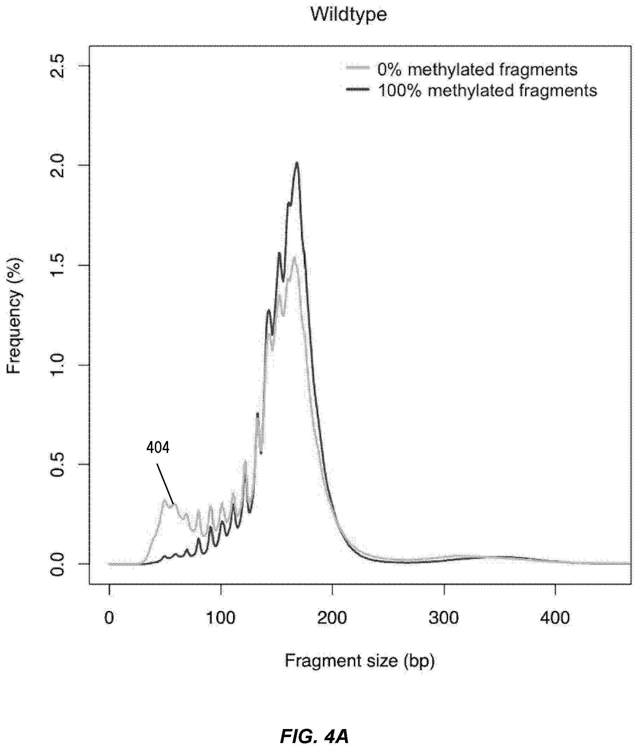

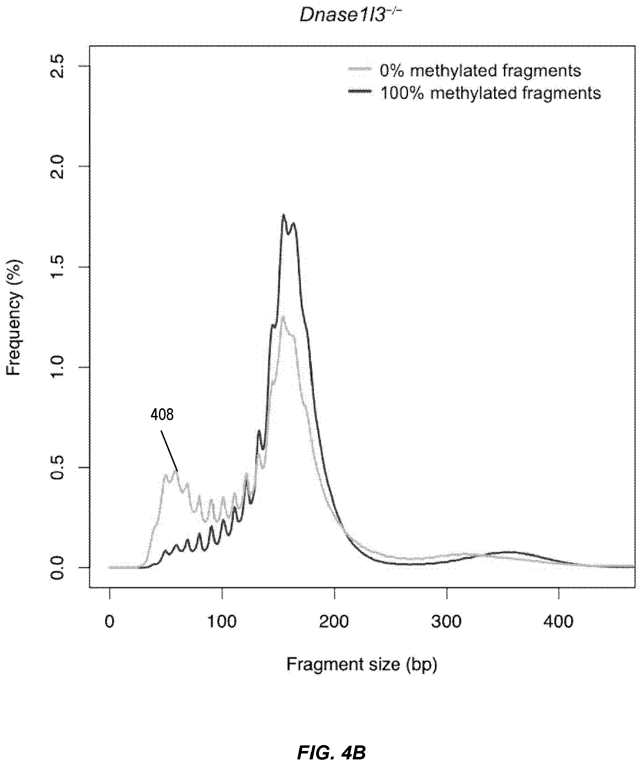

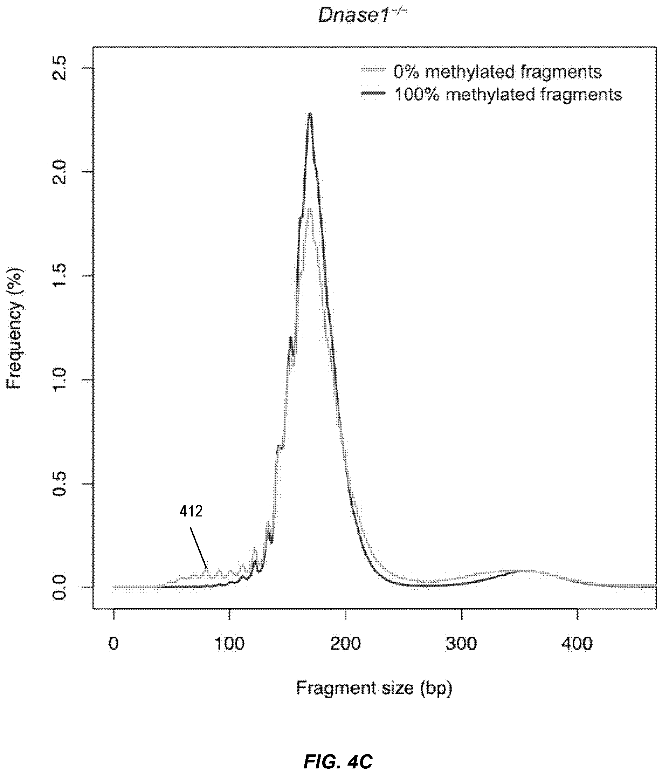

Previously, our group also found that hypomethylated cfDNA was shorter than hypermethylated cfDNA in human plasma (Lun et al., 2013). We checked to see if this relationship was still true in the plasma of mice with different nuclease genotypes. We identified cfDNA fragments with at least three CpGs and categorized the fragments with zero out of these CpGs being methylated as 0% methylated fragments and the fragments with all of its CpGs being methylated as 100% methylated fragments. We compared the median size profiles of these 0% methylated fragments and 100% methylated fragments in each of the three genotypes (**FIGS. 4A****,** **4B****,** and **4C**). In all three genotypes, the 0% methylated fragments (yellow lines 404, 408, and 412) had a size profile that had more short fragments than their 100% methylated counterparts. Thus, similar to our previous findings in human plasma, irrespective of the nuclease-related genotype, unmethylated fragments were more likely to be shorter than methylated fragments.

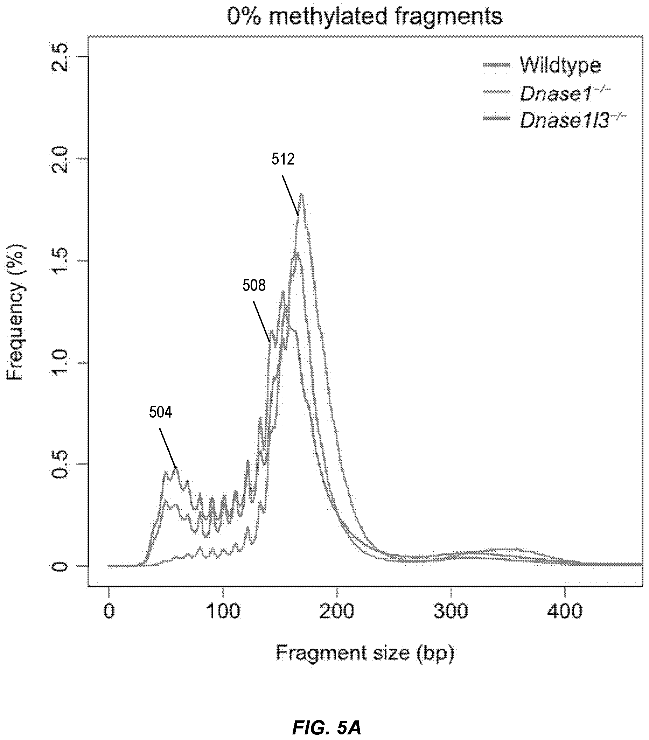

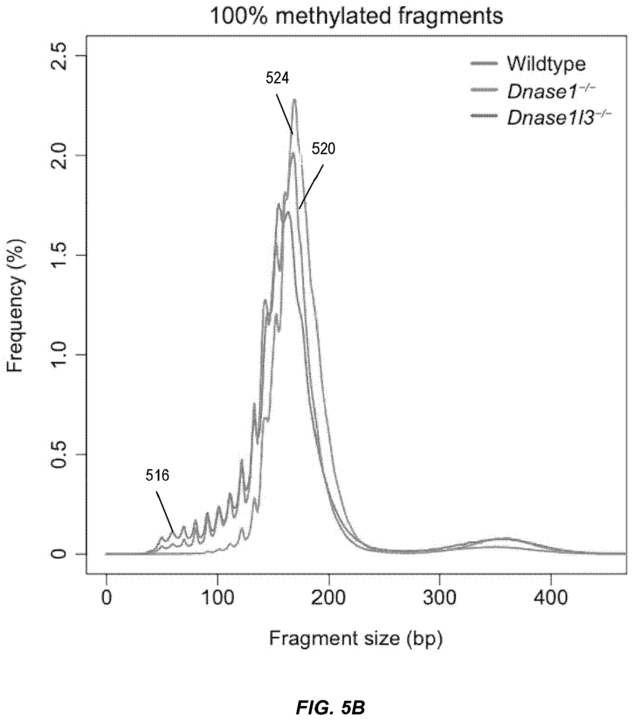

Knowing that unmethylated fragments tended to be shorter and that cfDNA from *Dnase1l3-*deficient mice had more short fragments raised the possibility that cfDNA from *Dnase1l3-*deficient mice was more hypomethylated solely because of the increase in short fragments. To tease out the relationship between these interrelated factors, we examined the median cfDNA size profile of each genotype within the 0% and the 100% methylated fragments to control for the methylation level **(****FIGS. 5A****,** **5B****).** In cfDNA from *Dnase1l3*-deficient mice, the shortening of the size profile that we previously observed using all fragments (FIG. 3) was also apparent in the 0% (red line 504, FIG. 5A) and in the 100% (red line 516, FIG. 5B) methylated fragments (modal size of 0% methylated fragments: 166 bp in WT vs. 155 bp in *Dnase1l3*-deficient mice; modal size of 100% methylated fragments: 168 bp in WT vs. 155 bp in *Dnase1l3*-deficient mice). Since both 0% and 100% methylated fragments from *Dnase1l3-*deficient mice were relatively shorter than those from WT mice, DNASE1L3 deficiency itself had an effect on the size profile. Moreover, the relative shortening of the cfDNA size profile from *Dnase1l3-*deficient mice was even more exaggerated in 0% methylated fragments than in 100% methylated fragments, particularly in fragments ≤ 80 bp. These ultrashort ≤ 80 bp fragments had a higher frequency in 0% methylated fragments than in 100% methylated fragments in both WT (green lines 508 and 520) and *Dnase1l3*-deficient mice (red lines 504 and 516). Thus, in both WT and *Dnase1l3*-deficient mice, the degree of cfDNA fragment size shortening was also affected by the methylation status of the fragments.

We then explored the median cfDNA size profile changes in 0% and 100% fragments from *Dnasel-deficient* mice (blue lines 512 and 524, FIGS. 5A, 5B). The slightly longer size profile that we saw previously with all fragments (FIG. 3) was apparent in the 0% (blue line 512, FIG. 5A) and 100% (blue line 524, FIG. 5B) methylated fragments, with similar degrees of lengthening (modal size of both 0% and 100% methylated fragments in *Dnase1-*deficient mice is 169 bp). These results suggested that the size profile changes in cfDNA from *Dnase1*-deficient mice occurred relatively independently of methylation status. Furthermore, the increased frequency of short ≤ 150 bp fragments, especially the ultrashort ≤ 80 bp fragments, in 0% methylated fragments seen in cfDNA from WT and *Dnase1l3*-deficient mice was absent in cfDNA of *Dnase1*-deficient mice. Thus, DNASE1 appeared to be responsible for the increased frequency of these short ≤ 150 bp fragments in the 0% methylated fragments.

In summary, while hypomethylated cfDNA tended to have a shorter size profile than hypermethylated cfDNA, the absence of these nucleases also exerted an independent effect on the cfDNA size profile. The cfDNA from *Dnase1*-deficient mice revealed that the increased frequency of short ≤ 150 bp fragments, especially the ultrashort ≤ 80 bp, in the 0% methylated fragments was associated with DNASE1 activity.

### 3. The role of OCR and CGI fragments in cfDNA methylation

We next explored the genomic origins of these DNASE1 activity-associated, short, unmethylated fragments in the cfDNA of *Dnase1l3*-deficient mice. We hypothesized that they might be associated with OCRs and CpG islands (CGIs) since these regions were known to be hypomethylated compared to the genome as a whole. We classified the ± 500 bp regions flanking the center of TSS and Pol II regions, and regions with H3K27ac and/or H3K4me3 as OCRs and merged these regions with CGIs.

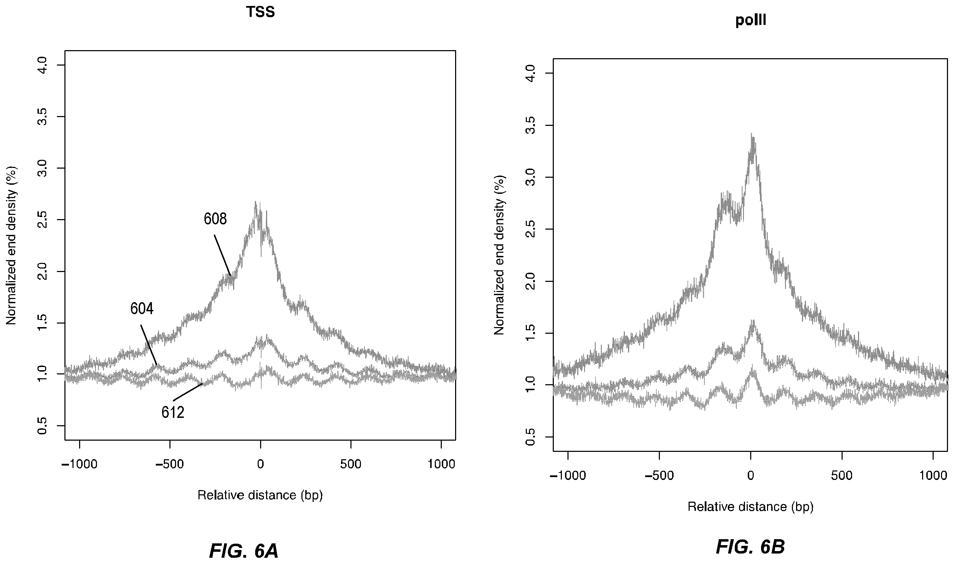

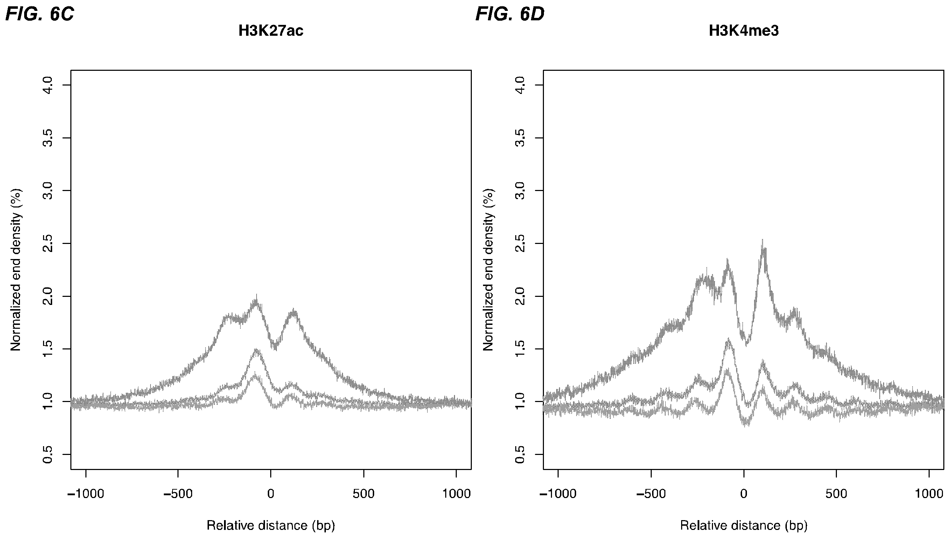

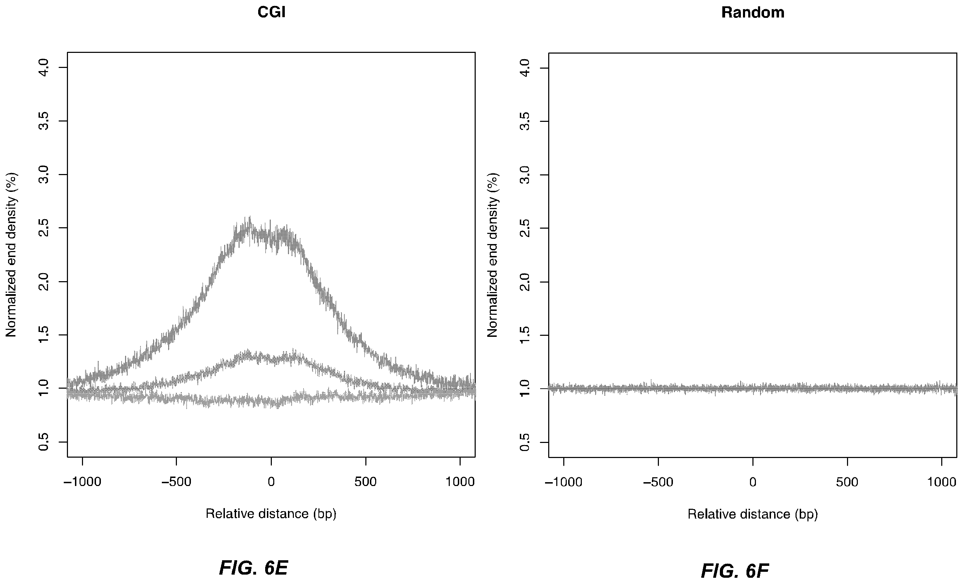

**FIGS. 6A, 6B****,** **6C, 6D****,** **6E,** and **6F** show relative distance to a genomic site on the X-axis and normalized end density as a percent on the Y-axis. The normalized end density is calculated from fragment end counts divided by the median end counts in the ± 3000 bp region. The median normalized end density for each genotype is shown in a ± 1000 bp window over the aggregated TSS region (FIG. 6A), RNA polymerase II (Pol II) (FIG. 6B), H3K4me3 (FIG. 6C), and H3K27ac regions (FIG. 6D), CGIs (FIG. 6E), and randomly selected regions (FIG. 6F). cfDNA from wildtype mice is in green (e.g., green line 604 in FIG. 6A), *Dnase1l3*-deficient mice is in red (e.g., red line 608), and *Dnasel-deficient* mice in blue (e.g., blue line 612). In FIGS. 6A-6E, the red line is the highest, the green line is the next highest, and the blue line is the lowest.

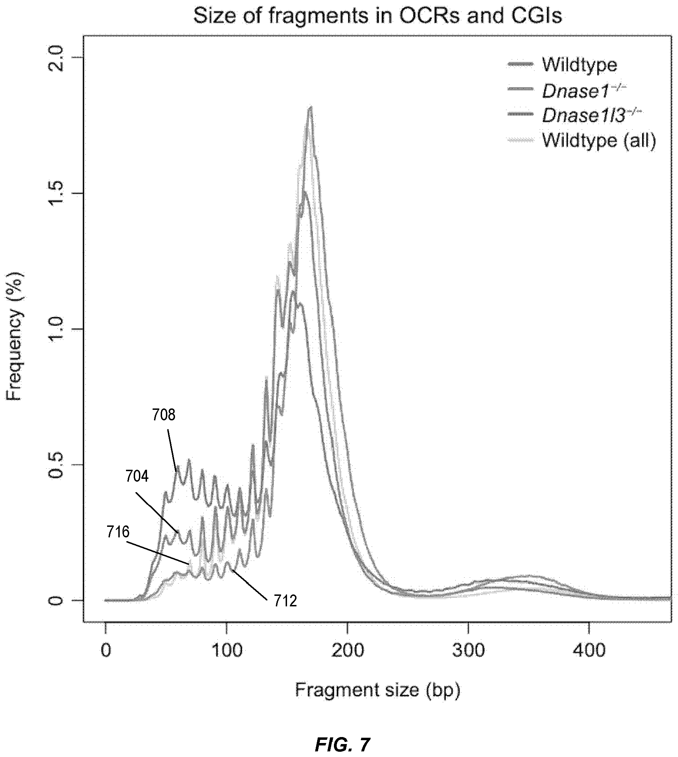

We observed that these OCR and CGI regions had increased end density in the cfDNA of *Dnase1l3*-deficient mice (e.g., red line 608) and decreased end density in the cfDNA of *Dnase1-*deficient mice (e.g., blue line 612) compared to WT (e.g., green line 604). In comparison, the normalized end density in random regions of the genome was similar and overlapping in the cfDNA of WT, *Dnase1l3-*deficient*,* and *Dnase1*-deficient mice **(****FIG. 6F****).** Thus, these OCR and CGI regions were differentially fragmented in the cfDNA of the different genotypes compared to random genomic regions. The increased fragmentation at these OCR and CGI regions in the cfDNA of *Dnase1l3-*deficient mice correlated with an increased proportion of short (≤ 150 bp) fragments from OCRs and CGIs (FIG. 7).

**FIG. 7** shows the size profile of fragments in OCRs and CGIs. The fragment size in base pairs is shown on the X-axis. The frequency of the fragment size is shown on the Y-axis. The median cfDNA size profile of fragments inside OCRs and CGIs is shown. cfDNA from wildtype mice is shown with green line 704, *Dnase1l3*-deficient mice is shown with red line 708, and *Dnase1*-deficient mice is shown with blue line 712. The cfDNA size profile of all wildtype fragments is shown as a comparison with gray line 716.The marked reduction in the proportion of short (≤ 150 bp) cfDNA in OCRs and CGIs in cfDNA of *Dnase1*-deficient mice linked these hypomethylated short fragments to DNASE1 fragmentation of OCRs and CGIs.

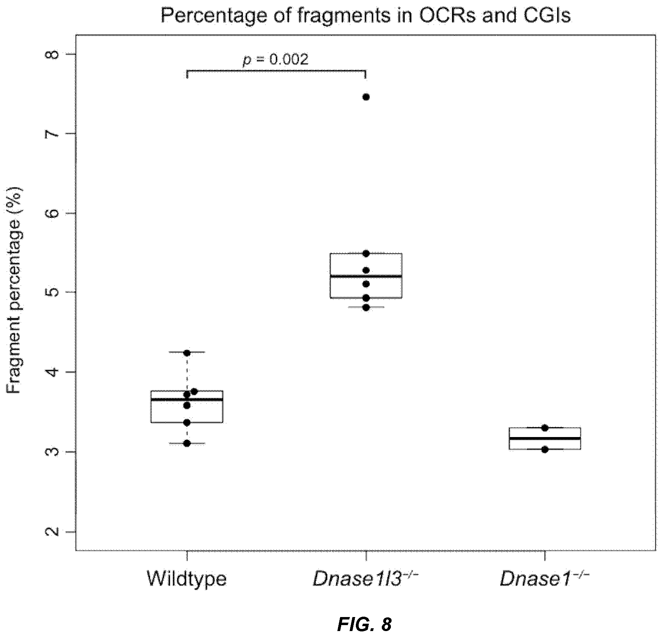

The percentages of fragments within these selected OCRs and CGIs are shown in **FIG. 8****.** The genotype is shown on the X-axis. The fragment percentage in regions ± 500 bp around the center of TSS, PoI II, H3K4me3, and H3K27ac regions merged with CGI regions is shown in the Y-axis. Compared to cfDNA from WT mice, cfDNA from *Dnase1l3*-deficient mice had significantly more fragments in the OCRs and CGIs (WT median percentage: 3.66% vs. *Dnase1l3-*deficient median percentage: 5.20%, Wilcoxon rank-sum test, p *=* 0.002). There also appeared to be a slightly lower percentage of these fragments in the cfDNA of *Dnase1*-deficient mice compared with cfDNA of WT mice (*Dnase1*-deficient median percentage: 3.17%). These OCR and CGI fragment percentages from each sample were all greater than the expected percentage of these OCRs and CGIs in the mouse genome, which is 2.61%. Thus, these hypomethylated OCR and CGI fragments generally appeared to be slightly enriched in plasma cfDNA.

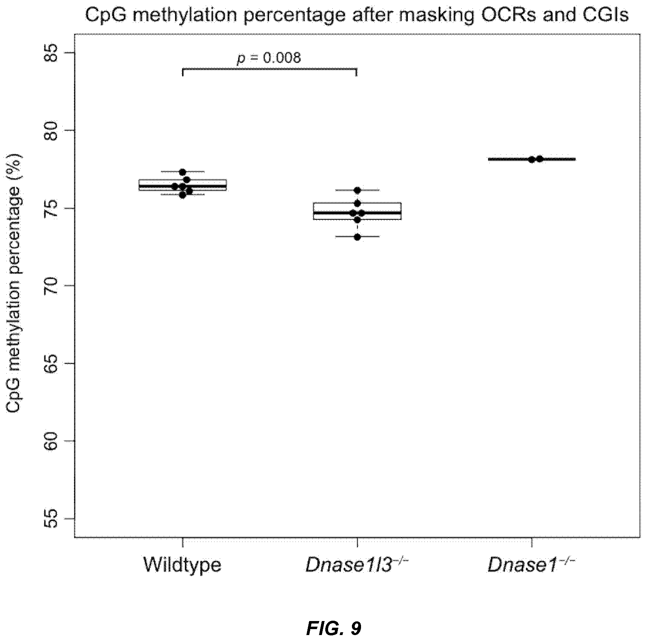

To explore the contribution of these OCR and CGI fragments to the methylation differences of cfDNA from mice of the different nuclease genotypes, we recalculated the overall methylation levels of cfDNA in each of the genotypes after bioinformatically masking these fragments from the OCRs and CGIs **(****FIG. 9****).** In FIG. 9, the genotype is shown on the X-axis. The Y-axis shows the CpG methylation percentage after bioinformatically excluding OCR and CGI fragments in masking analysis. Strikingly, the large degree of hypomethylation that was seen in the cfDNA of *Dnase1l3*-deficient mice (FIG. 1) was returned to a median CpG methylation of 74.7% (FIG. 9), similar to its buffy coat methylation percentage. In fact, the overall methylation level for all genotypes increased after these OCR and CGI originated fragments were excluded (WT median percentage: 76.4%, *Dnase1*-deficient median percentage: 78.2%), essentially reverting to the methylation level of their paired buffy coat.

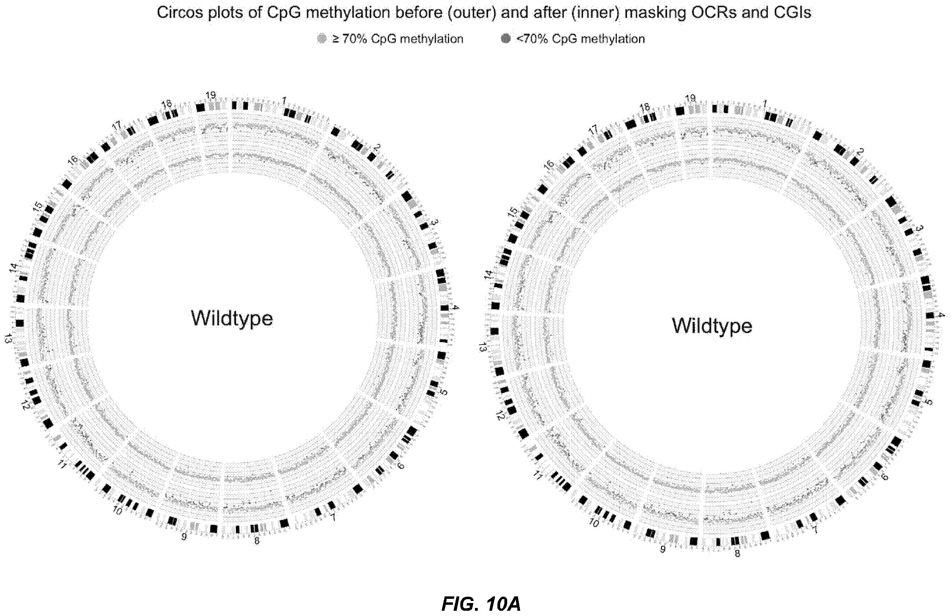

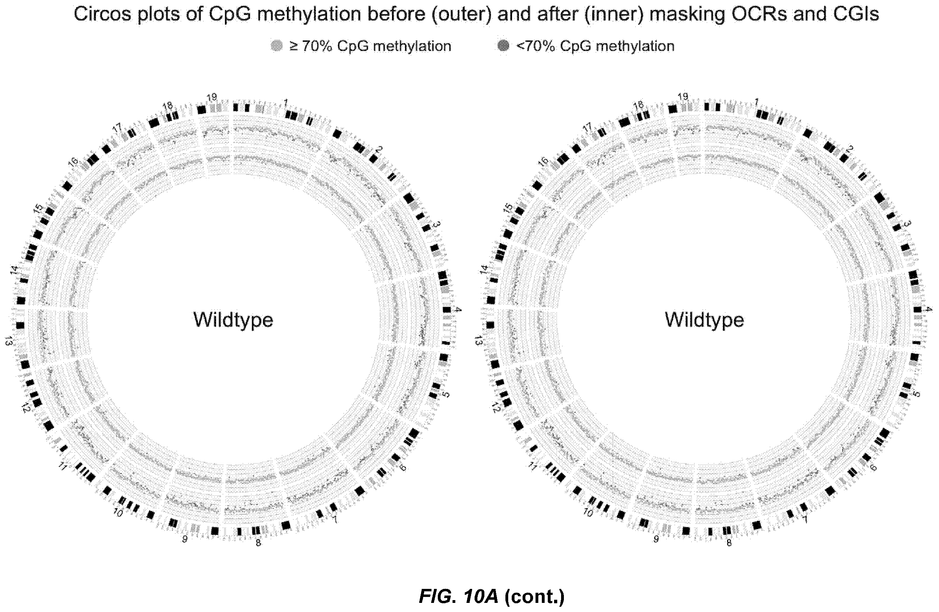

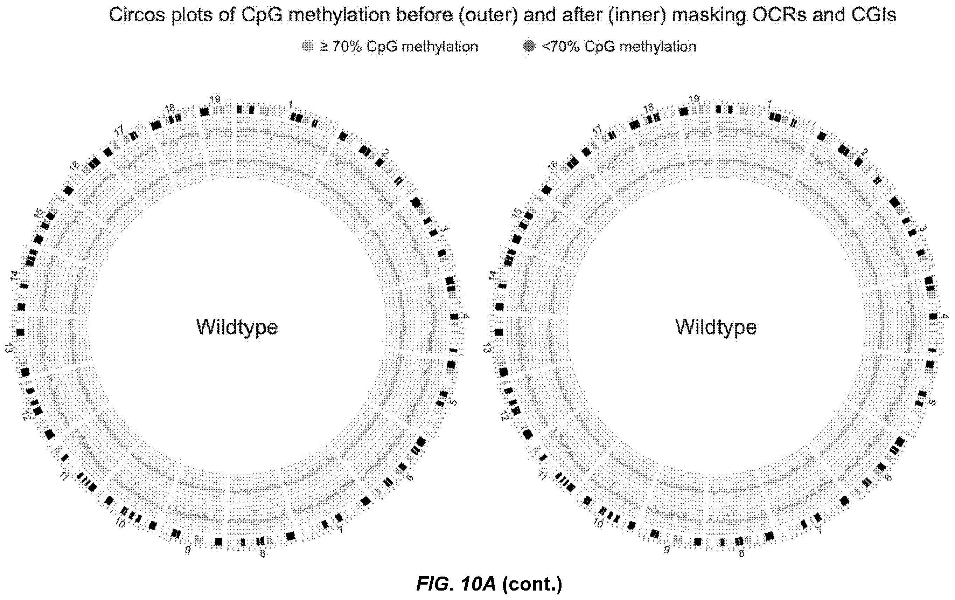

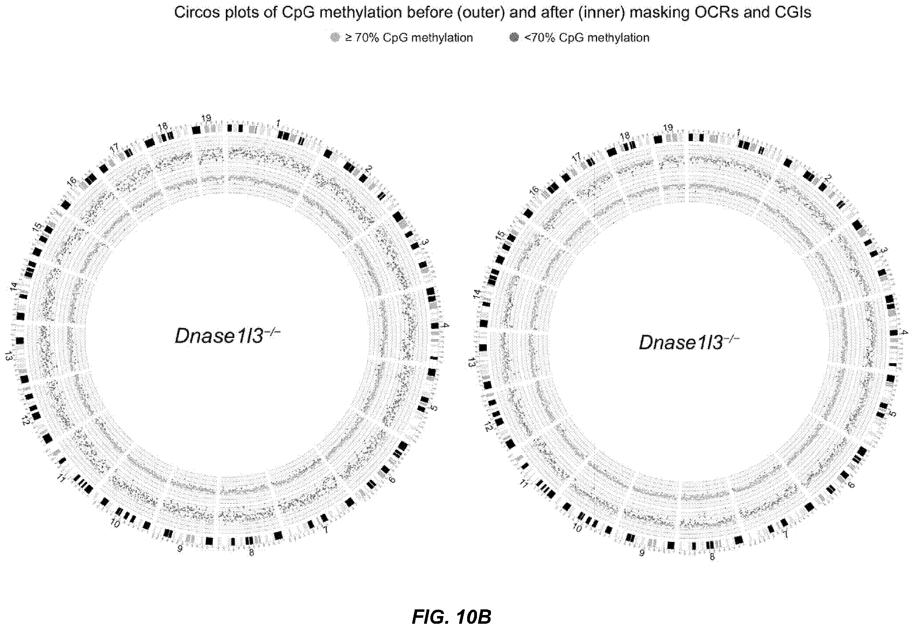

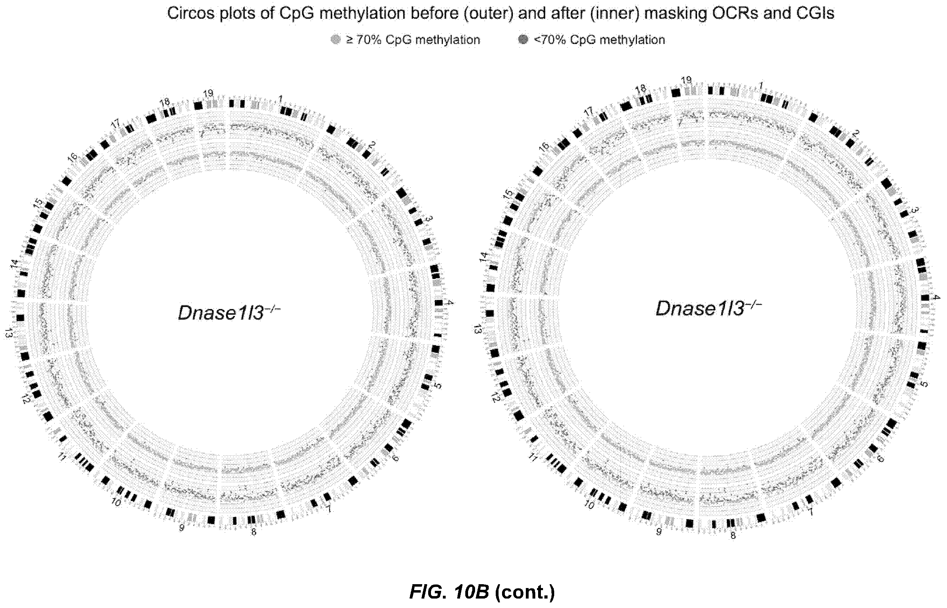

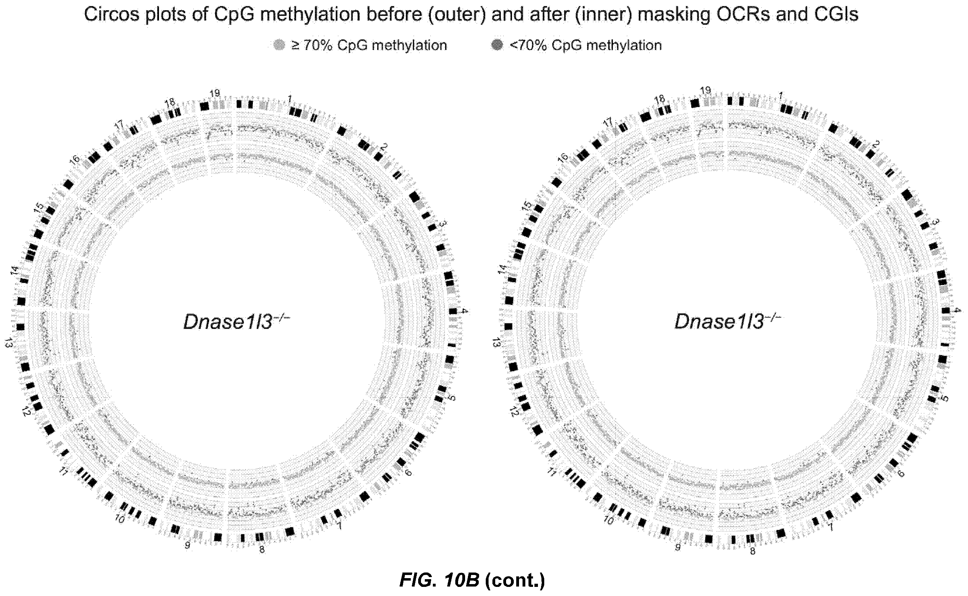

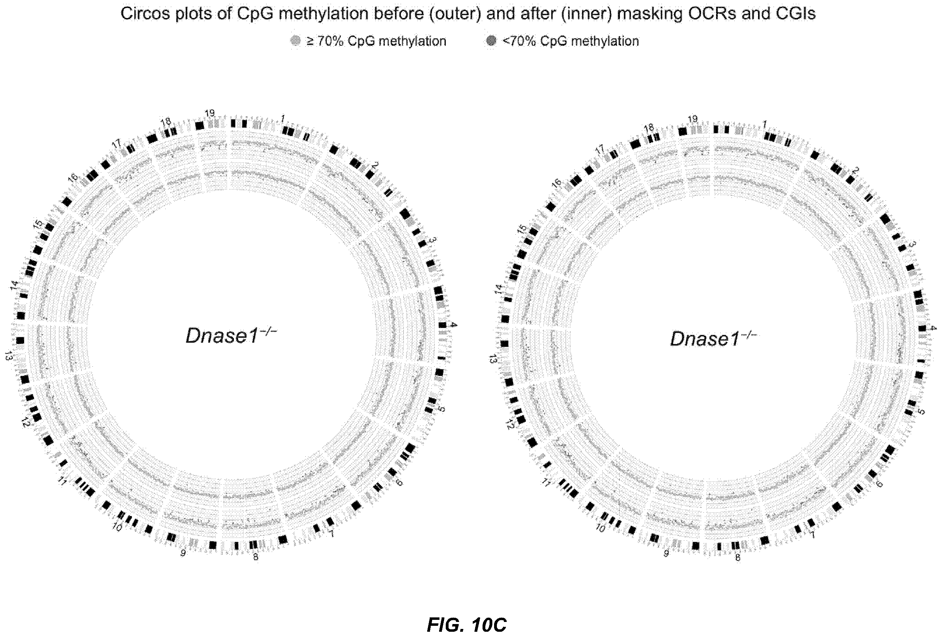

**FIGS. 10A-10C** are circos plots from each sample where each dot represents the CpG methylation percentage in a 1 Mb bin of each of the murine autosome and colored in blue if ≥ 70% and in red if < 70%. The outer ring is the CpG methylation percentage in each 1 Mb bin with all fragments included, and the inner ring is with the fragments in OCRs and CGIs excluded. Masking the fragments in OCRs and CGIs reduced the relatively hypomethylated regions of cfDNA from WT (FIG. 10A), *Dnase1-*deficient (FIG. 10B), and *Dnase1l3*-deficient mice (FIG. 10C) in a genome-wide manner. The majority of hypomethylated regions in cfDNA from *Dnase1l3*-deficient mice disappeared after masking these OCRs and CGIs. These results suggested that it was the fragments in these OCRs and CGIs that were a major cause of the observed hypomethylation of cfDNA from *Dnase1l3*-deficient mice; these OCR and CGI fragments also played a role in the general hypomethylation seen in cfDNA compared with genomic DNA.

### 4. Differential methylation levels and OCR and CGI proportions by fragment size

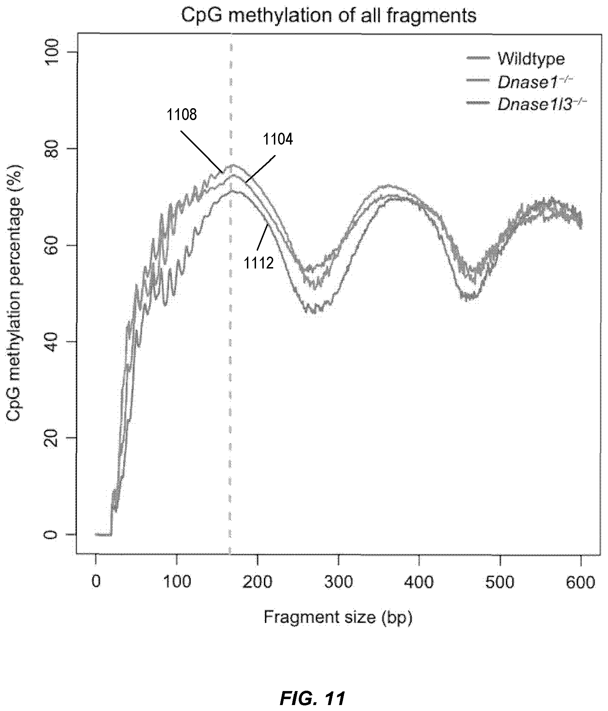

We then analyzed the methylation level of cfDNA by fragment size. For all the fragments of a particular size, the CpG methylation percentage was calculated and the median of each genotype was plotted in **FIG. 11****.** The X-axis shows the fragment size. The Y-axis shows the CpG methylation percentage. Wildtype is shown with green line 1104. *Dnase1*-deficient mice is shown with blue line 1108. *Dnasell3-deficient* mice is shown with red line 1112. Gray dashed lines mark the 166 bp fragment size. In all genotypes, the CpG methylation appeared to follow a periodic pattern with peaks in methylation at around 170 bp, 360 bp, and 550 bp fragment sizes. These fragment sizes corresponded to sizes associated with mono-, di-, and tri-nucleosomes, suggesting that nucleosome-associated cfDNA fragments were more likely to be methylated.

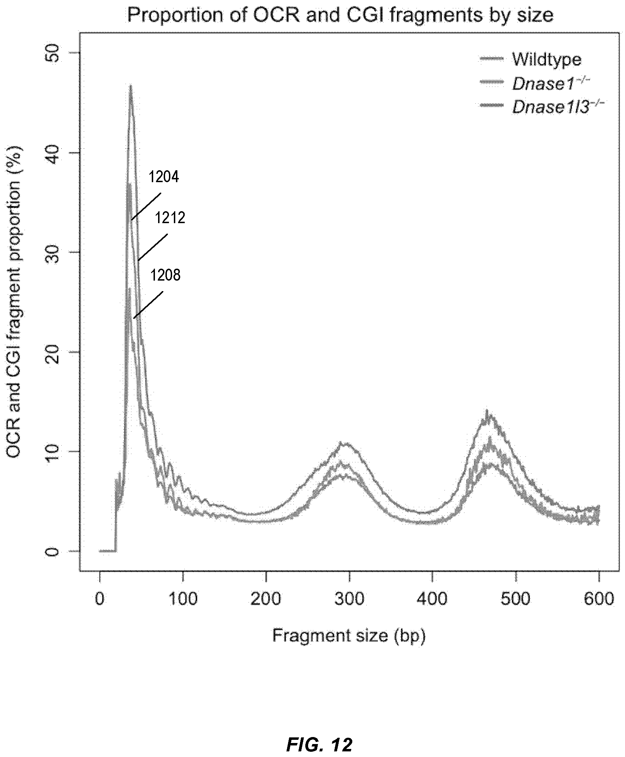

The troughs in cfDNA methylation percentage were around fragment sizes 270 bp and 460 bp. These troughs in cfDNA methylation corresponded to higher proportions of OCR and CGI fragments for all genotypes **(****FIG. 12).** FIG. 12 has fragment size on the X-axis. The proportion of OCR and CGI fragments within each fragment size was calculated, and the median of each genotype is shown on the Y-axis. Wildtype is shown with green line 1204. *Dnase1-*deficient mice is shown with blue line 1208. *Dnase1l3*-deficient mice is shown with red line 1212. Across all fragment sizes, the OCR and CGI fragment proportions were higher in cfDNA from *Dnase1l3-*deficient mice than in cfDNA from WT and *Dnase1*-deficient mice. In cfDNA from *Dnase1*-deficient mice, the OCR and CGI fragment proportions were relatively reduced in the ultrashort fragments ≤ 80 bp, suggesting a strong role for DNASE1 in increasing the OCR and CGI proportions of cfDNA at these fragment sizes. Interestingly, in the fragment sizes associated with troughs in methylation, cfDNA from *Dnase1*-deficient mice had a slightly higher OCR and CGI fragment proportion than in cfDNA from WT mice. The slightly higher OCR and CGI proportions in these fragment sizes might be related to other enzymes. Hence, we have shown that different fragments sizes of cfDNA were associated with different methylation levels and different proportions of OCR and CGI fragments.

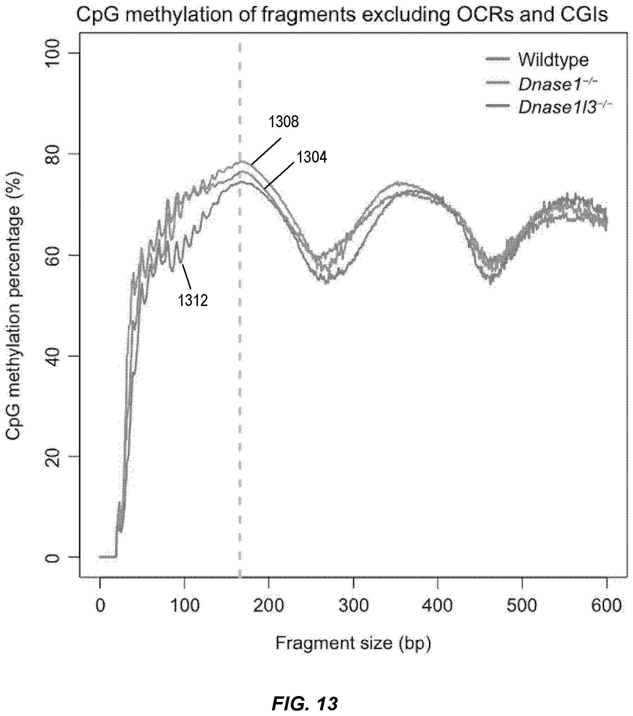

To tease out the relationship between the methylation level and OCR and CGI fragment proportion among different fragment sizes, we bioinformatically masked the OCR and CGI fragments and replotted the CpG methylation level of each fragment size after masking **(****FIG. 13****).** In FIG. 13, wildtype is shown with green line 1304; *Dnase1-deficient* mice is shown with blue line 1308; *Dnase1l3*-deficient mice is shown with red line 1312. Gray dashed lines mark the 166 bp fragment size.

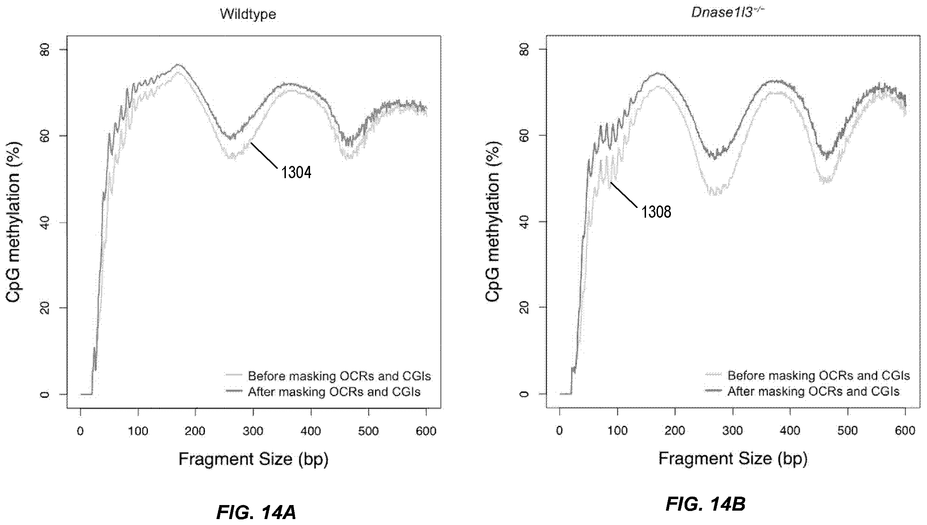

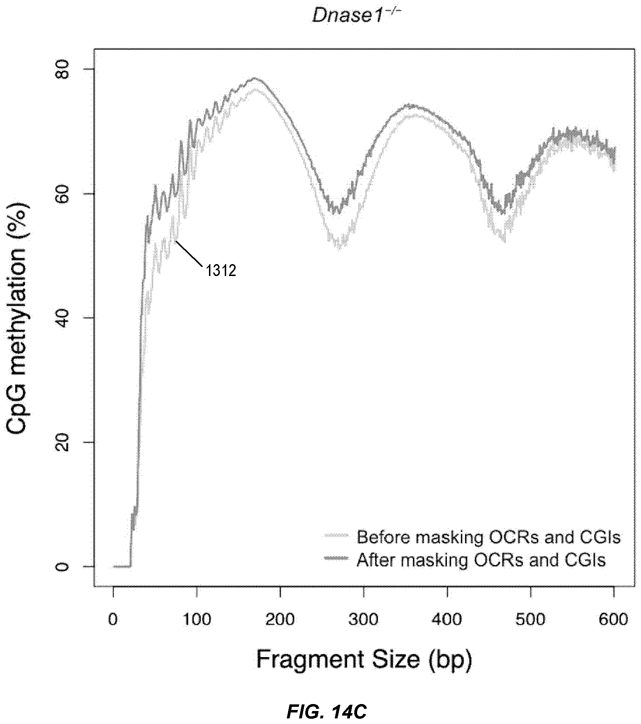

**In** **FIGS. 14A, 14B****, and** **14C****,** CpG methylation percentage with each fragment size is shown before and after masking OCR and CGI fragments. The before masking percentages are shown with gray lines 1404, 1408, and 1412.

After masking, while the periodic pattern persisted, the peak-trough difference decreased and the methylation percentage of all fragment sizes increased for all genotypes (**FIG. 13** and **FIGS. 14A** and **14B**)**.** For all genotypes, the fragment sizes that exhibited the greatest increase in methylation after masking these OCR and CGI fragments were those ≤ 80 bp and around the first trough around 270 bp (FIG. 14), corresponding to sizes that had higher proportions of OCR and CGI fragments. At 270 bp, the methylation percentage rose from 55.4% to 60.3% in cfDNA from WT mice, 50.9% to 56.9% in cfDNA from *Dnase1-deficient* mice, and 46.7% to 55.0% in cfDNA from *Dnase1l3*-deficient mice. On the other hand, the methylation level of likely mono-nucleosome-associated fragments increased only minimally; for example, the 166 bp fragment methylation percentage increased from 74.3% to 76.3% in WT, 76.3 to 78.2% in DNASE1-deficient cfDNA, and 71.1% to 74.3% in DNASE1L3-deficient cfDNA. These results illustrated, again, that the effect of the OCR and CGI fragments on cfDNA methylation was more evident for certain cfDNA sizes.

Comparing between the nuclease genotypes in FIG. 4A, cfDNA from *Dnase1l3-*deficient mice was hypomethylated compared to that of WT and *Dnase1*-deficient mice in most fragment sizes up to ~ 500 bp, which comprised of 98-99% of the total population of sequenced cfDNA. After masking, the degree of hypomethylation was diminished more in certain fragment sizes than in others (FIG. 13). Interestingly, fragment sizes from around 80 bp to 200 bp, and 250 bp to 350 bp, still had a substantial difference in methylation percentage between cfDNA from *Dnase1l3-*deficient mice and cfDNA from WT and *Dnase1*-deficient mice after masking the OCR and CGI fragments. This could be due to the existence of other hypomethylated fragments not accounted for by our bioinformatic masking and/or the relative absence of hypermethylated fragments in these regions.

In contrast, the relative hypermethylation of cfDNA from *Dnase1*-deficient mice occurred only in certain size ranges, most obviously around the 166 bp and 360 bp methylation peaks (FIG. 11). This relative hypermethylation did not appreciably change after masking the OCR and CGI fragments (FIG. 13). Thus, the relative hypermethylation seen in cfDNA from *Dnase1*-deficient mice was mostly in mono- and di-nucleosomally sized cfDNA and was unlikely to be related to OCR and CGI fragments.

### 5. DNASE1L3 cuts methylated CpGs

While we had demonstrated that DNASE1 activity in the hypomethylated OCRs and CGIs was a major contributor to the relative hypomethylation in cfDNA of *Dnase1l3*-deficient mice, DNASE1 activity appeared to be only part of the whole picture. Even after masking the OCR and CGI fragments, the relative hypomethylation of cfDNA from *Dnase1l3-*deficient mice compared with cfDNA from WT mice persisted (Wilcoxon rank-sum test, p = 0.008) (FIG. 9), especially in fragment sizes 80 bp to 200 bp, and 250 bp to 350 bp (FIG. 13). Hence, we proceeded to explore the role of DNASE1L3 as well. We reasoned that the relative hypomethylation seen in the plasma of *Dnase1l3*-deficient mice could also result from a decreased contribution of methylated fragments by DNASE1L3.

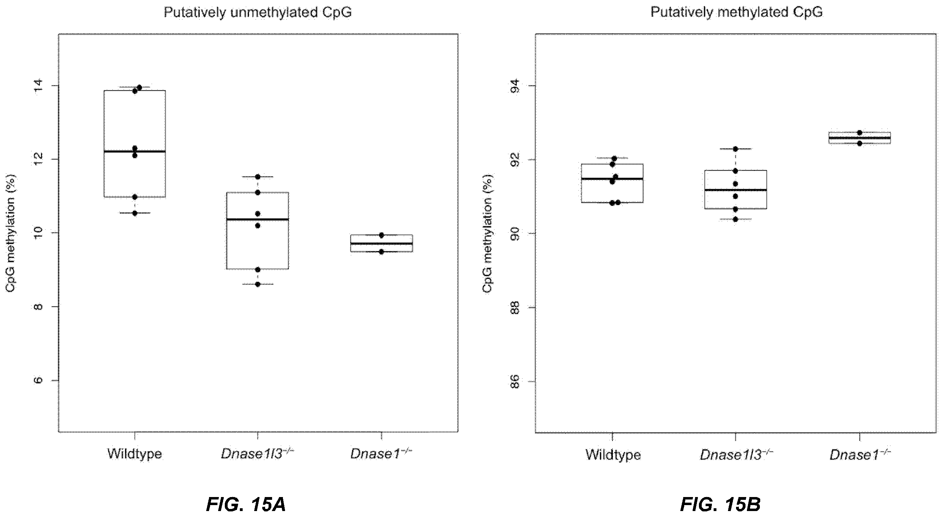

We devised a method to interrogate whether, or not, DNASE1L3 could cut methylated CpGs. To do this, we first identified methylated and unmethylated CpGs. From a downloaded dataset comprised of bisulfite sequencing of eight different mouse tissues (bone marrow, thymus, spleen, kidney, heart, liver, large intestines, small intestines) with two replicates each, we mined for CpGs that were methylated in 90% of all tissue and replicate reads and identified them as putatively methylated CpGs (545,720 CpGs in total). Similarly, we also mined for CpGs that were unmethylated in 80% of the reads in the dataset and identified them as putatively unmethylated CpGs (7,140 CpGs in total). Using CpGs unmethylated in 90% of reads for subsequent analysis was more difficult due to the extremely low number of CpGs that fulfilled this condition (11 CpGs in total). With these putatively methylated and unmethylated CpGs identified from this downloaded dataset, we confirmed that the actual methylation level of these CpGs in our plasma dataset would be similar to its expected methylation level. For putatively methylated CpGs, these CpGs had a > 90% methylation level in the plasma cfDNA of each sample, and for the putatively unmethylated CpGs, these CpGs had a < 20% methylation level in our plasma cfDNA of each sample **(****FIGS. 15A** and **15B**).

With these putatively methylated and unmethylated CpGs identified, we calculated the normalized end density over these CpGs and its surrounding region. When aggregated together placing the putatively methylated C at position 0, there was an end density pattern over the surrounding ± 1000 bp that was strongly periodic, reminiscent of the nucleosomal array found around CTCF regions **(****FIG. 16A****)** (Fu et al., 2008; Kelly et al., 2012). These results suggested that these putatively methylated CpGs that we identified likely originated from DNA in a packed chromatin structure, which, only when the surrounding nucleosomes were well-phased, would give rise to such clear periodicity. Narrowing our focus to just the putatively methylated C, we found that there was an increase in normalized end density at the putatively methylated C in all plasma samples from WT and *Dnase1*-deficient mice **(****FIG. 16B****).** Thus, in the presence of DNASE1L3 in the cfDNA of WT and *Dnase1*-deficient mice, there were very specific cuts at the putatively methylated C. In contrast, in the plasma of *Dnase1l3*-deficient mice, the putatively methylated C was no longer preferentially cut compared to its surrounding -6 to +8 bp region **(****FIG. 16C****).** Red line 1604 shows normalized end density for *Dnase1l3*-deficient mice. Therefore, this evidence suggested that DNASE1L3 was responsible for cleaving these putatively methylated Cs in these nucleosomal arrays, and that in its absence, the fragmentation pattern was no longer as specific, falling in a broader -6 to +8 bp region, which was likely a linker region between nucleosomes. Intriguingly, in the absence of DNASE1L3, the positions with the highest end density in this linker region, i.e., the new preferential cutting sites, were exactly 10 bp apart. This could explain the increase in prominence of the 10 bp periodicity in the cfDNA size profile of *Dnase1l3*-deficient mice.

On the other hand, the putatively unmethylated CpGs appeared to originate from very different genomic regions compared with the putatively methylated CpGs. The surrounding region of the putatively unmethylated CpGs demonstrated a generalized increase in normalized end density in the -400 to +400 bp regions around the putatively unmethylated CpGs **(****FIG. 17A****).** These larger regions were thus more accessible to fragmentation, a property which would suggest that these regions were likely to be OCRs. In cfDNA from WT and *Dnase1*-deficient mice, there was no particular preference for the putatively unmethylated C to be cut compared to its surrounding ± 1000 bp region **(****FIG. 17B****).** In the plasma of *Dnase1l3*-deficient mice, the putatively unmethylated C was also not preferentially cut, instead, its flanking bases had a higher end density compared to cfDNA of WT mice **(****FIG. 17C****).** Red line 1704 shows normalized end density for *Dnase1l3*-deficient mice. This increase in fragment ends in the region flanking the putatively unmethylated C in cfDNA of *Dnase1l3*-deficient mice echoed our previous findings in the OCRs and CGIs. Similarly, the decrease in end density in the region flanking the putatively unmethylated C in cfDNA of *Dnase1*-deficient mice (Fig. 17B) was suggestive again that DNASE1 played a major role in creating the fragment ends around unmethylated regions. Thus, from this analysis, we have uncovered the cutting preference of DNASE1L3 at methylated CpGs and unmethylated CpGs using nuclease-deficient mice.

### 6. DNASE1L3-deficient human subjects

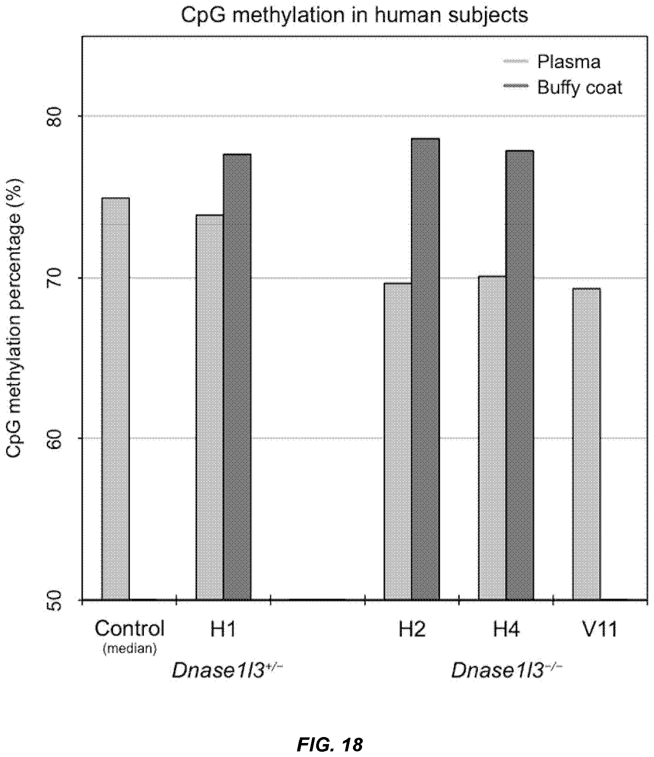

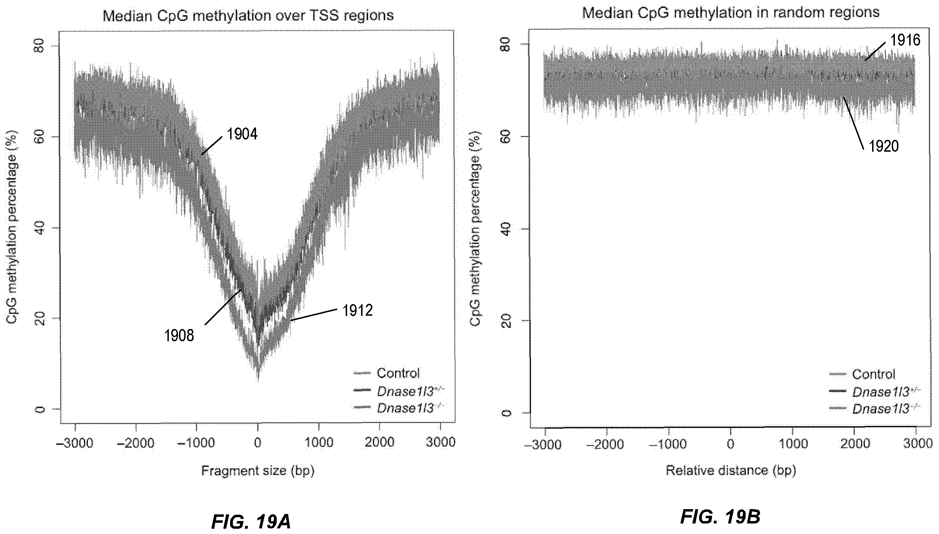

To extrapolate our findings to human cfDNA, we performed bisulfite sequencing of plasma samples from three *DNASE1L3*-deficient subjects (H2, H4, and V11) and one heterozygous parent (H1) (Chan et al., 2020). Similar to *Dnase1l3*-deficient mice, the plasma cfDNA of *DNASE1L3-*deficient subjects was hypomethylated compared to both controls and the heterozygous parent (CpG methylation of *DNASE1L3-*deficient subjects H2: 69.66%, H4: 70.1%, and V11: 69.32%, vs. median of 8 controls: 74.90%, and H1: 73.84%) **(****FIG. 18).** FIG. 18 shows samples from human subjects of different genotypes on the X-axis. The first column (or only column with Control and V11 samples), shown in orange, is from plasma. The second column, shown in purple, is from buffy coat. The Y-axis shows the CpG methylation percentage. The plasma cfDNA methylation levels of all controls and subjects were also lower than that of buffy coat samples (FIG. 18). This hypomethylation of plasma cfDNA from *DNASE1L3-*deficient patients was seen in both TSS and random regions and is thus also a genome-wide phenomenon **(****FIGS. 19A, 19B****).** In FIG. 19A, the control samples are shown with green line 1904; the heterozygous *DNASE1L3* parent is shown with dark green line 1908; and the *DNASE1L3-*deficient subjects is shown with red line 1912. In FIG .19B, the control samples are shown with green line 1916; the *DNASE1L3-*deficient subjects is shown with red line 1920. The heterozygous *DNASE1L3* parent is shown with a dark green line that is sometimes visible between red line 1920 and green line 1916 and sometimes visible above green line 1916.

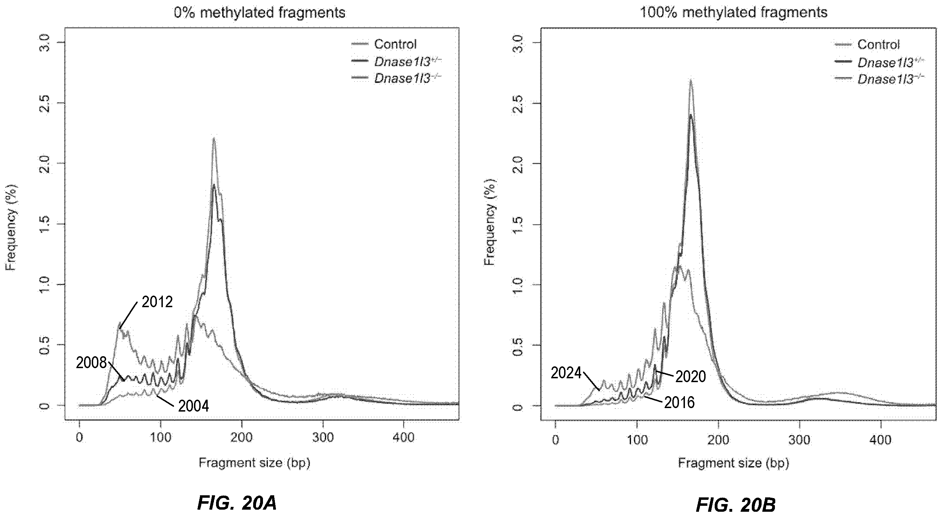

Similarly, the plasma cfDNA of *DNASE1L3-*deficient patients had a shorter size profile that is more exaggerated in 0% methylated fragments than in 100% methylated fragments **(****FIGS. 20A, 20B****).** In FIG. 20A, the control samples are shown with green line 2004; the heterozygous *DNASE1L3* parent is shown with dark green line 2008; and the *DNASE1L3-*deficient subjects is shown with red line 2012. In FIG. 20B, the control samples are shown with green line 2016; the heterozygous *DNASE1L3* parent is shown with dark green line 2020; and the *DNASE1L3-*deficient subjects is shown with red line 2024.

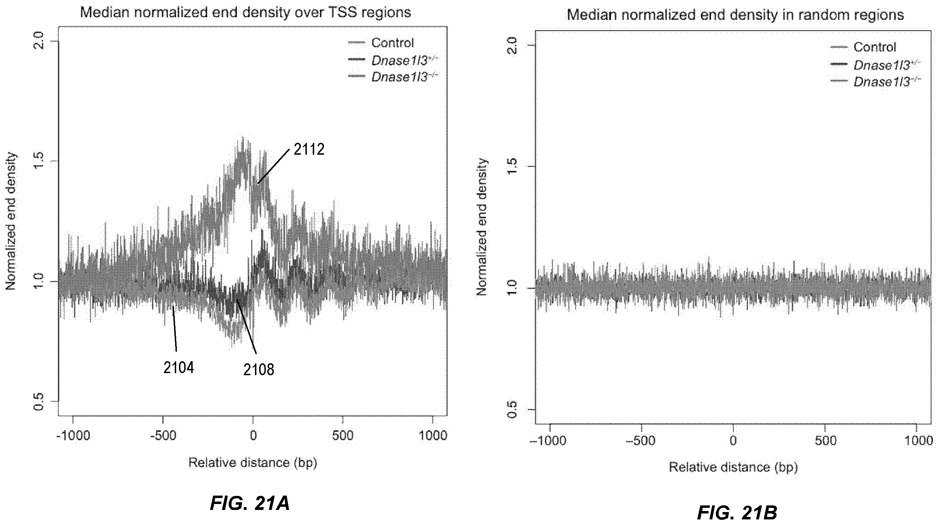

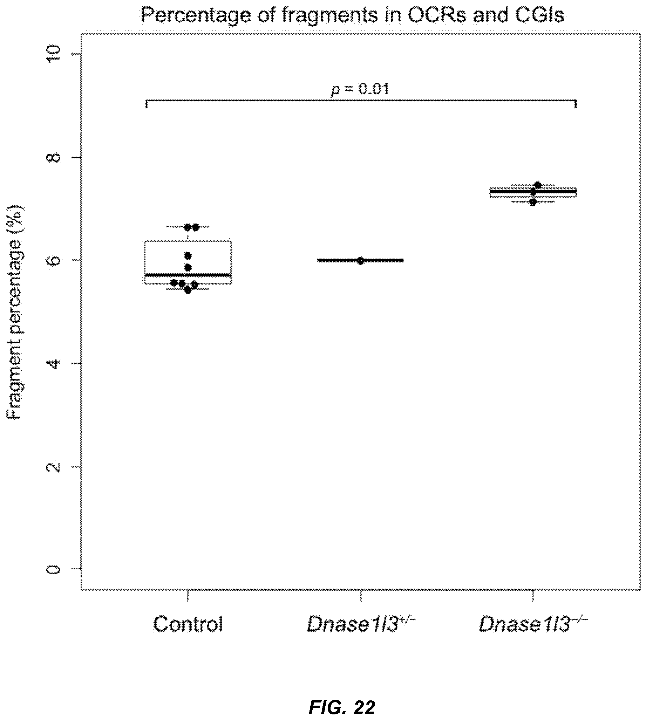

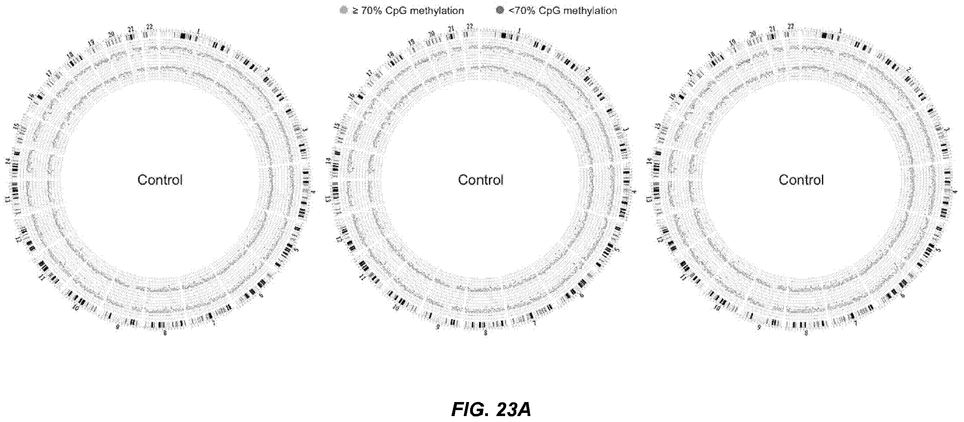

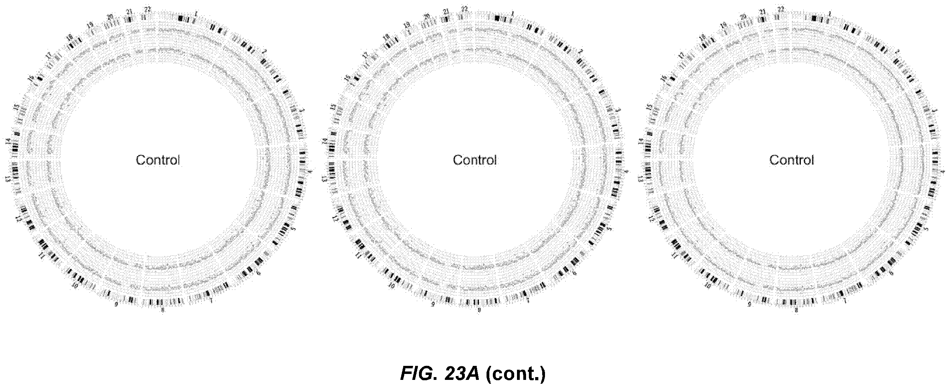

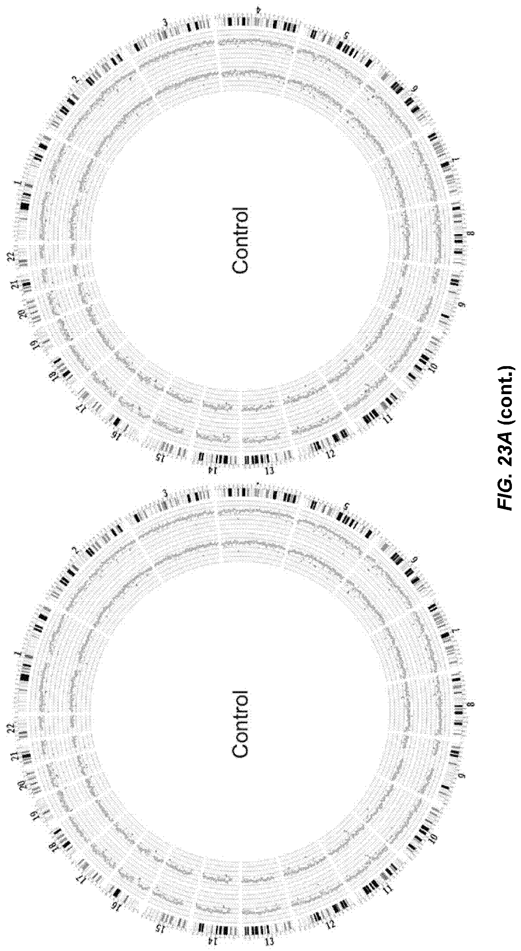

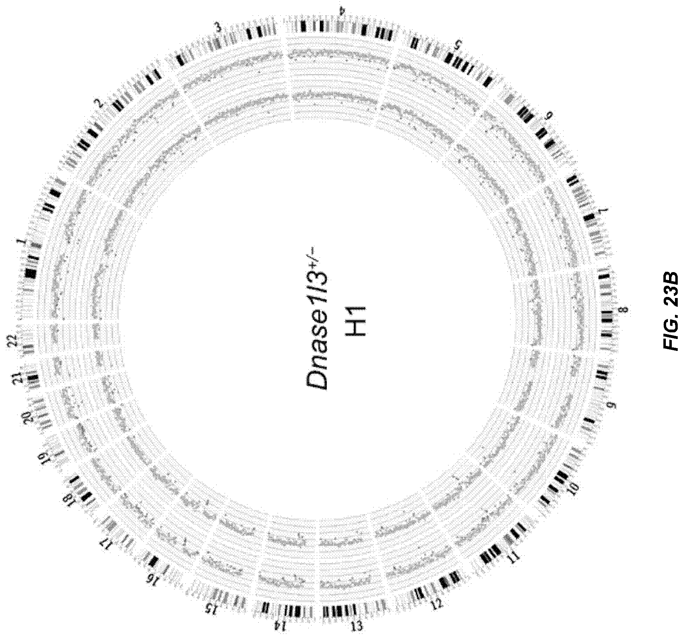

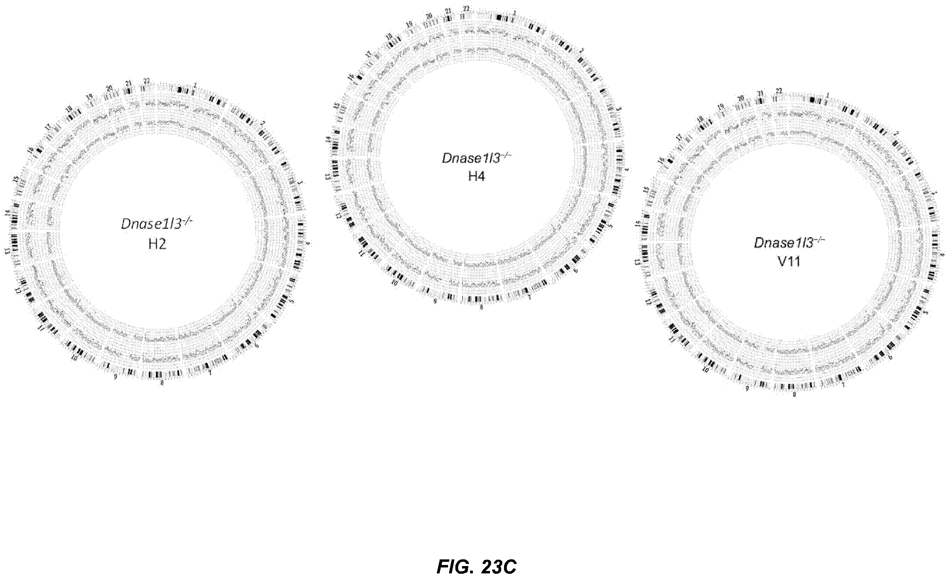

The shorter size profile corresponds to an increase in normalized end density in hypomethylated open chromatin TSS regions **(****FIG. 21A****),** which is in contrast to random regions **(****FIG. 21B****).** In FIG. 21A, the control samples are shown with green line 2104; the heterozygous *DNASE1L3* parent is shown with dark green line 2108; and the *DNASE1L3-*deficient subjects is shown with red line 2112. In FIG. 21B, the different lines for different samples overlap. This suggests that there is increased fragmentation of DNA at hypomethylated regions in the cfDNA of *DNASE1L3-*deficient patients. This is substantiated by the significant increase in fragments from OCR and CGI regions in the plasma of *DNASE1L3-*deficient patients compared to that of controls (Control median percentage: 5.71 % vs. *DNASE1L3*-deficient median percentage: 7.34%, Wilcoxon rank-sum test, *p* = 0.01) **(****FIG. 22****).** When these OCR and CGI fragments were bioinformatically masked, the plasma CpG hypomethylation reverts to the level seen in controls **(****FIGS. 23A****,** **23B****,** and **23C**). FIG. 23A shows circos plots from normal subjects. FIGS. 23B and 23C show circos plots from *DNASE1L3-*deficient subjects. Overall, the increased cutting of OCR and CGI regions into short hypomethylated fragments also accounts for the relative hypomethylation seen in the cfDNA of *DNASE1l3-*deficient subjects.

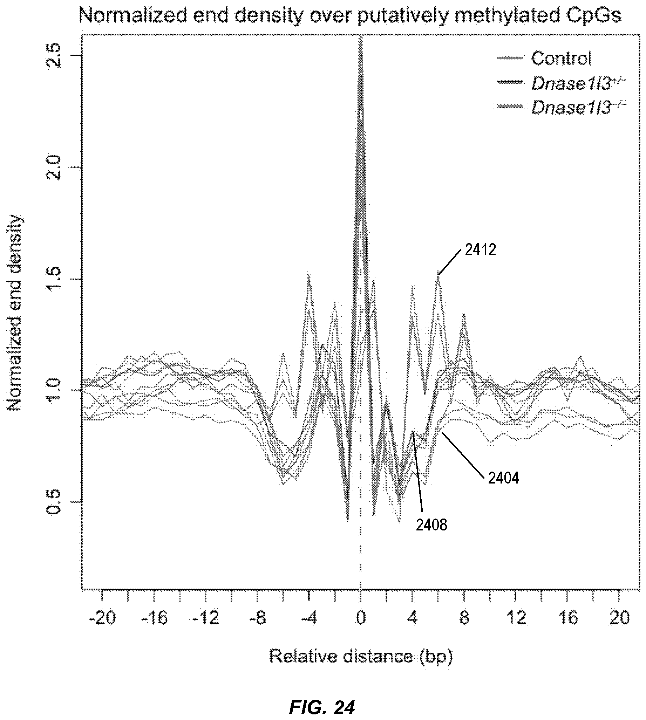

The cutting preference of DNASE1L3 was also demonstrated in human cfDNA. Control plasma cfDNA was found to end preferentially at putatively methylated CpGs **(****FIG. 24****).** A control sample is shown with green line 2404; the heterozygous *DNASE1L3* parent is shown with dark green line 2408; and a *DNASE1L3-*deficient subject is shown with red line 2412. This striking preference for fragments to end at the putatively methylated CpGs appears to be more pronounced in human cfDNA compared to mice cfDNA with the normalized end density around 2.4 in humans compared to 1.5 in mice. This end preference is absent in the cfDNA of DNASE1L3-deficient subjects with a resulting end density profile showing peaks in the broader -6 to +8 bp region (FIG. 24). Thus, we found that *DNASE1L3-*deficient patients have cfDNA that is largely similar to *Dnase1l3*-deficient mice, confirming this link between nuclease activity and cfDNA methylation in human plasma.

### B. Size profile and methylation changes

In this work, we have discovered that different nuclease deficiencies profoundly affect the apparent methylation level and size profile of plasma cfDNA on a genome-wide level. We have found that the plasma cfDNA of *Dnase1l3*-deficient mice and *DNASE1L3-*deficient humans is much more hypomethylated than cfDNA from control samples and has a shorter size profile with an increase in short ≤ 150 bp fragments and a decrease in 166 bp fragments. This is in contrast to the cfDNA of *Dnase1*-deficient mice, which is slightly more hypermethylated than WT cfDNA and has a slightly longer size profile with a decrease in short ≤ 150 bp fragments and an increase in 166 bp fragments. Since the methylation levels of the buffy coat genomic DNA are similar among the different genotypes, the differences in plasma cfDNA methylation are likely related to the nuclease activities during the DNA fragmentation process.

In our exploration of the cause of hypomethylation and hypermethylation in the plasma cfDNA of *Dnase1l3*-deficient and *Dnase1*-deficient mice, respectively, we found that cfDNA from *Dnase1l3-*deficient mice had more hypomethylated fragments originating from increased fragmentation of open chromatin regions and CpG islands across the whole genome. The reduction of these fragments in cfDNA of *Dnase1*-deficient mice revealed the culprit to be DNASE1. The absence of DNASE1 activity in *Dnase1-deficient* mice allowed us to deduce that DNASE1 increased the fragmentation of these OCRs and CGIs and gave rise to an increased proportion of short fragments, especially ultrashort fragments, in these regions. This understanding of DNASE1 activity is consistent with the whole field and technology of using DNASE1 to probe DNase I hypersensitivity regions in DNASE-seq (Boyle et al., 2008).

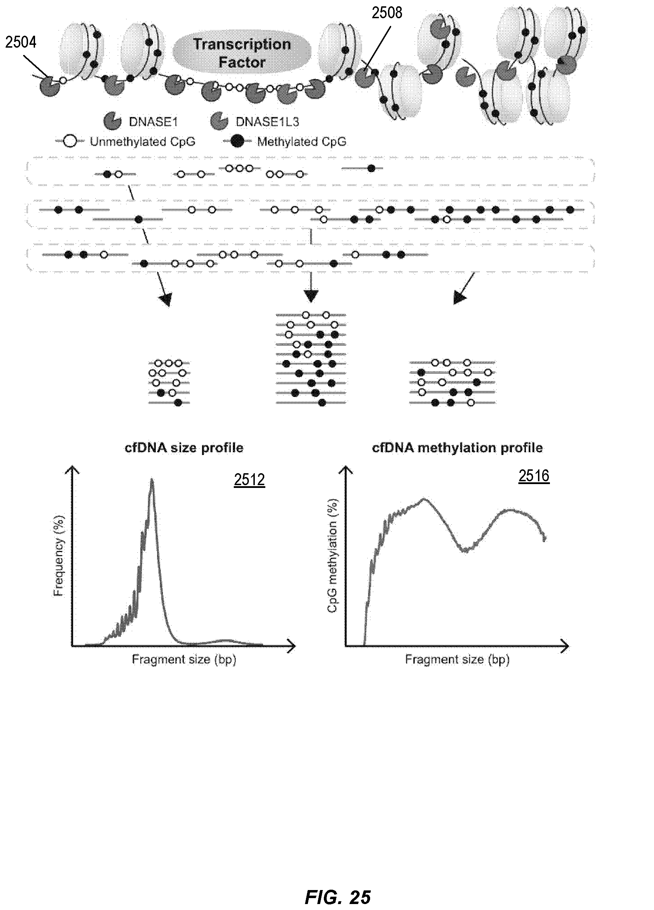

**FIG. 25** shows deduced activities of DNASE1 and DNASE1L3. DNASE1 (e.g., DNASE1 2504 shown in blue) prefers to cleave unmethylated and open chromatin DNA. By fragmenting these regions, DNASE1 increases the representation of these OCR and CGI regions in plasma, resulting in the relative hypomethylation of cfDNA. These OCR and CGI regions are unequally represented among different cfDNA sizes. DNASE1L3 (e.g., DNASE1L3 2508 shown in red) is effective at cutting methylated fragments and increases the representation of methylated fragments in plasma cfDNA compared with DNASE1. DNASE1L3's cutting preference likely result in the prominence of the 166 bp fragment size. The combination of these preferences lead to the eventual cfDNA size profile 2512 and methylation profile 2516 observed for each fragment size.

Bioinformatically masking these OCR and CGI fragments demonstrated that these regions were a major contributor to the relative hypomethylation seen in the plasma cfDNA of *Dnase1l3*-deficient mice. Furthermore, we found that these OCR and CGI fragments were relatively enriched in plasma cfDNA, generally, and that this enrichment explained the relative hypomethylation of plasma cfDNA compared to its genomic DNA (FIG. 3). It appears that DNASE1 activity in hypomethylated OCRs and CGIs increased their fragmentation and allowed for the enrichment of these hypomethylated regions in plasma cfDNA. This also explains the relative hypermethylation of plasma cfDNA from *Dnase1*-deficient mice. It is quite remarkable that these OCR and CGI fragments, which account for only 3-6% of the total sequenced cfDNA population in our samples, could have such a dramatic effect on the apparent methylation level of plasma cfDNA.

The cfDNA size profile actually changes most dramatically in the absence of DNASE1L3. Our analysis with the putatively methylated and unmethylated CpGs shed some light on the reason. We demonstrated that the absence of DNASE1L3 decreased cuts at methylated CpGs. This is supported by existing literature showing that DNASE1L3 can cleave chromatin with high efficiency to almost undetectable levels without proteolytic help (Sisirak et al., 2016; Napirei et al., 2009).

Since the genome is >97% heterochromatin with most of its CpGs methylated, most of the genome is susceptible to DNASE1L3 activity but less so to DNASE1. Thus, it is not surprising that the absence of DNASE1L3 would markedly affect the cfDNA size profile. One of the more noticeable changes of the cfDNA size profile in cfDNA from *Dnase1l3*-deficient mice is the diminished prominence of the 166 bp peak. We hypothesize that the 166 bp fragment size may be produced by the relatively strong local preference for cutting these methylated Cs by DNASE1L3 in the linker regions of chromatin. It is striking to note that in the absence of DNASE1L3, two new fragment end preferences appear that are exactly 10 bp away from each other. This may also account for the increased prominence of the 10 bp periodicity in the cfDNA of *Dnase1l3*-deficient mice.

In fact, this preference by DNASE1L3 for creating 166 bp fragments is apparent in cfDNA from *Dnase1*-deficient mice. In such mice, both 0% and 100% methylated cfDNA were fragmented to a very similar size profile with a very sharp 166 bp peak and exhibited remarkably limited shortening of unmethylated fragments. Thus, in the absence of DNASE1, DNASE1L3 appears to have limited preference to cut unmethylated fragments into smaller fragments. In fact, the end density over the putatively unmethylated CpGs decreased in the cfDNA of *Dnase1-*deficient mice. These results suggest that DNASE1L3 cuts largely agnostically to DNA methylation status, which would increase the methylated portion of plasma cfDNA since methylated CpGs are more abundant than unmethylated CpGs in the genome (FIG. 25).

This work also reveals that different sizes of cfDNA are associated with different methylation levels. cfDNA fragments with sizes that are widely presumed to be associated with mono-, di-, and tri-nucleosomes (around 170 bp, 360 bp, and 550 bp) are relatively hypermethylated, while fragments with intermediary sizes (around 270 bp and 460 bp) are relatively hypomethylated. Masking the OCR and CGI fragments demonstrated that the hypomethylation was disproportionally affected in fragments <= 80 bp and around the troughs for all three genotypes. These fragment sizes actually have a higher proportion of OCR and CGI fragments and may reflect more the activity of DNASE1. We have thus demonstrated that different genomic regions are not represented evenly in the different sizes of cfDNA.

Examining the differences in the methylation level of each cfDNA size between the genotypes reveals that DNASE1L3 plays a role as well. DNASE1L3, which can cut methylated CpGs, appeared to give rise to more 166 bp fragments that are methylated in the cfDNA of *Dnase1*-deficient mice. Mono-nucleosomally sized fragments in the cfDNA of *Dnase1*-deficient mice appear to be the most methylated with the methylation level decreasing with each additional nucleosome, suggesting that DNASE1L3 contribution of methylated fragments is highest for mononucleosomes (FIG. 11). One interpretation of this is that the nucleosome-associated fragment sizes appear more methylated because of increased contribution of methylated fragments by the cutting preferences of DNASE1L3. Also, the remaining difference in methylation level in fragment sizes 80 to 200 bp, and 250 to 350 bp, between cfDNA of *Dnase1l3-*deficient mice and cfDNA of both WT and *Dnase1*-deficient mice after masking the OCR and CGI fragments suggests that a proportion of these fragment sizes may originate from DNASE1L3 cutting preferences. A potential reason why DNASE1L3 could play a role in these particular fragment sizes is that these fragment sizes could originate from intranucleosomal cutting of methylated DNA. There may be other nucleases that may play a role as well and future studies with double knockout models would further refine the analysis. However, our observations demonstrate that particular cfDNA sizes reflect a fragmentation process that is influenced to methylation.

In this paper, we have been able to deduce the actions and preferences of DNASE1 and DNASE1L3. We have shown not only that nucleases affect the apparent cfDNA methylation level, but also how each nuclease affects it. We have also demonstrated that the cfDNA size profile, which is quintessentially the end product of the fragmentation process, reflects these differential nuclease activities on methylation. Thus, we have shed some light into these fundamental properties of cfDNA.

These findings have been replicated in human cfDNA with *DNASE1L3*-deficiency*.* Homozygous *DNASE1L3*-deficiency in humans results in familial autosomal recessive forms of childhood systemic lupus erythematosus (SLE) and vasculitis (Al-Mayouf et al., 2011; Ozcakar et al., 2013; Carbonella et al., 2017). The loss of DNA self-tolerance with *Dnase1l3* deletion is presumably related to the disrupted clearance of nucleosomes by DNASE1L3 (Sisirak et al., 2016; Napirei et al., 2000). Even in SLE patients that do not have *Dnase1l3-*deficiency, we have previously found that they have an increased proportion of short, hypomethylated cfDNA similar to the profile seen in *Dnase1l3*-deficient patients (Chan et al., 2014). This may be related to a functional aberration in nuclease clearance of nucleosomes; more studies would help clarify the relationship between nuclease activity and the pathogenesis of SLE.

These observations have profound implications for the field of cfDNA. The fragmentation process of cfDNA contributes to the apparent methylation of cfDNA. The nuclease activity in a person could affect the overall cfDNA methylation and result in a false positive testing. Since certain fragment sizes have different methylation levels reflecting different proportions of different genomic regions, it may be advantageous to focus diagnostic testing on certain fragment sizes because of this underlying biology. As cfDNA fragmentomics are an emerging cancer biomarker, a deeper understanding of nuclease effect on cfDNA fragmentation is vital. Ultimately, the combination of size-based and nuclease-based analysis is a powerful approach for investigating cfDNA biology and may have diagnostic applications.

### C. Using methylation in regions to analyze samples

Analyzing certain biological samples using only the methylation level may be difficult. For example, methylation level differences may not be significantly different between samples of subjects having different conditions. The amount of fragments in open chromatin regions (OCRs) and around certain CpG sites in a biological sample may vary depending on certain conditions of the subject. For example, the amount of fragments in OCRs and around CpG sites may differ depending on a cancer classification of the subject in the sample or whether the fragments in a samples are maternal or fetal. Analyzing CpG sites that are putatively methylated or putatively unmethylated may aid in analyzing biological samples to distinguish between different conditions or different tissue types.

### 1. Cancer

Measuring the proportion of fragments in OCR (500 bp upstream and downstream of TSS, H3K27ac, and H3K4me3 markers) and CGI regions results in statistically significant difference comparing cancer and non-cancer. In one embodiment, plasma cfDNA from 8 healthy controls, 17 patients infected with chronic hepatitis B virus (HBV), and 34 patients with HCC were bisulfite sequenced with a median of 38 million paired-end reads (range, 18-65 million).

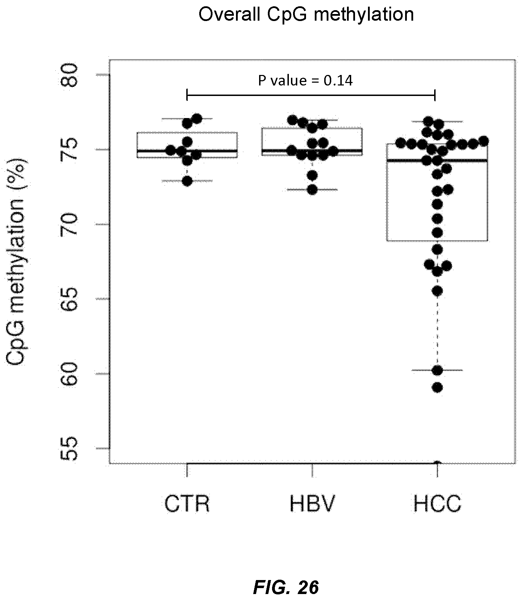

**FIG. 26** shows the overall CpG methylation percentages calculated from all sequenced fragments in the plasma cfDNA of each sample. The X-axis shows the source of the different cfDNA samples: control individuals (CTR), chronic hepatitis B carriers (HBV), and subjects with hepatocellular carcinoma (HCC). Some HCC samples are overall very hypomethylated compared to control; however, as a group the overall CpG methylation is not statistically significant between control and HCC.

On the other hand, in **FIG. 27A****,** the proportion of fragments in OCR and CGI regions are compared between healthy controls and patients with HCC. cfDNA from HCC patients had significantly decreased proportion of OCR and CGI fragments compared to controls (P value = 0.009, Wilcoxon rank-sum test). In **FIG. 27B****,** this trend is also seen in bladder cancer. The X-axis shows the source of different samples: control individuals (CTR), subjects with low-grade non-muscle-invasive bladder cancer (NMIBC_LG), high-grade non-muscle-invasive bladder cancer (NIMBC_HG), and high-grade muscle-invasive bladder cancer (MIBC_HG). The fragments that fall in OCR and CGI regions decrease with severity of bladder cancer, from low-grade non-muscle-invasive bladder cancer (NMIBC_LG) to high-grade non-muscle-invasive bladder cancer (NMIBC_HG) to high-grade muscle-invasive bladder cancer (MIBC_HG). As a group, bladder cancers have significantly few fragments in OCR and CGIs than in controls (P value 0.003, Wilcoxon rank-sum test).

### 2. Fetal vs maternal-specific fragments

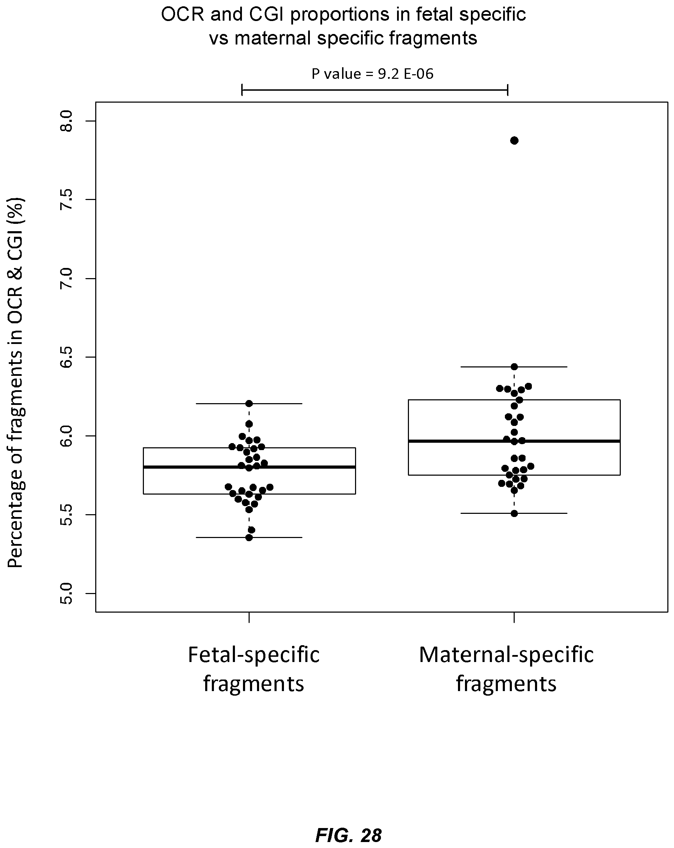

**FIG. 28** shows that the proportions of fetal-specific and maternal-specific fragments in OCR and CGI regions are also significantly different. The X-axis shows fetal-specific fragments and maternal-specific fragments. OCRs was defined as ± 500 bp around the center of TSS, H3K4me3, and H3K27ac regions and were merged with CGI regions. Fetal-specific fragments and maternal-specific fragments from a single plasma sample of a pregnant woman were identified by genotyping and the proportions of fragments in these regions were quantified. In a paired Wilcoxon signed-rank test, fetal-specific fragments had significantly fewer proportion of fragments in OCR and CGI regions than maternal-specific fragments (P value = 9.2 E-06).

### 3. SLE

Autoimmune disease occurs when the body's immune system loses the self-tolerance and mistakenly attacks the cells or tissues of the body itself. Systemic lupus erythematosus (SLE), in particular, is characterized by autoantibodies to double-stranded DNA (dsDNA). Levels of anti-DNA autoantibodies are correlated with disease activity, and the deposition of immune complexes formed by DNA and anti-DNA autoantibodies are associated with the development of lupus nephritis (Soni et al. Current Opinion in Immunology, 2018;55:31-37). Previously, we have observed that the plasma of SLE patients have an increased proportion of short cfDNA, and high resolution analysis on the genomic and epigenetic signatures of plasma DNA has been shown to reflect disease activities of SLE patients (Chan et al. Proc. Natl. Acad. Sci USA 111, E5302-E5311). Plasma cfDNA of patients with SLE may show aberrant genomic representations (copy number changes) that may mimic that of patients with cancer. In the following, we show an example active SLE case with aberrant genomic representation and how analysis using OCR can be useful in distinguishing these aberrant genomic representations from those with cancer.

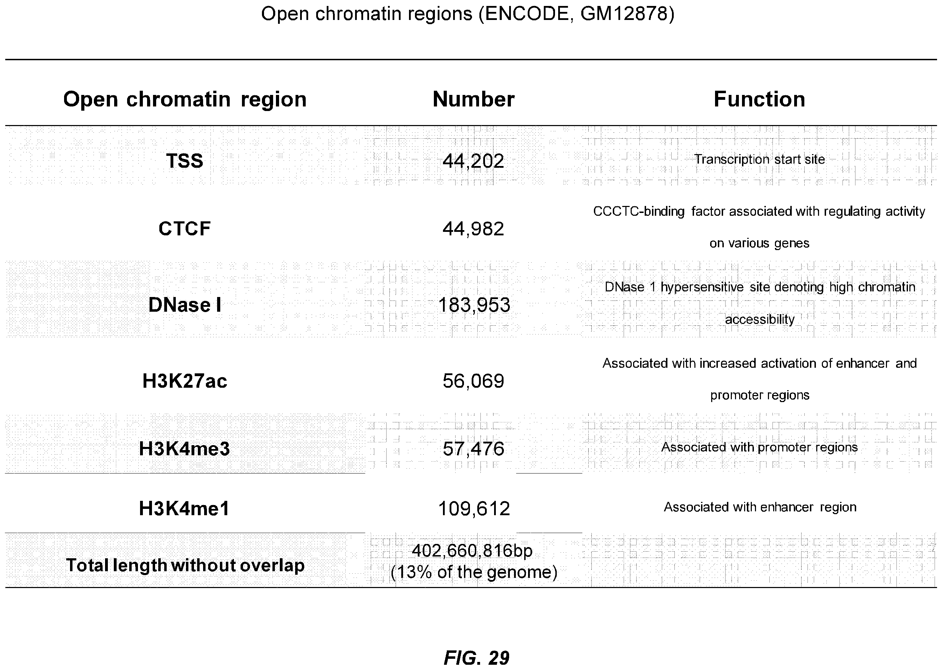

**FIG. 29** is a table showing another set of open chromatin regions that includes transcription start sites (TSS), CCCTC-binding factor (CTCF) sites, DNase1 hypersensitivity sites (DNaseI), and H3K27ac, H3K4me3, and H3K4me1 histone markers. The number of each marker in the human genome is shown with its corresponding function. Altogether this set of OCR comprise of 402,660,816 bp, which is 13% of the genome. As listed in FIG. 29, OCR could be expanded to TSS, CCCTC-binding factor (CTCF) sites, DNase1 hypersensitivity sites (DNaseI), and H3K27ac, H3K4me3, and H3K4me1 histone markers. Altogether this set of OCR comprise of 402,660,816 bp, which is 13% of the genome, by including the flanking 3000 bp of each region. This flanking region could be changed to 1000 bp or 500 bp, or 4000 bp with a tradeoff in sensitivity and specificity.

In SLE, especially active SLE, aberrant genomic representation across the genome is observable in cfDNA **(****FIG. 30).** FIG. 30 is a circos plot showing the genomic representation in 1 Mb bins across the whole genome in a healthy individual (inner layer), inactive SLE patient (middle layer), and an active SLE patient (outer layer). Each dot represents the genomic representation of 1 Mb bin, and colored in red if -3 SD from the mean genomic representation in the group of healthy controls, and colored in green if +3 SD from the mean genomic representation in the group of healthy controls. Active SLE patients have cfDNA with widely divergent genomic distributions.

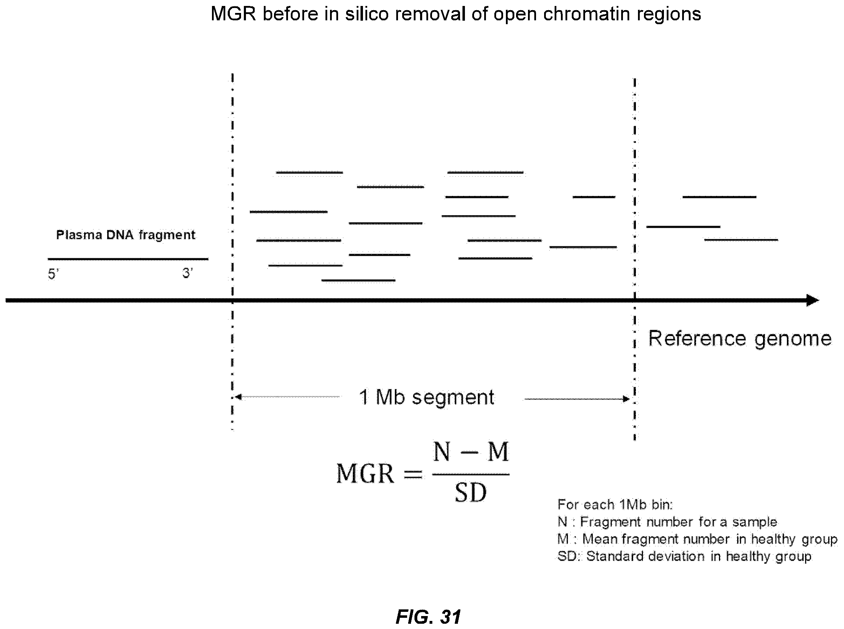

The measured genomic representation (MGR) is shown in **FIG. 31****.** In each 1 Mb bin, the mean and standard deviation of fragment counts in the healthy group was calculated. Then the sample MGR was calculated by a z-score calculation as follows: Mean fragment number (M) of the healthy group is subtracted from the fragment number of a sample (N) divided by the standard deviation (SD)..

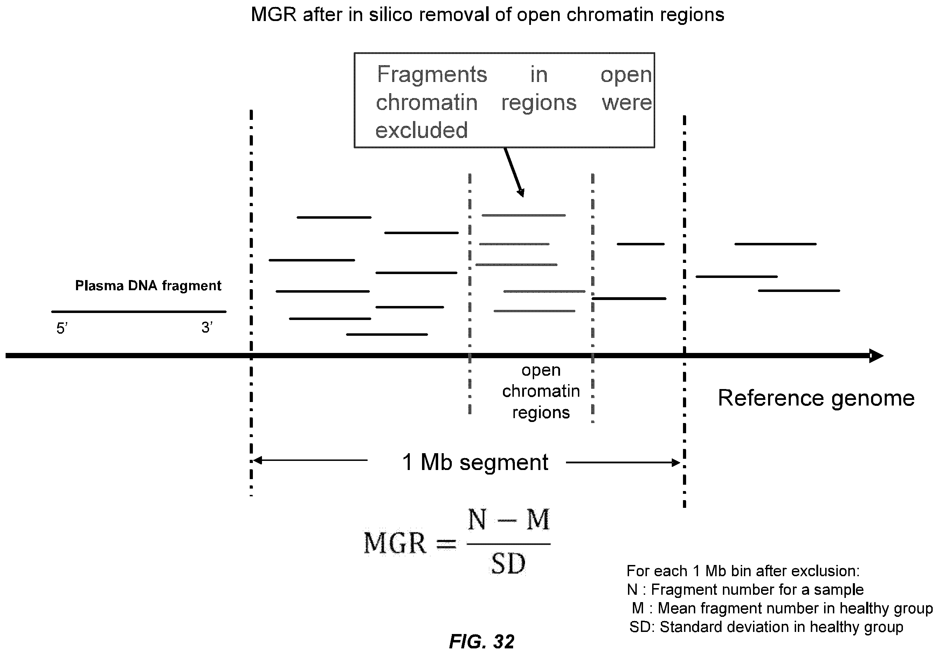

**FIG. 32** shows how bioinformatic masking of the selected OCR was performed. Fragments that fell within the OCRs in FIG. 29 were excluded from the healthy mean and SD calculation and in the final MGR calculation. OCRs are shown in red.

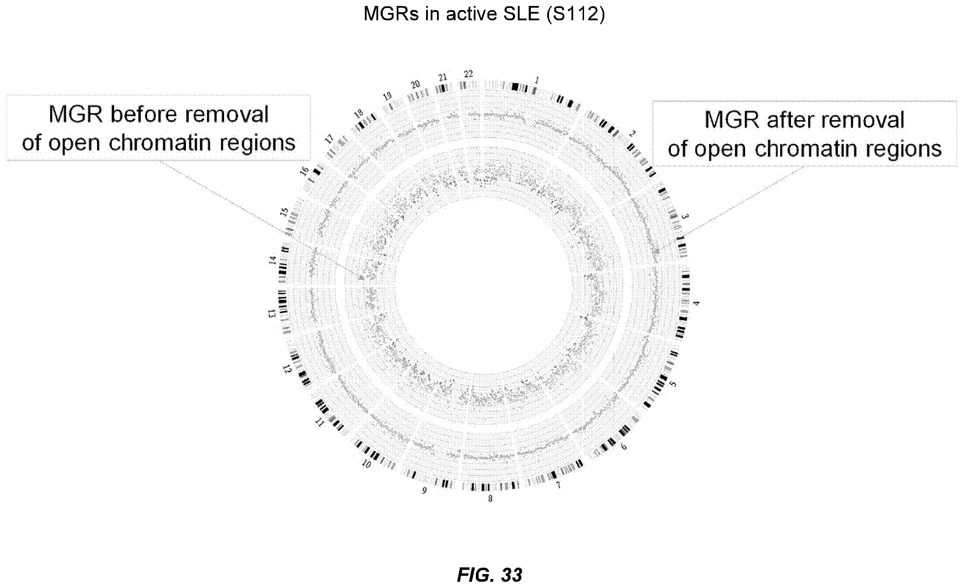

**FIG. 33** is a circos plot showing how the measured genomic representation (MGR) would change after bioinformatically masking the specified open chromatin regions. The inner ring shows the MGR of each bin before bioinformatic masking of the OCRs where there are many aberrant genomic representations. After bioinformatically masking these OCRs, the aberrand genomic representation is diminished.

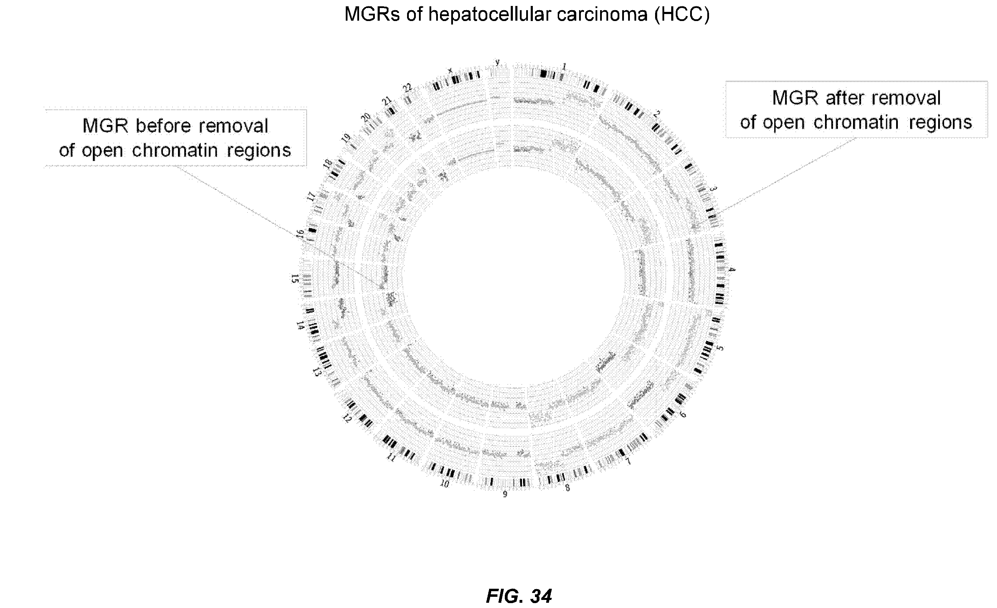

This is in contrast to **FIG. 34****,** which is a circos plot that shows the MGR in a plasma cfDNA sample of a patient with HCC. Copy number changes are also commonly observed in cancer, and in FIG. 34, we can see copy number gains (green) and losses (red) in multiple regions throughout the genome. The inner ring is the MGR before masking the OCRs and the outer ring is the MGR after masking the OCR. Notably, there is not significant change in the MGR and copy number aberrations before or after masking the OCRs.

**FIG. 35** is a boxplot of the percentage of bins with aberrant MGRs (more than +3 SD or less than -3 SD) in healthy controls, SLE, and HCC subjects before and after bioinformatically masking the OCR. The X-axis shows the source of the samples (healthy controls, SLE, HCC subjects) and whether the samples were taken before or after OCR masking. The Y-axis shows the percentage of bins with aberrant MGRs. While there is no significant change in the proportion of aberrant MGRs before and after masking the OCRs in healthy controls and true positive HCC cases, the proportion of aberrant MGRs decrease after masking these regions in SLE. Thus, masking these OCR and CGI regions may be useful in reducing false positives in detecting copy number changes that are often seen in the plasma cfDNA of cancer patients.

### 4. Coverage of putatively methylated or unmethylated CpGs

The end density at putatively methylated or unmethylated CpGs was previously shown to demonstrate the cutting preference of specific nucleases at putatively methylated or unmethylated CpGs. Putatively methylated or unmethylated CpGs were identified from 9 human tissues that underwent whole genome bisulfite sequencing as part of the Roadmap Epigenomics Project. CpG sites what were methylated in ≥ 90% of all fragments in all tissues were considered putatively methylated CpGs, and CpGs that were methylated in ≤ 20% of all fragments in all tissues were considered putatively unmethylated CpGs. Fragments overlapping with either the C or G of the identified CpGs were considered as covering the CpG and included in calculating the coverage.

**FIGS. 36A** and **36B** show the proportion of fragments covering the putatively methylated (FIG. 36A) or unmethylated (FIG. 36B) CpG in plasma cfDNA of 8 healthy controls (CTR), 17 patients infected with chronic hepatitis B virus (HBV), and 34 patients with hepatocellular carcinoma (HCC). The Y-axis shows the proportion of fragments covering the putatively methylated CpG sites in FIG. 36A and covering the putatively unmethylated CpG sites in FIG. 36B. While the coverage over the putatively methylated CpG is not significantly different (P value 0.89, Wilcoxon rank sum test), the coverage over the putatively unmethylated CpG is significantly lower in the plasma cfDNA of patients with HCC compared with CTR (P value 8.4 E-05, Wilcoxon rank sum test).

**FIGS. 37A** and **37B** are boxplots that show the proportion of fragments covering the putatively methylated (FIG. 37A) or unmethylated (FIG. 37B) CpGs in the plasma cfDNA of 14 control, 14 patients with inactive SLE, and 20 patients with active SLE. While the coverage over the putatively methylated CpG is not significantly different (P value 0.57, Wilcoxon rank sum test), the coverage over the putatively unmethylated CpG is significantly lower in the plasma cfDNA of patients with active SLE compared with the healthy controls (P value 0.04, Wilcoxon rank sum test).

**FIGS. 38A** and **38B** are boxplots that show the proportion of fragments covering the putatively methylated or unmethylated CpGs in the fetal-specific vs maternal-specific fragments in the plasma cfDNA of pregnant women. The coverage over the putatively methylated CpG is significantly lower in fetal-specific fragments than in maternal-specific fragments (P value 3.2 E-06, Wilcoxon signed rank test). The coverage over the putatively unmethylated CpG is slightly but not significantly lower in fetal-specific fragments compared with maternal-specific fragments (P value 0.06, Wilcoxon signed rank test).

### D. Example methods

Methods may include using methylation statuses or levels in different ways to analyze a sample. Methylation levels may be determined from only particular sites. For example, the sites may include CpG sites that are all methylated or unmethylated and may include or exclude certain regions. The relative abundance of sequence reads from certain sites that are all hypomethylated or all hypermethylated can be used to analyze a sample. Relative abundance of sequence reads in a sample can be used to diagnose a subject or to determined the fractional concentration of clinically-relevant DNA in the sample. Additionally, copy number aberrations determined in regions excluding open chromatin regions and copy number aberrations determined using other regions may be used to determine whether a subject has a condition. A condition detected by any method may be treated in the subject by any treatment described herein (e.g., Section III).

### 1. Methylation level to analyze biological samples

The methylation levels determined using methylation statuses at certain sites can be used to determine various characteristics of a biological sample or the subject from which the biological sample is obtained. The certain sites used may be CpG sites that are all methylated or all unmethylated. The sites may include or exclude sites in OCRs or CGIs. The methylation levels may used to detect a genetic disorder for a gene associated with a nuclease, to determine an efficacy of a treatment for a blood disorder, or monitoring nuclease activity. Detecting a genetic disorder for a gene associated with a nuclease

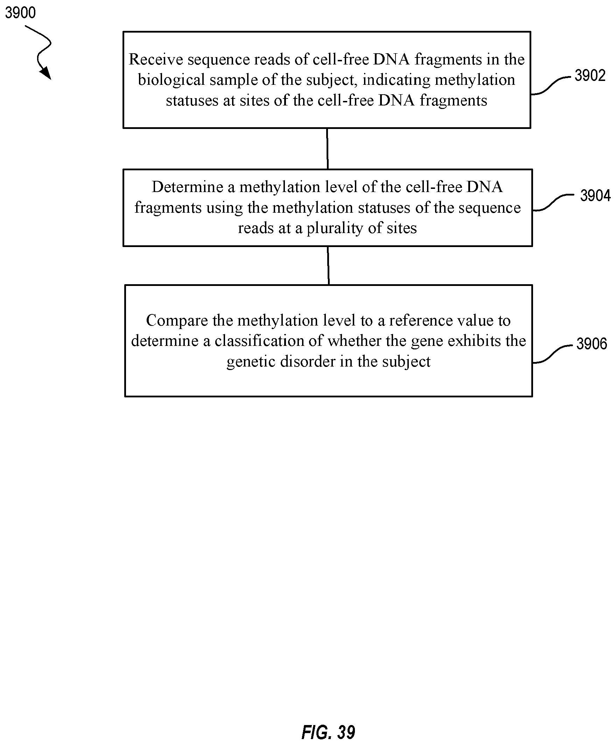

**FIG. 39** shows a flowchart illustrating a method 3900 for detecting a genetic disorder for a gene associated with a nuclease using a biological sample of a subject including cell-free DNA according to embodiments of the present disclosure. Method 3900 and others method herein can be performed entirely or partially with a computer system. The biological sample can be any cell-free DNA sample, e.g., as described herein.

At block 3902, first sequence reads obtained from sequencing first cell-free DNA fragments in a first biological sample of a subject are received. The sequencing may be performed in various ways, e.g., as described herein. The sequencing may be targeted sequencing as described herein. For example, biological sample can be enriched for DNA fragments from a particular region. The enriching can include using capture probes that bind to a portion of, or an entire genome, e.g., as defined by a reference genome.

The sequence reads may indicate methylation statuses at sites of the cell-free DNA fragments. For example, the methylation status at sites of the cfDNA fragments can be interrogated using bisulfate conversion, as described herein. Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by the Pacific Biosciences single molecule real time analysis (Tse et al. Proc Natl Acad Sci U S A 2021; 118: e2019768118).

At block 3904, the methylation statuses of the sequence reads are used to determine a methylation level of the cell-free DNA fragments. The methylation level may be determined using all of the sequence reads or just certain ones that satisfy certain criteria, e.g., location or size. The methylation level may be determined using sequence reads at a plurality of sites. The sites may have specific characteristics, e.g., being CpG sites. The methylation level can be determined from the total number of cytosines not converted after bisulfite treatment (which corresponds to methylated cytosine). For example, at CpG sites methylated sites can be determined as a proportion of all CpG sites covered by sequence reads mapped to a region of interest (e.g., a 100-kb region). This analysis can also be performed for regions with other bin sizes, e.g. 500 bp, 5 kb, 10 kb, 50-kb or 1-Mb, etc. A region could be the entire genome or a chromosome or part of a chromosome (e.g. a chromosomal arm).

At block 3906, the methylation level of the cell-free DNA fragments are compared to a reference value to determine a classification of whether the gene exhibits the genetic disorder in the subject. The reference value may comprise or be used to determine a cutoff or a threshold value. The cutoff or threshold may be derived from a reference value that is representative of a particular classification or discriminates between two or more classifications. A subject with methylation levels above or below the cutoff (threshold) value may be classified as carrying a genetic disorder. The cutoff value may be defined by a statistical metric (e.g., significance, P-value, Z-score) relative to a reference value, e.g., so that the methylation level is statistically different. Alternatively, calibration value(s) may be used as the reference value. For example, methylation level of cfDNA in a calibration sample (whose classification is known) can be used to determine the classification of whether the gene exhibits the genetic disorder in the subject based on the methylation level of the cell-free DNA fragments. The calibration sample may have known methylation levels at certain positions, regions, or the entire genome, as well as having a known classification.

The reference value may be determined in various ways, as will be appreciated by the skilled person. For example, a reference value may be determined from a wildtype animal or a healthy human subject. A reference value may be determined froma tissue-specific sample or a portion of a sample obtained from the same subject (e.g., sequence reads obtained from plasma but at a different time or masked (e.g., for OCR or CGI) or a buffy coat portion of a sample), as shown, for example, in FIG. 1. For example, a presence or an extent of methylation in positions or regions in a genome that are predominantly methylated or unmethylated can be extracted from datasets pertaining to a healthy individual or a population of healthy individuals and be used as a reference value.

The reference value may comprise a plurality of cutoffs or threshold values. Methylation levels may fall between two cutoffs or threshold values, denoting a subtype of the genetic disorder or a level of progression of the genetic disorder. For example, methylation level for two or more different cohorts of subjects with different known classifications can be determined, and a reference value can be selected as representative of one classification (e.g., a mean) or a value that is between two clusters of the metrics (e.g., chosen to obtain a desired sensitivity and specificity).

A presence of a genetic disorder or an extent of such disorder in the subject, based on methylation level of the cell-free DNA fragments comparison to a reference value, may be determined using statistical approaches or machine learning methods for example but not limited to, including logistic regression, support vector machines (SVM), decision tree, CART algorithm (Classification and Regression Trees), naive Bayes classification, clustering algorithm, principal component analysis, singular value decomposition (SVD), t-distributed stochastic neighbor embedding (tSNE), artificial neural network, ensemble methods which construct a set of classifiers and then classify new data points by taking a weighted vote of their prediction, etc.

In some implementations, the cell-free DNA fragments are filtered before determining the methylation level(s) or the classification. for example, only fragments from a certain region (e.g., transcription start sites, RNA polymerase II sites, H3K4me3 marker regions, H3K27ac marker regions, or random regions) may be used to determine the methylation level or consequently the classification of the genetic disorder in the subject.

### a) Determining an efficacy of treatment for a blood disorder

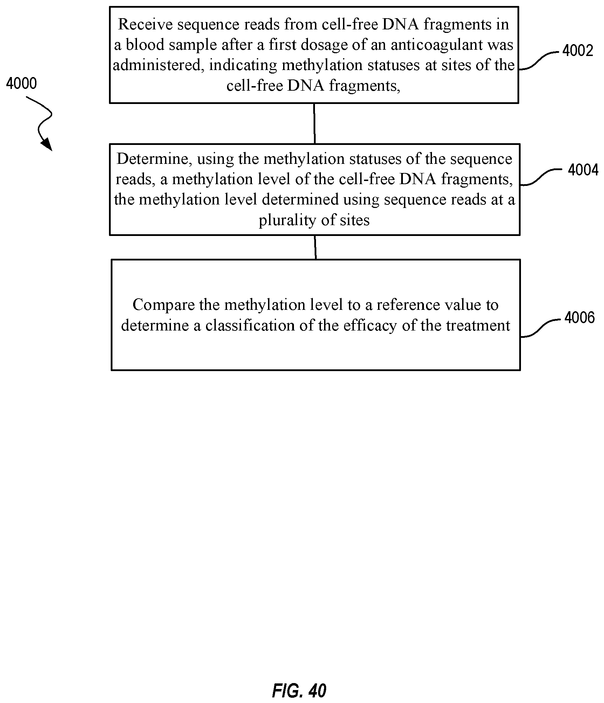

**FIG. 40** shows a flowchart illustrating a method 4000 for determining an efficacy of a treatment of a subject having a blood disorder according to embodiments of the present disclosure. The blood disorder may include predisposition to thromboembolic events. Certain blocks of method 4000 can be performed in a similar manner as blocks of method 3900.

At block 4002, sequence reads obtained from sequencing cell-free DNA fragments in a blood sample of the subject are received. The blood sample is obtained after the subject that was administered a first dosage of an anticoagulant. The anticoagulant can be heparin. The sequence reads may indicate methylation statuses at sites of the cell-free DNA fragments. The blood sample can be obtained from the subject prior to receiving the sequence reads. Consequently, sequencing of the cell-free DNA fragments in the blood sample can be performed to obtain the sequence reads.

At block 4004, the methylation statuses of the sequence reads are used to determine a methylation level of the cell-free DNA fragments. The methylation level may be determined using sequence reads at a plurality of sites. The methylation level may be determined for cell-free DNA fragments that have all CpG sites methylated or unmethylated (e.g., as shown in FIG. 4). A DNA fragment may have one or more CpG sites, and all may be methylated or unmethylated.

At block 4006, the methylation level of the cell-free DNA fragments is compared to a reference value to determine a classification of the efficacy of the treatment. A second dosage of the anticoagulant can be administered to the subject based on the comparison, the second dosage being greater than the first dosage. In other examples, the second dosage can be less than the first dosage, e.g., if the amount overshoots the reference value. Treatments may include hemodialysis, a kidney transplant, or any treatment described herein.

In some embodiments, genomic sites that are located in open chromatin regions or in CpG islands are excluded when determining the methylation level (e.g., as shown in FIGS. 10 and 13). The reference value may be determined using the methylation statuses at the sites that include open chromatin regions or in CpG islands (e.g., as shown in FIGS. 10 and 23). The determined methylation level may correspond to a number of regions that have a methylation density below a specified percentage. For example, as shown in FIG. 15, the methylation level may only be determined in sites that are hypomethylated in a reference genome. In other examples, methylation level may be determined for the cell-free DNA fragments having a specified size (e.g., as shown in FIGS. 11 and 13).

The reference value can correspond to a measurement previously performed in the subject before administering the anticoagulant. The change in the amount from the previous measurement can indicate an efficacy of the dosage of the anticoagulant. In another implementation, the reference value can correspond to the amount measured in a healthy subject. An efficacious dosage can be one that brings the amount to within a threshold of the reference value for the healthy subject. In yet another implementation, the reference value can correspond to the amount measured in a subject that has the blood disorder (e.g., as may be previously measured in the subject before administering the anticoagulant). For example, a reference value may comprise a wildtype animal or a healthy human subject. A reference value may comprise a tissue specific sample or a portion of a sample obtained from the same subject (e.g., sequence reads obtained from plasma or buffy coat portion of a sample), as shown, for example, in FIG. 1.

### b) Monitoring nuclease activity

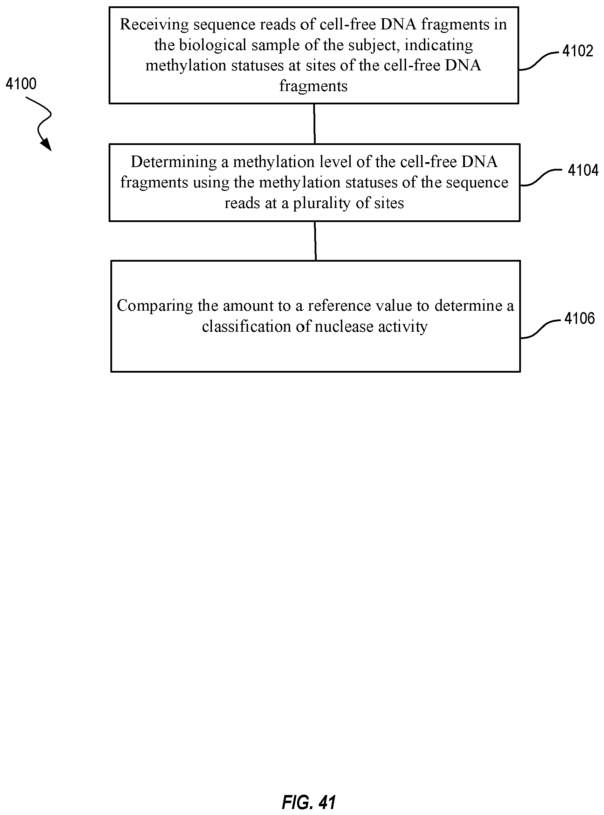

**FIG. 41** shows a flowchart illustrating a method 4100 for monitoring an activity of a nuclease using a biological sample of a subject including cell-free DNA according to embodiments of the present disclosure. Certain blocks of method 4000 can be performed in a similar manner as blocks of method 3900 and 4000.

At block 4102, sequence reads of cell-free DNA fragments in the biological sample of the subject may be received. The sequence reads may indicate methylation statuses at sites of the cell-free DNA fragments. Receiving may be similar to block 3902 or 4002.

At block 4104, the methylation statuses of the sequence reads are used to determine a methylation level of the cell-free DNA fragments. The methylation level may be determined using sequence reads at a plurality of sites. The methylation level may be determined for cell-free DNA fragments that have all CpG sites methylated or unmethylated (e.g., as shown in FIG. 4A-4C).

At block 4106, the methylation level of the cell-free DNA fragments is compared to a reference value to determine a classification of the activity of the nuclease (e.g., as shown in FIG. 1). Some embodiments can be used to monitor the activity of a nuclease, e.g., DFFB, DNASE1, and DNASEIL3. Such activity can be from internal nucleases (i.e., as a natural process of the body) and/or from the result of adding a nuclease, e.g., DNASE1. Such monitoring can be used to determine a change in a genetic disorder for the efficacy of a treatment. For example, DNASE1 can be used to treat a subject. An effect of the treatment or the abnormality in activity of a nuclease (e.g., due to genetic disorder) can be measured by analyzing the methylation level at a site or plurality of sites using cfDNA fragments, as described herein. In some embodiments, DNASE1 (e.g., exogenously added) can be used to treat auto-immune conditions, such as SLE. Depending on the determination of the activity, the dosage of treatment of the nuclease can be changed. The determination of abnormal nuclease activity (e.g., above or below a reference value corresponding to normal/healthy values) can indicate a level of pathology alone or in combination with other factors. The pathology can be cancer. In some embodiments, the classification may be a numerical representation of the nuclease activity (e.g., a measured value of nuclease activity).

### 2. Region-specific sequence read quantification

The proportion of sequence reads from a certain set of CpG sites can be used to analyze a biological sample. The certain set of CpG sites may be CpG sites that are all hypomethylated or hypermethylated in a reference genome. The relative abundance of sequence reads covering these particular sites may differ for samples from subjects with different levels of a condition and for samples from subjects with different nuclease activities.

### a) Differentiating genotypes and phenotypes

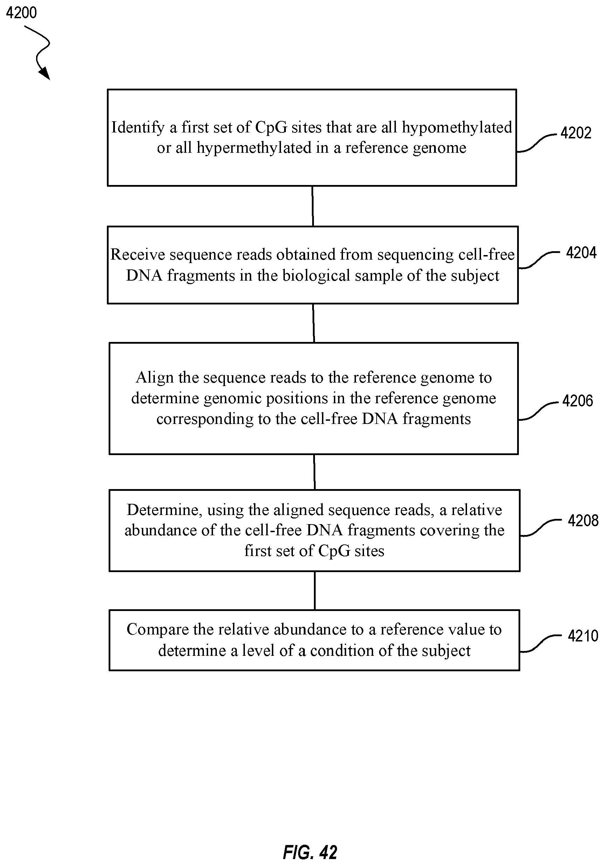

**FIG. 42** shows a flowchart illustrating a method 4200 for analyzing a biological sample of a subject including cell-free DNA according to embodiments of the present disclosure. The biological sample (e.g., whole blood, plasma, etc.), as described herein, can be obtained from the subject and a sequencing of the cell-free DNA fragments in the blood sample can be performed to obtain sequence reads. Certain blocks of method 4200 can be performed in a similar manner as blocks of other methods.

At block 4202, a first set of CpG sites that are all hypomethylated or all hypermethylated in a reference genome can be identified. The reference genome may be obtained from a healthy individual or a population of healthy individuals. The reference genome may contain regions that are predominantly (or putatively) unmethylated or predominantly methylated. The methylation level in the reference genome may be compared to a first threshold. A methylation level below the first threshold may indicate a hypomethylated site (e.g., in FIG. 15A, the first threshold may be set below the median methylation level for wildtype). In other embodiments, the methylation level in the reference genome may be compared to a second threshold. A methylation level above the second threshold may indicate a hypermethylated site (e.g., in FIG. 15B, the second threshold may be set above the median methylation level for wildtype). The threshold may be any threshold described herein. An individual who is not carrying a genetic disorder or a disease of interest may be considered a healthy individual.

At block 4204, sequence reads obtained from sequencing cell-free DNA fragments in the biological sample of the subject are received. Block 4204 may be performed in a similar manner as described in block 3902.

At block 4206, the sequence reads are aligned to the reference genome to determine genomic positions in the reference genome corresponding to the cell-free DNA fragments. For example, a sequence read of the entire DNA fragment (or a pair of reads from the ends, or just one read from one end) can be aligned to the reference genome (e.g., hg19 or other reference) using any one of various alignment tools, such as BLAST, BOWTIE, or SOAP. As part of the alignment, a coordinate for at least one end of the DNA fragment can be determined. In this manner, the coverage (number of reads/fragments) can be determined for just end positions or for any position covered by a DNA fragment. Accordingly, a genomic position in the reference genome may correspond to one end of one or more cfDNA fragments or to other parts of one or more cfDNA fragments.

At block 4208, a relative abundance of the cell-free DNA fragments covering the first set of CpG sites is determined by using the aligned sequence reads. The relative abundance may be determined in various ways. For example, the relative abundance may comprise a percentage of putatively methylated (or hypermethylated) or unmethylated (or hypomethylated) CpG sites in the reference genome that may be covered by cfDNA fragments (e.g., as shown in FIG. 8, FIG. 27A, or FIG. 27B). In another embodiment, with a condition involving a genetic disorder associated with nuclease activity, the relative abundance may comprise a normalized end density, as described herein (e.g., as shown in FIG. 6A-6E).

At block 4210, the relative abundance is then compared to a reference value to determine a level of a condition of the subject. A reference value may comprise the level of abundance determined using sequence reads from a biological sample from a healthy individual. The relative abundance of cfDNA fragments that cover positions that map to a CpG site in the first set of CpG sites may be different (e.g., significantly lower, or significantly higher) than the reference value for relative abundance. The observed difference may be used to determine a level or classification of a condition of the subject. The condition may comprise enzyme deficiencies. The condition may comprise a cancer (e.g., as shown in FIGS. 27A and 27B and FIGS. 36A and 36B). The condition may be an auto-immune disease. The condition may be caused by a genetic disorder. The gene that exhibits the genetic disorder may be associated with a nuclease (e.g., as shown in FIGS. 6A-6E and 8). The condition may comprise an auto-immune disease (e.g., as shown in FIGS. 37A and 37B).

### b) Monitoring nuclease activity

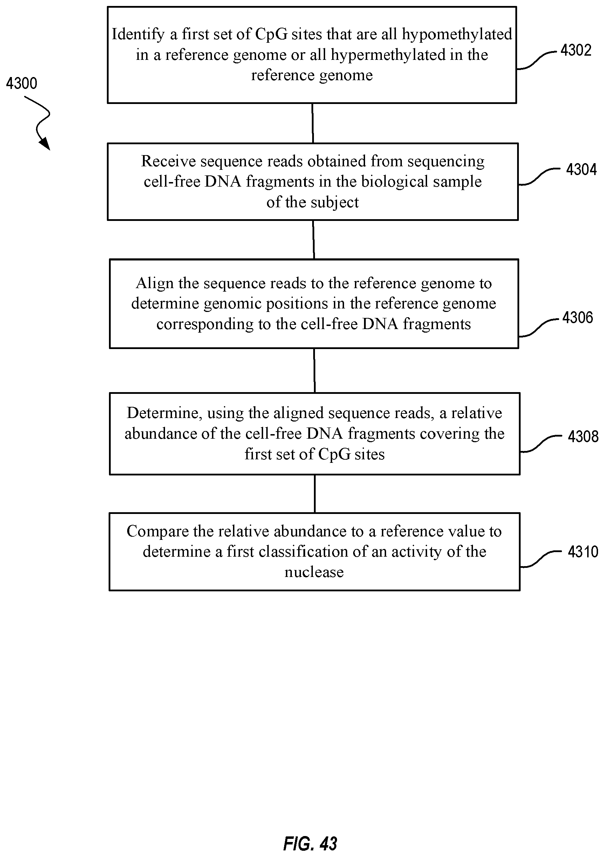

**FIG. 43** shows a flowchart illustrating a method 4300 for monitoring activity of a nuclease using a biological sample of a subject including cell-free DNA according to embodiments of the present disclosure. The biological sample (e.g., whole blood, plasma, etc.), as described herein, can be obtained from the subject and a sequencing of the cell-free DNA fragments in the sample can be performed to obtain sequence reads. Certain blocks of method 4300 can be performed in a similar manner as blocks of other methods.

At block 4302, a first set of CpG sites that are all hypomethylated or all hypermethylated in a reference genome can be identified. The reference genome may be obtained from a healthy individual or a population of healthy individuals. The reference genome may contain CpG sites that are predominantly unmethylated or predominantly methylated. An individual who is not carrying a genetic disorder or a disease of interest may be considered a healthy individual. Block 4302 may be performed in a similar manner as block 4202.

At block 4304, sequence reads obtained from sequencing cell-free DNA fragments in the biological sample of the subject are received.

At block 4306, the sequence reads are aligned to the reference genome to determine genomic positions in the reference genome corresponding to the cell-free DNA fragments. Block 4306 may be performed in a similar manner as block 4206.

At block 4308, a relative abundance of the cell-free DNA fragments covering the first set of CpG sites is determined by using the aligned sequence reads, as described in block 4208. The relative abundance may be a number of DNA molecules covering the first set of CpG sites normalized against the number of DNA molecules analyzed.

In another example, the relative abundance may be a percentage of cfDNA fragments covering a site (e.g., OCRs or CGIs) as shown, for example, in FIG. 22. In another embodiment, the relative abundance of the cell-free DNA fragments may be a statistical value of a size distribution of the cell-free DNA fragments at the first set of CpG sites (e.g., as shown in FIG. 7). The statistical value may be a size ratio of a first amount of the cell-free DNA fragments covering the first set of CpG sites having a first size relative to a second amount of the cell-free DNA fragments covering the first set of CpG sites having a second size.

The relative abundance may be an end density of sequence reads covering the first set of CpG sites. The biological sample obtained from the subject may contain greater or less cfDNA fragments that are enzymatically cleaved at CpG sites that are predominantly hypermethylated, hypomethylated, or unmethylated.

The relative abundance of the cell-free DNA fragments may be determined for the cell-free DNA fragments having a specified size (e.g., as shown in FIG. 12). The specified size may comprise 5 base-pairs (bp), 10 bp, 50 bp, 100 bp, 200, bp, 500 bp, 1000 bp or more or other sizes in between. The specified size may be a size range. For example, the specified size may comprise a range from about 5-100 bp, 70-150 bp, 50-500 bp, 5-50 bp, from about 100-500 bp, from about 100-1000 bp, or other ranges.

At block 4310, the relative abundance is then compared to a reference value to determine a first classification of an activity of the enzyme. A reference value may comprise the level of abundance determined using sequence reads from a biological sample from a healthy individual. Activity of an enzyme (e.g., a nuclease such as DFFB, DNASE1, or DNASE1L3) can be classified and used to determine a genetic disorder or a change in a genetic disorder for the efficacy of a treatment as describe in 4104. The level of the condition may be determined based at least in part on a genetic disorder, where the gene is associated with a nuclease (e.g., as shown in FIGS. 6A-6E and 8). The determination of abnormal enzyme activity (e.g., above or below a reference value corresponding to normal/healthy values) can indicate a level of pathology alone or in combination with other factors. The pathology can be cancer.

### 3. Fractional concentration

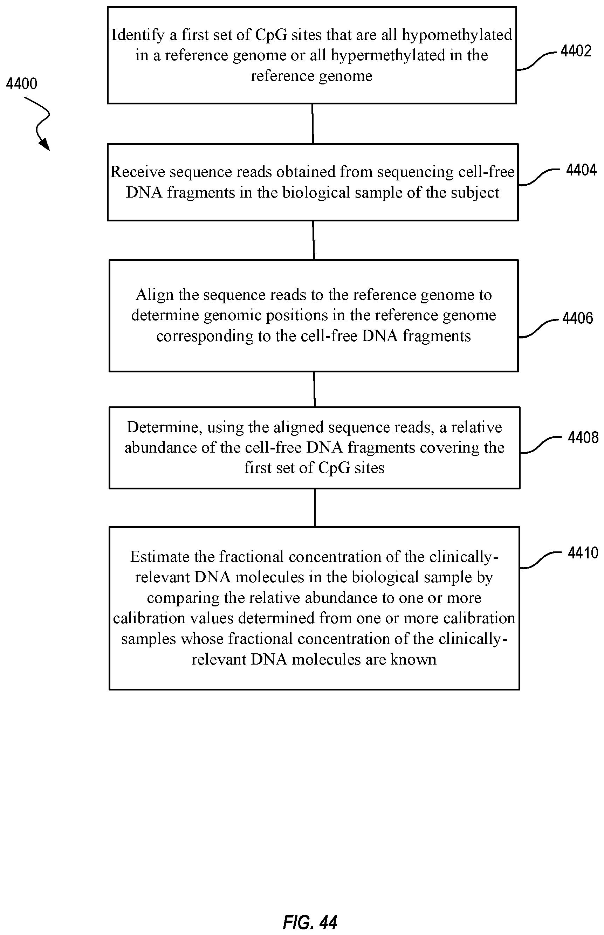

**FIG. 44** shows a flowchart illustrating a method 4400 for estimating a fractional concentration of clinically-relevant DNA molecules in a biological sample of a subject according to embodiments of the present disclosure. The biological sample may include a mixture of cell-free DNA molecules from a plurality of tissue types. For example, the biological sample may be obtained from a pregnant woman comprising maternal cfDNA molecules and fetal cfDNA molecules (e.g., as shown in FIG. 28 and FIGS. 38A and 38B). The biological sample may comprise tumor specific cfDNA molecules as well as other tissue-specific cfDNA molecules. The clinically-relevant DNA molecules may comprise fetal DNA. In other embodiments, the clinically-relevant DNA can be tumor DNA or transplant DNA. Certain blocks of method 4400 can be performed in a similar manner as blocks of other methods.

At block 4402, a first set of CpG sites that are all hypomethylated or all hypermethylated in a reference genome can be identified. The reference genome may be obtained from a healthy individual or a population of healthy individuals and/or nonpregnant subjects. The reference genome may contain positions or regions that are predominantly unmethylated or predominantly methylated. An individual who is not carrying a genetic disorder or a disease of interest may be considered a healthy individual. The sites may be identified as described with block 4202.

At block 4404, sequence reads obtained from sequencing cell-free DNA fragments in the biological sample of the subject are received.

At block 4406, the sequence reads are aligned to the reference genome to determine genomic positions in the reference genome corresponding to the cell-free DNA fragments. Block 4406 may be performed in a similar manner as block 4206.

At block 4408, a relative abundance of the cell-free DNA fragments covering the first set of CpG sites is determined by using the aligned sequence reads. The relative abundance may comprise a percentage of fragments covering putatively methylated or unmethylated sites (e.g., OCR or CpG (or CGI) sites) (e.g., as shown in FIG. 28 or FIG. 38A and 38B).

At block 4410, a fractional concentration of the clinically-relevant DNA molecules in the biological sample can be estimated by comparing the relative abundance to one or more calibration values determined from one or more calibration samples whose fractional concentration of the clinically-relevant DNA molecules are known. As shown in FIG. 28 and FIG. 38A and 38B, the fetal and maternal DNA have difference relative abundances. A sample having a mixture of both will have a relative abundance that depends on the proportion of fetal/maternal DNA in the sample. The fractional concentration for a calibration sample can be determined in other ways, e.g., using a locus on a Y chromosome for a male fetus or a fetal-specific marker (e.g., an allele inherited from the father or a fetal-specific epigenetic marker).

Calibration data points can include a relative abundance and a measured/known fraction of the clinically-relevant DNA. The comparison can involve comparing to a calibration curve (composed of the calibration data points), and thus the comparison can identify the point on the curve having the measured relative abundance for the test sample. The fractional concentration corresponding to the identified point can then be used to estimate the fractional concentration. For example, the relative abundance can be provided as an input to the calibration function (e.g., a linear or non-linear fit) to obtain an output of the fractional concentration.

### 4. Determining a condition

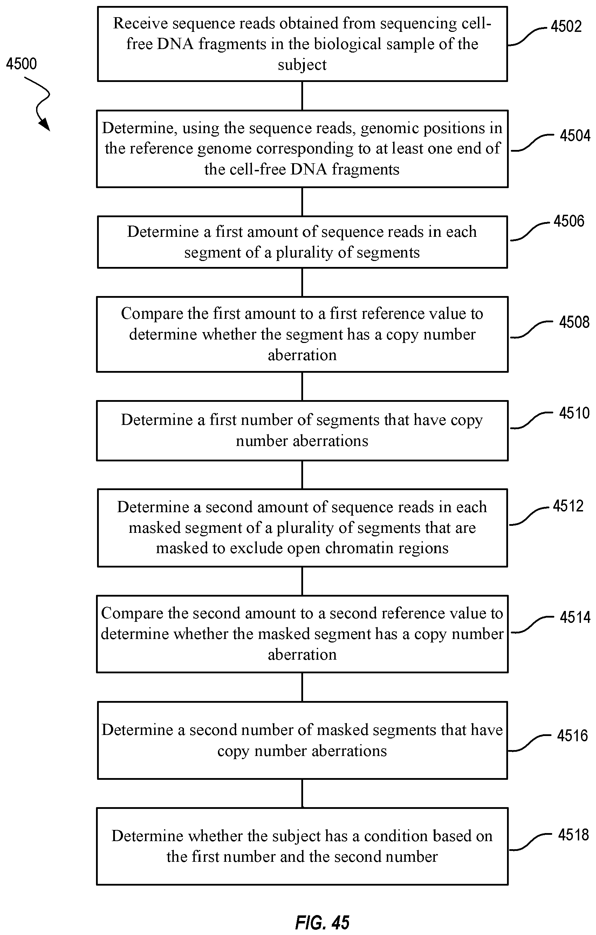

**FIG. 45** shows a flowchart illustrating a method 4500 for analyzing a biological sample of a subject including cell-free DNA according to embodiments of the present disclosure. Certain blocks of method 4500 can be performed in a similar manner as blocks of other methods

At block 4502, sequence reads obtained from sequencing cell-free DNA fragments in a blood sample of the subject are received. The biological sample (e.g., whole blood, plasma, etc.), as described herein, can be obtained from the subject and a sequencing of the cell-free DNA fragments in the sample can be performed to obtain sequence reads.

At block 4504, genomic positions in the reference genome corresponding to at least one end of the cell-free DNA fragments are determined using the sequence reads.

At block 4506, a first amount of sequence reads in each segment of a plurality of segments are determined. For example, the reference genome may be divided to segments (or bins) with a specific size. As examples, the segment or bin size may be about 10 kb, 50 kb, 100 kb, 500 kb, 1 Mb or more. The segment size may be between any two sizes mentioned here. The frequency or copy numbers of sequence reads corresponding to a segment may be determined. In other embodiments, the amount can be a statistical value of a size distribution of the DNA fragments for that segment. Other properties can also be used, e.g., a methylation level for the region.

At block 4508, the first amount is compared to a first reference value to determine whether the segment has a copy number aberration. The reference value may comprise an amount of sequence reads obtained from a healthy individual that would correspond to each segment. In a segment, a difference between the amount of sequence reads from a sample obtained from the subject and the reference value may denote a copy number aberration. For example, measured genomic representation (e.g., as shown in FIG. 31 - FIG. 35), can be used.

At block 4510, a first number of segments that have copy number aberrations are determined. For a plurality of segments, the processes mentioned in blocks 4506 and 4508 can be repeated to determine a first number of segments that have copy number aberrations.

At block 4512, a second amount of sequence reads in each masked segment of a plurality of segments that are masked to exclude open chromatin regions. The masking may comprise in silico masking (e.g., using bioinformatic methods to exclude a segment). Certain regions can be masked, e.g., as shown in FIG. 29.

At block 4514, the second amount is compared to a second reference value to determine whether the masked segment has a copy number aberration. The second reference value may comprise an amountof sequence reads obtained from a healthy individual that are in the masked segment. A difference in the amount of reads for a masked segment obtained from the subject and the reference value (for the same segment) can be used to determine a copy number aberration.

At block 4516, steps described in blocks 4510 and 4512 can be repeated for one or more masked segments to determine a second number of masked segments that have copy number of aberrations. Accordingly, a measured genomic representation before and after masking, described herein (e.g., as shown in FIG. 31 - FIG. 35), can be used.

At block 4518, a condition of a subject is determined based at least on the first number and the second number. The first number and the second number can be used in a variety of ways. For example, a difference between the two numbers and/or an analysis of the individual numbers can be performed. For example, an initial classification can be made that a condition exists using the first number (e.g., that an auto-immune or a cancer exists), e.g., using a cutoff of a few percentage of gins (e.g., 3 or 5 %). Then, the second number can be used to determine the specific type of condition, e.g., whether it an auto-immune or cancer, e.g., a cutoff of about 25% can distinguish between SLE and cancer. Thus, the condition may be an auto-immune disease, e.g., SLE. For example, a percentage of bins with aberrant MGRs determined before masking (as described at blocks 4504-4510) and after masking (as described at blocks 4512-4516) can be used to determine a condition (e.g., SLE, or HCC) in a subject.

### II. NUCLEASE EFFECT ON EXTRACHROMOSOMAL CIRCULAR DNA

Cell-free DNA (cfDNA) molecules are present in plasma either in linear or circular forms (T. Paulsen, P. Kumar, M. M. Koseoglu, A. Dutta, Discoveries of Extrachromosomal Circles of DNA in Normal and Tumor Cells. Trends Genet. 34, 270-278 (2018), Y. M. D. Lo, D. S. C. Han, P. Jiang, R. W. K. Chiu, Epigenetics, fragmentomics, and topology of cell-free DNA in liquid biopsies. Science (80-. ). 372, eaaw3616 (2021)). A subset of mitochondrial genomes of certain tissues-of-origin exhibited as circular forms in plasma (M.-J. L. Ma, et al., Topologic Analysis of Plasma Mitochondrial DNA Reveals the Coexistence of Both Linear and Circular Molecules. Clin. Chem., clinchem.2019.308122 (2019)). Additionally, cell-free extrachromosomal circular DNA (eccDNA) was also detectable in plasma of pregnant women, normal subjects and patients with cancer, albeit of lower abundance than their linear counterparts (S. T. K. Sin, et al., Identification and characterization of extrachromosomal circular DNA in maternal plasma. Proc. Natl. Acad. Sci. U. S. A. 117, 1658-1665 (2020), J. Zhu, et al., Molecular characterization of cell-free eccDNAs in human plasma. Sci. Rep. 7, 10968 (2017), P. Kumar, et al., Normal and Cancerous Tissues Release Extrachromosomal Circular DNA (eccDNA) into the Circulation. Mol. Cancer Res. 15, 1197-1205 (2017)).

In contrast to the linear cfDNA with one predominant peak at 166-bp, size profiles of eccDNA in plasma exhibited two major peak clusters with summits at around 202- and 338-bp and sharp 10-bp periodicities within both clusters, reflecting possible involvements of nucleosomal structures (Sin et al., PNAS (2020)). Fetal-specific eccDNA molecules were detectable in the maternal plasma of pregnant women, which were shorter and less methylated than the maternal ones (Sin et al., PNAS (2020)), S. T. K. Sin, et al., Characteristics of Fetal Extrachromosomal Circular DNA in Maternal Plasma: Methylation Status and Clearance. Clin. Chem. 67 (2021)). Therefore, the biological properties of eccDNA molecules might be dependent on their tissues-of-origin.

The fragmentation of linear cfDNA is a non-random process. Multiple lines of evidence suggested that such fragmentation patterns could be linked to the activity of various nucleases (D. S. C. Han, Y. M. D. Lo, The Nexus of cfDNA and Nuclease Biology. Trends Genet. 0 (2021)). For instance, it has been reported that deoxyribonuclease 1 like 3 (DNASE1L3) contributes to cell-free DNA fragmentation and preferentially generates fragments with CC-ends both in mouse and in human (Serpas et al., PNAS (2019), R. W. Y. Chan, et al., Plasma DNA Profile Associated with DNASE1L3 Gene Mutations : Clinical Observations , Relationships to Nuclease Substrate Preference , and In Vivo Correction. Am. J. Hum. Genet., 1-13 (2020)). Han et al. systematically studied the effects of deoxyribonuclease 1 (DNASE1), DNASE1L3, and DNA fragmentation factor subunit beta (DFFB) on cell-free DNA fragmentation and found that these enzymes act on DNA degradation during cell apoptosis in a stepwise manner (D. S. C. Han, et al., The Biology of Cell-free DNA Fragmentation and the Roles of DNASE1, DNASE1L3, and DFFB. Am. J. Hum. Genet. 106, 202-214 (2020)). In addition, fragmented cfDNA was undetectable in mice with double deletion of Dnase1l3 and Dffb (T. Watanabe, S. Takada, R. Mizuta, Cell-free DNA in blood circulation is generated by DNase1L3 and caspase-activated DNase. Biochem. Biophys. Res. Commun. 516, 790-795 (2019)). It is therefore important to find out if certain nucleases might also play roles in the generation and/or degradation of eccDNA in plasma.

Herein, knockout mouse models were used to explore whether nucleases such as DNASE1L3 and DNASE1 would affect the biological properties of plasma eccDNA. By comparing the extents of eccDNA size shifts between plasma and tissue eccDNA in mice deficient in either nuclease, the ability of these nucleases to act on eccDNA in intracellular or extracellular manners were analyzed. Furthermore, by applying a mouse pregnancy model, the effects of extracellular DNASE1L3 on cell-free eccDNA were examined. Further evidence of nuclease effects on eccDNA in human was provided by comparing the cell-free eccDNA profiles between *DNASE1L3*-mutated patients and healthy controls.

Overall, *Dnase1l3* deletion can lengthen eccDNA in plasma. EccDNA size profiles of mouse tissues were seemingly not affected by *Dnase1l3* deletion, suggesting that the size alterations of cell-free eccDNA by DNASE1L3 can be related to the degradation instead of the generation of eccDNA. Such mechanistic insight was further highlighted by data from a mouse pregnancy model that the extracellular DNASE1L3 released by the fetuses can digest maternal cell-free eccDNA. Notably, human subjects with *DNASE1L3* deficiency exhibited longer size distributions than healthy controls, which was consistent with the effects of *Dnase1l3* deficiency in mice. The methods provided herein can use cell-free eccDNA as biomarkers for *DNASE1L3* deficiency-related diseases, such as systemic lupus erythematosus and certain types of cancer. The experimental design, materials and methods, and results are described in more details herein.

### A. Experimental Design and Results

Knockout mouse models were employed to investigate the effects of deoxyribonuclease 1 (DNASE1) and deoxyribonuclease 1 like 3 (DNASE1L3) on the characteristics of plasma extrachromosomal circular DNA (eccDNA). The plasma eccDNA counts were found to be elevated in *Dnase1l3*^{*-*/*-*} mice when compared to wild-type mice, with no significant change observed in *Dnase1*^{*-*/-} mice. The cell-free eccDNA in *Dnase1l3*^{-/-} mice exhibited larger size distributions than those of wild-type mice. Notably, such size alterations were not found in tissue eccDNA of either *Dnase1*^{-/-} or *Dnase1l3*^{-/-} mice. These data suggest that DNASE1L3 may digest cell-free eccDNA extracellularly. Intracellular eccDNA may be digested at a lower rate than cell-free eccDNA. This may be partially due to accessibility of the extracellular eccDNA compared to the intracellular eccDNA. Profiling plasma eccDNA in a mouse pregnancy model showed that in *Dnase1l3*^{-/-} mice pregnant with *Dnase1l3*^{*+*/*-*} fetuses, the eccDNA in the maternal plasma was shortened compared with that of *Dnase1l3*^{-/-} mice carrying *Dnase1l3*^{*-*/*-*} fetuses. Therefore, DNASE1L3 released from the *Dnase1l3*^{*+*/*-*} fetuses into the maternal blood circulation can have systemic activity. This pregnancy model highlighted that circulating DNASE1L3 could degrade the maternal eccDNA molecules in a cell-extrinsic manner. Furthermore, plasma eccDNA in human subjects with *DNASE1L3* loss-of-function mutations also exhibited longer size distributions compared to that of control subjects (e.g., healthy individuals).

### 1. Study design

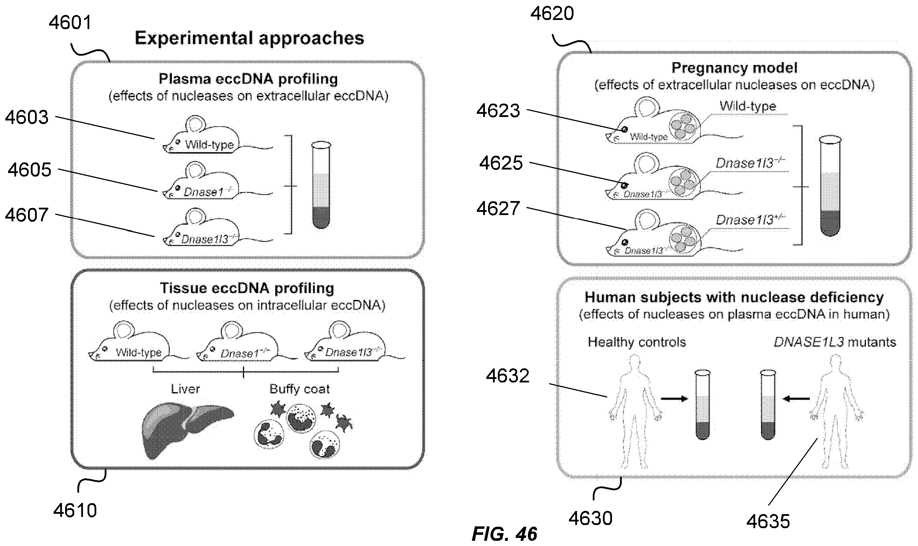

**FIG. 46** illustrates a schematic of the conceptual design of this study. It was investigated whether nucleases (e.g., DNASE1 and DNASE1L3) would have any effects on eccDNA characteristics regarding quantities and size distributions. Firstly, at stage 4601, plasma eccDNA molecules from wild-type mice 4603, *Dnase1*^{*-*/*-*} mice 4605, and *Dnase1l3*^{-/-} mice 4607 were identified. Normalized counts and size profiles of eccDNA were compared among these three groups of mice. Secondly, at stage 4610, to explore whether nucleases act on eccDNA molecules inside live cells, the cellular eccDNA from the three groups of mice in two tissue types: the liver and buffy coat were profiled. Such comparisons were performed in both plasma and tissue (the liver and buffy coat) eccDNA amongst the three groups of mice to elucidate whether the nuclease effects on eccDNA, if any, were exerted extracellularly or intracellularly. To further interrogate the extracellular effects of nucleases on eccDNA, a pregnancy model 4620 was applied to examine whether DNASE1L3 released by the heterozygous (*Dnase1l3*+/-) fetuses would have any impacts on the cell-free eccDNA in maternal plasma of wild-type mice with a wild-type fetus 4623, *Dnase1l3*^{-/-} mice with homozygous fetus 4625, and *Dnase1l3*^{-/-}mice with heterozygous fetus 4627. Lastly, at stage 4630, nuclease effects on cell-free eccDNA were further evaluated in human subjects by comparing eccDNA characteristics between healthy controls 4632 and subjects with DNASE1L3 loss-of-function mutations 4635.

### 2. Amounts of eccDNA in mouse plasma

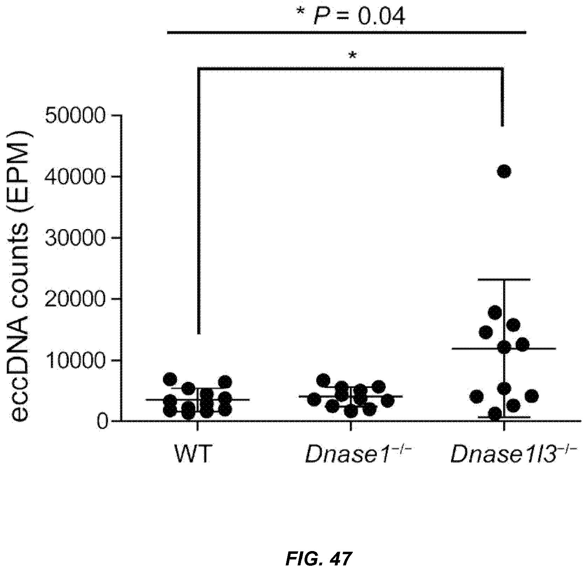

The frequency or amount of eccDNA may indicate whether a gene exhibits a genetic disorder. Plasma eccDNA libraries with a median of 17,463,304 paired-end reads (range: 11,845,852-27,836,098) were sequenced using the tagmentation-based eccDNA library preparation protocol as previously described (4), and identified a median of 15,337 eccDNA loci (range: 3,309-94,248). **FIG. 47** shows a plot for the amounts of plasma eccDNA molecules identified among the three groups of mice. The three groups of mice represented on the x-axis included 12 wildtype (WT) mice (group on the left side of the plot closest to the y-axis), 11 *Dnase1*^{-/-} mice (group in the middle), and 11 *Dnase1l3*^{*-*/*-*} mice (group on the right side of the plot). The amounts, shown on the y-axis, were normalized by the number of mappable reads for each sample, denoted as eccDNA per million mappable reads (EPM) values. The EPM values of *Dnase1l3*^{*-*/*-*} mice were significantly higher (median: 12,206; range: 1,241-40,897) than those of wild-type mice (median: 3,056; range: 1,404-6,952) (P = 0.04, Kruskal-Wallis test). No statistically significant difference was observed between wild-type and *Dnase1*^{-/-} mice. The lack of a statistically significant difference between wild-type and *Dnase1*^{-/-} mice may be a result of other nucleases having similar roles as DNASE1 in digesting DNA molecules remaining present. FIG. 47 shows that a frequency or an amount of eccDNA can be used to determine whether a gene exhibits a genetic disorder.

### 3. EccDNA size distributions in mouse plasma

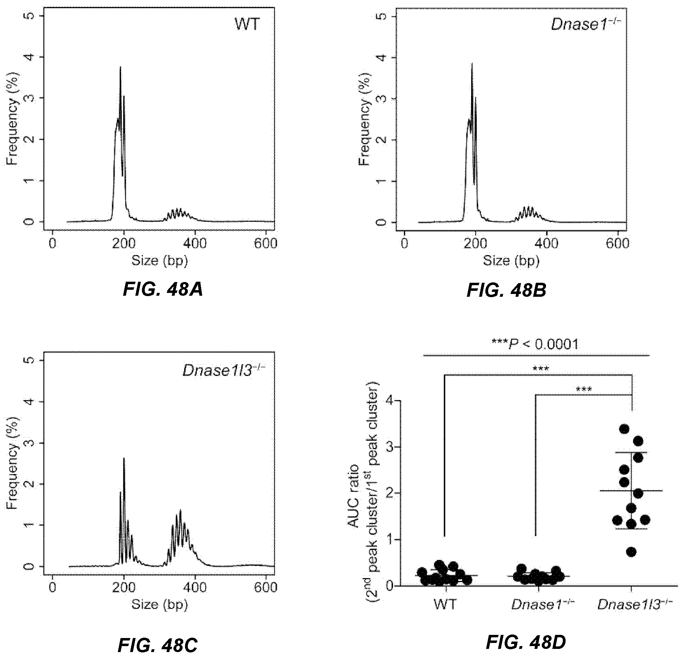

Size distributions of eccDNA can distinguish between organisms with and without certain nuclease deficiencies. Plasma eccDNA molecules were pooled into three groups according to the mouse genotypes. Their size frequencies (Y-axis) are plotted in **FIGS. 48A-48C** with the eccDNA fragment size is shown on the X-axis. Plasma eccDNA from all three groups of mice showed bimodal size distributions with summits at around 200 bp (1^{st} peak cluster) and 350 bp (2^{nd} peak cluster). Compared to wild-type mice, *Dnase1l3*^{*-*/*-*} mice showed a reduction of the 1^{st} peak cluster (150-250 bp) and an enhancement of the 2^{nd} peak cluster (300-450 bp). However, no such difference was observed between *Dnase1*^{*-*/*-*} and wild-type mice. The values of area under the size profile curve (AUC) for the two peak clusters were calculated for the three groups of mice.

**FIG. 48D** plots the AUC ratios (2^{nd} peak cluster: 1^{st} peak cluster) (Y-axis) of the three groups of mice (X-axis). FIGS. 48A-48D shows that that *Dnase1l3*^{-/-} mice had significantly higher AUC ratios than wild-type and *Dnase1*^{-/-} mice. The AUC ratio was calculated as a way to better show the overall size distribution features of eccDNA. The higher the AUC ratio, the longer the overall sizes of eccDNA. For example, the AUC ratio for the *Dnase1l3*^{*-*/*-*} group is greater than one, while the AUC ratios for wild type and *Dnase1*^{-/-} are less than one. The *Dnase1l3*^{-/-} group has statistically longer sizes than the other two gropus.

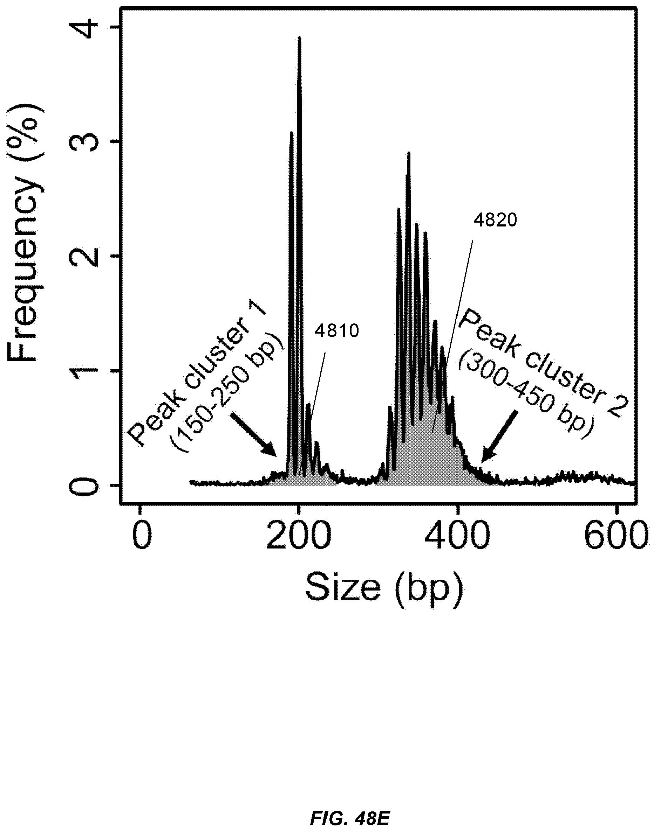

**FIG. 48E** illustrates an example for determining an AUC value for a first peak cluster 4810 and a second peak cluster 4820. FIG. 48E shows size of eccDNA fragment size in base pairs on the X-axis. Frequency of each fragment size is show in the Y-axis. The area under each peak cluster is highlighted. The graph may be integrated to determine the area under the curve for each peak cluster. As shown in FIG. 48D, ratios of the areas can be calculated.

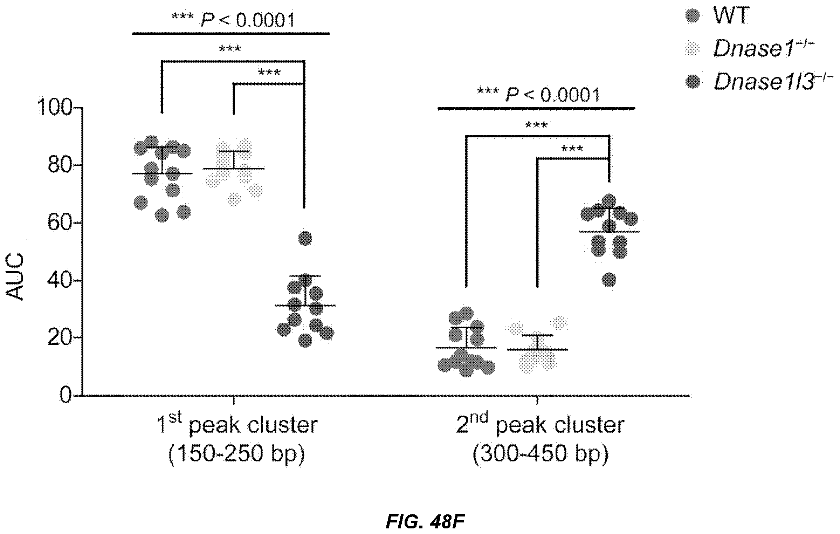

**FIG. 48F** shows the AUC values of the 1^{st} peak cluster (150-250 bp) and the 2^{nd} peak cluster (300-450 bp) for the three groups of mice (Wild type "WT", *Dnase1*^{*-*/}*⁻,* and *Dnase1l3*^{-/-})*.* The X-axis shows the two peak clusters. The Y-axis shows the AUC. In each peak cluster graph, data from the wild type group is shown on the left, the *Dnase1*^{-/-} group is shown in the middle, and the *Dnase1l3*^{-/-} group is shown on the right. FIG. 48F showed a reduction of the 1^{st} peak cluster and an enhancement of the 2^{nd} peak cluster of *Dnase1l3*^{-/-} mice (AUC_{1st peak cluster}: median 30.3%, range 19.0-54.6%; AUC_{2nd peak cluster}: median 58.9%, range 40.4-67.7%), which were statistically significant in comparison with the wild-type mice (AUC_{1st peak cluster}: median 77.9%, range 62.7-88.1%; AUC_{2nd peak cluster}: median 12.8%, range 8.9-28.5%) (*P*_{1st peak cluster} < 0.0001, *P*_{2nd peak cluster} < 0.0001; Kruskal-Wallis test).

In summary, these data indicated that the plasma eccDNA molecules of *Dnase1l3*^{*-*/*-*}mice were longer than those of wild-type and *Dnase1*^{-/-} mice. These figures show that the size distributions of plasma eccDNA molecules can be used to distinguish mice having certain nuclease deficiencies from mice not having the nuclease deficiencies.

### 4. EccDNA size distributions in mouse tissues

To explore whether the size differences of plasma eccDNA among different genotypes of mice described above occurred intracellularly or extracellularly, the eccDNA extracted from the liver and buffy coat collected from wild-type, *Dnase1*^{-/-}, and *Dnase1l3*^{-/-} mice were profiled. Two approaches for tissue eccDNA identification were applied in parallel: the tagmentation-based approach and the rolling circle amplification (RCA)-based approach. Tissue eccDNA molecules were pooled for size profiling according to the mouse genotypes and tissue types. EccDNA size differences are observed among different genotypes in plasma but not in tissue.

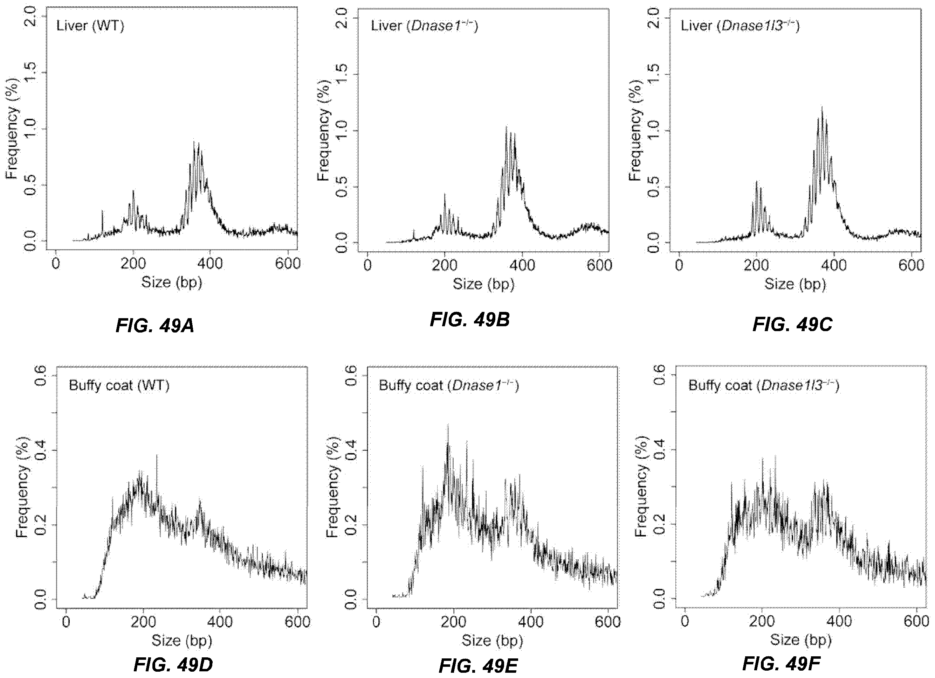

**FIGS. 49A-49F** show size profiling results from the tagmentation approach. The X-axis shows the size of fragments. The Y-axis shows the frequency. Liver eccDNA was analyzed in 5 wild-type mice (FIG. 49A), 5 *Dnase1*^{-/-} mice (FIG. 49B) and 5 *Dnase1l3*^{-/-} mice (FIG. 49C). Buffy coat eccDNA was analyzed in 6 wild-type mice (FIG. 49D), 4 *Dnase1*^{-/-} mice (FIG. 49E), and 5 *Dnase1l3*^{-/-} mice (FIG. 49F). eccDNA molecules, with medians of 3,051 (range: 1,633-29,176) and 4,217 (range: 1,952-10,034), were identified from the liver and buffy coat tissues, respectively.

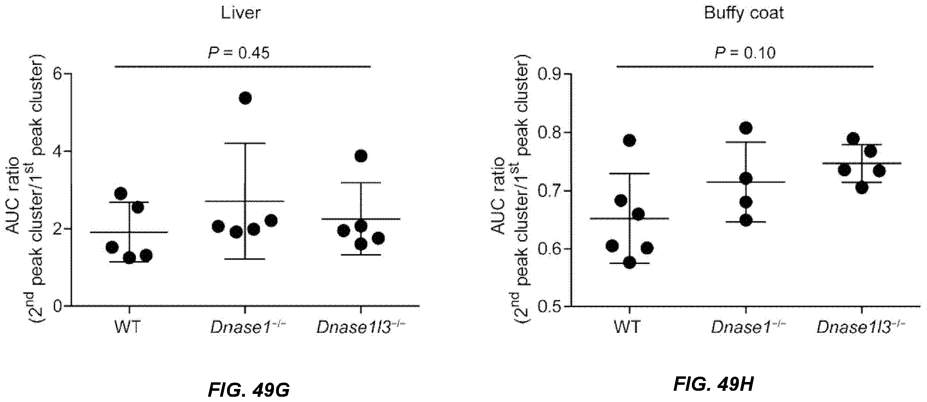

**FIG. 49G** shows AUC ratios for liver eccDNA for the three groups (wild type, *Dnase1*^{*-*/-}*,* and *Dnase1l3*^{*-*/-} mice) using the tagmentation approach. The X-axis shows the group. The Y-axis shows the AUC ratio, which is the 2^{nd} peak cluster (300-450 bp) area divided by the 1^{st} peak cluster (150-250 bp) area. No statistically significant difference in AUC ratios was detected in the liver eccDNA amongst the three groups of mice (P = 0.45, Kruskal-Wallis test).

**FIG. 49H** shows AUC ratios for buffy coat eccDNA for the three groups (wild type, *Dnase1*^{*-*/-}*,* and *Dnase1l3*^{*-*/-}mice) using the tagmentation approach. The X-axis shows the group. The Y-axis shows the AUC ratio, which is the 2^{nd} peak cluster (300-450 bp) area divided by the 1^{st} peak cluster (150-250 bp) area. No statistically significant difference in AUC ratios was detected in the buffy coat samples (P = 0.10, Kruskal-Wallis test), similar to the liver samples.

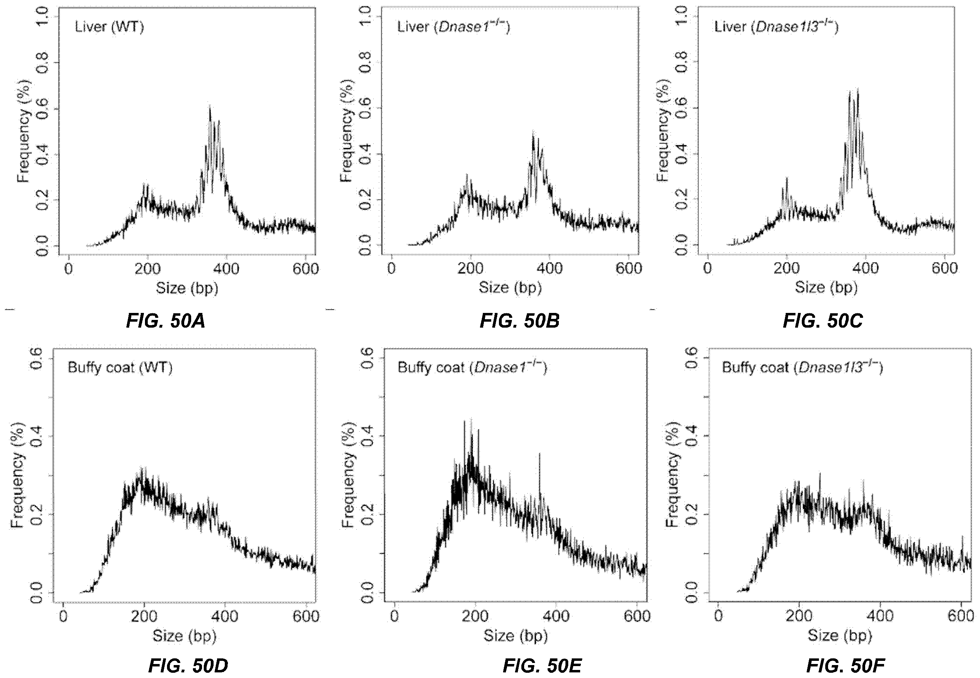

**FIGS. 50A-50F** show size profiling results from the RCA approach. The X-axis shows the size of fragments. The Y-axis shows the frequency. Liver eccDNA was analyzed in 5 wild-type mice (FIG. 50A), 5 *Dnase1*^{*-*/*-*} mice (FIG. 50B) and 5 *Dnase1l3*^{-/-} mice (FIG. 50C). Buffy coat eccDNA was analyzed in 6 wild-type mice (FIG. 50D), 4 *Dnase1*^{-/-} mice (FIG. 50E), and 5 *Dnase1l3*^{*-*/*-*} mice (FIG. 50F). eccDNA molecules, with medians of 10,402 (range: 4,355-42,473) and 12,490 (range: 6,260-43,288), were identified from the liver and buffy coat tissues, respectively.

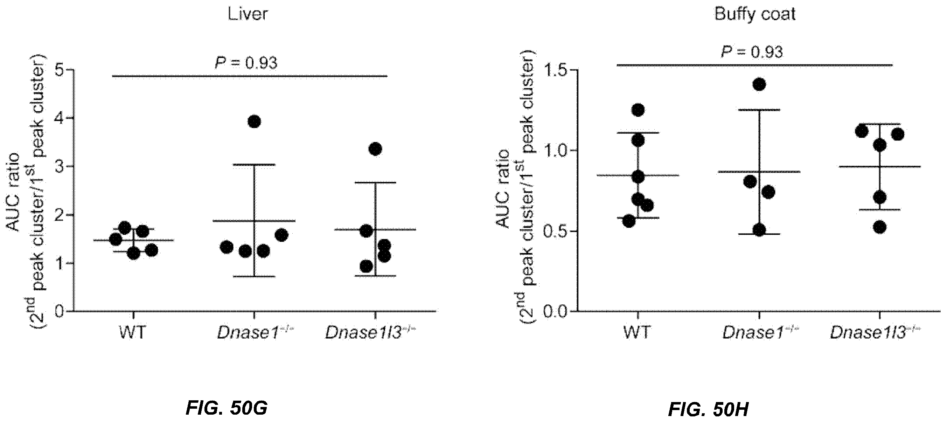

**FIG. 50G** shows AUC ratios for liver eccDNA for the three groups (wild type, *Dnase1*^{*-*/-}*,* and *Dnase1l3*^{*-*/-} mice) for the RCA approach. The X-axis shows the group. The Y-axis shows the AUC ratio, which is the 2^{nd} peak cluster (300-450 bp) area divided by the 1^{st} peak cluster (150-250 bp) area. No statistically significant difference in AUC ratios was detected in the liver eccDNA amongst the three groups of mice (*P* = 0.93, Kruskal-Wallis test).

**FIG. 50H** shows AUC ratios for buffy coat eccDNA for the three groups (wild type, *Dnase1*^{*-*/-}*,* and *Dnase1l3*^{*-*/-} mice) for the RCA approach. The X-axis shows the group. The Y-axis shows the AUC ratio, which is the 2^{nd} peak cluster (300-450 bp) area divided by the 1^{st} peak cluster (150-250 bp) area. No statistically significant difference in AUC ratios was detected in the buffy coat samples (*P* = 0.93, Kruskal-Wallis test), just like the liver samples.

For both tagmentation and RCA approaches, eccDNA identified in each tissue type was pooled for each genotype of mice and size profiled. The eccDNA molecules originating from these tissues all displayed bimodal size distributions with the two summits at around 200 bp and 350 bp. Of note, the two peak clusters of the liver eccDNA were sharper than those of the buffy coat eccDNA. The 10-bp periodic oscillations were also apparent in the liver eccDNA (reminiscent of plasma eccDNA patterns) but relatively obscure in the buffy coat eccDNA. Such variations possibly hinted that the characteristics of eccDNA might depend on their tissues of origin. No obvious difference in eccDNA size distributions could be observed among wild-type, *Dnase1*^{-/-} and *Dnase1l3*^{-/-} mice for either the liver or buffy coat.

The results showing that the eccDNA size differences among genotypes were observed in plasma but not in tissue suggested that the effects of DNASE1L3 on intracellular eccDNA may be insignificant. In contrast, this enzyme may be able to act on eccDNA after these molecules were released into the blood circulation.

### 5. Dnase1l3^{-/-} mouse pregnancy model

To test the hypothesis that the size differences of eccDNA observed in plasma between wild-type and *Dnase1l3*^{-/-} mice were due to extracellular DNASE1L3 effects, the *Dnase1l3*^{-/-}mouse pregnancy model was employed. In this model female mice of the C57BL/6 (B6) strain with or without *Dnase1l3* deficiency were crossed with wild-type mice from the BALB/c genomic background. As such, three mating groups were generated: (1) wild-type females pregnant with wild-type fetuses; (2) *Dnase1l3*^{-/-} females pregnant with *Dnase1l3*^{-/-} fetuses; (3) *Dnase1l3*^{*-*/*-*} females pregnant with *Dnase1l3*^{+/-} (heterozygous) fetuses. The genomic differences between the B6 and BALB/c strains may also be used to distinguish fetal-specific molecules from those shared by the mother and the fetuses (i.e., shared molecules) (see details in Materials and Methods). The results show that DNASE1L3 released by the fetus can digest the eccDNA in maternal plasma.

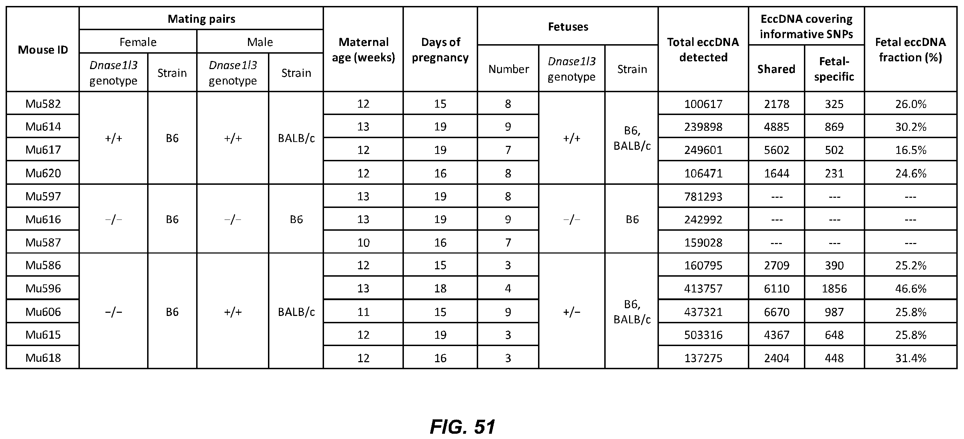

**FIG. 51** shows information of mouse pregnancy samples and their respective eccDNA fetal fractions in the maternal plasma. The mouse pregnancy samples in the figure have different *Dnase1l3* genotypes for the mouse and the fetus. The samples are analyzed for eccDNA sizes and amounts for the different genotypes. The first column of FIG. 51 is the mouse ID. The second through fifth columns include information of the mating pairs. The second column is the *Dnase1l3* genotype of the female. The third column lists the strain for the female. The fourth column is the *Dnase1l3* genotype of the male. The fifth column lists the strain for the male. The sixth column lists the maternal age in weeks. The seventh column is the days of the pregnancy. The eight through tenth columns provide information of the fetus. The eight column is the number of fetuses. The ninth column is the *Dnase1l3* genotype of the fetuses. The tenth column lists the strain(s) of the fetus. The eleventh column includes the total eccDNA fragments detected. The twelfth and thirteenth columns provide information on eccDNA covering informative SNPs. The fourteenth column lists the number of eccDNA covering informative SNPs that are shared between the fetus and the mother. The fifteenth column lists the number of eccDNA that cover fetal-specific SNPs. The sixteenth column includes the fetal eccDNA fraction. The median fetal eccDNA fraction was 25.8% (range: 16.5-46.6%).

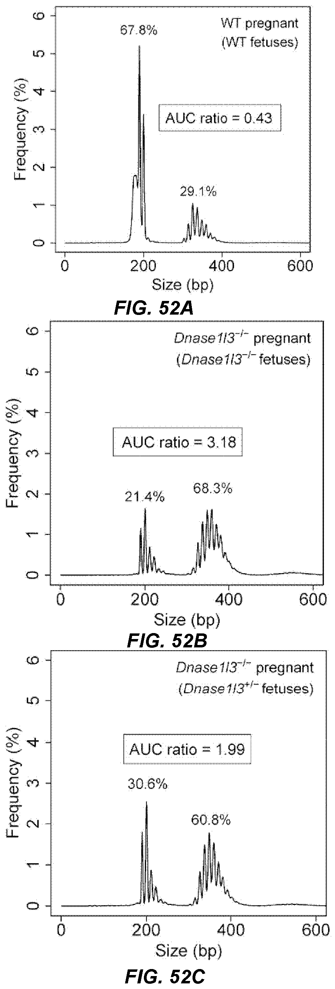

**FIGS. 52A-52C** plot the mean size distributions of total plasma eccDNA in the three groups of pregnant mice. The mean size distributions of eccDNA in maternal plasma were plotted for wild-type females carrying wild-type fetuses (FIG. 52A), *Dnase1l3*^{*-*/*-*} females carrying *Dnase1l3*^{*-*/*-*} fetuses (FIG. 52B) and *Dnase1l*3^{-/-} females carrying *Dnase1l3*^{+/-} fetuses (FIG. 52C). The eccDNA fragment size is shown on the X-axis. The frequency is shown on the Y-axis. AUC values were labeled for each peak clusters, and AUC ratios were calculated accordingly. For wild-type females carrying wild-type fetuses (FIG. 52A), the plasma eccDNA showed a high 1^{st} peak cluster (AUC = 67.8%) and a relatively low 2^{nd} peak cluster (AUC = 29.1%), with an AUC ratio of 0.43. On the other hand, for *Dnase1l3*^{-/-} females carrying *Dnase1l3*^{-/-} fetuses (FIG. 52B), the plasma eccDNA showed a low 1^{st} peak cluster (AUC = 21.4%) and a higher 2^{nd} peak cluster (AUC = 68.3%), with an AUC ratio of 3.18. Thus, *Dnase1l3*^{*-*/*-*} females pregnant with *Dnase1l3*^{*-*/*-*} fetuses had longer plasma eccDNA than wild-type mice carrying wild-type fetuses. This is consistent with the eccDNA size differences observed between the non-pregnant wild-type and *Dnase1l3*^{-/-} mice (FIG. 48A and FIG. 48C). However, for the *Dnase1l3*^{-/-} females carrying *Dnase1l3*^{+/-} (heterozygous) fetuses (FIG. 52C), the plasma eccDNA sizes showed a partial reversal from the *Dnase1l3*^{*-*/*-*} phenotype to the wild-type phenotype, with both AUC values (AUC_{1st peak cluster} = 30.6%; AUC_{2nd peak cluster} = 60.8%) and AUC ratio (1.99) falling between the first two groups of pregnant mice. FIGS. 52A-52C suggest the presence of systemic effects of fetally-released DNASE1L3 on the eccDNA molecules in maternal circulation, shortening their sizes.

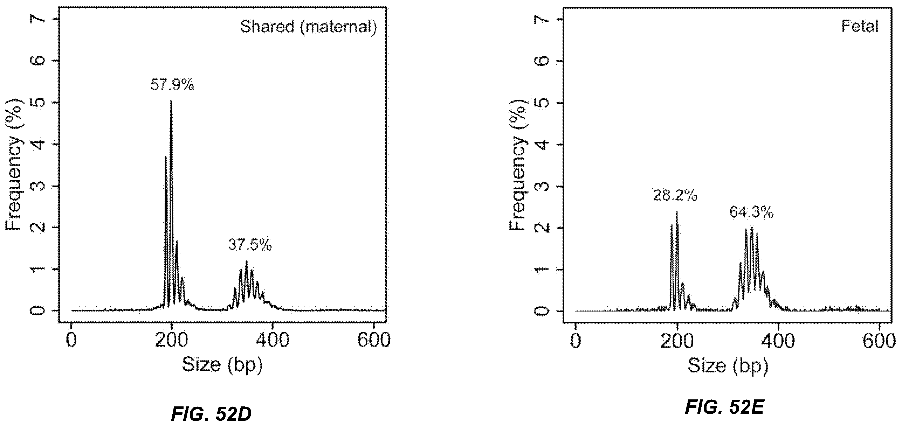

**FIG. 52D** shows the size profile of maternal eccDNA pooled separately from fetal eccDNA. **FIG. 52E** shows the size profile of fetal eccDNA pooled separately from maternal eccDNA. The eccDNA fragment size is shown on the X-axis, and the frequency is shown on the Y-axis. The fetal eccDNA was not observed to be shorter than the maternal eccDNA (shared molecules) in the plasma of *Dnase1l3*^{-/-} mice pregnant with *Dnase1l3*^{*+*/*-*} fetuses, suggesting that the local effect of DNASE1L3 on eccDNA digestion might not be as significant as it may be on linear cfDNA as previously reported (Serpas et al., PNAS (2019)). The figures show that the fetally-released DNASE1L3 can digest the eccDNA in maternal plasma.

### 6. Human subjects with DNASE1L3 deficiency

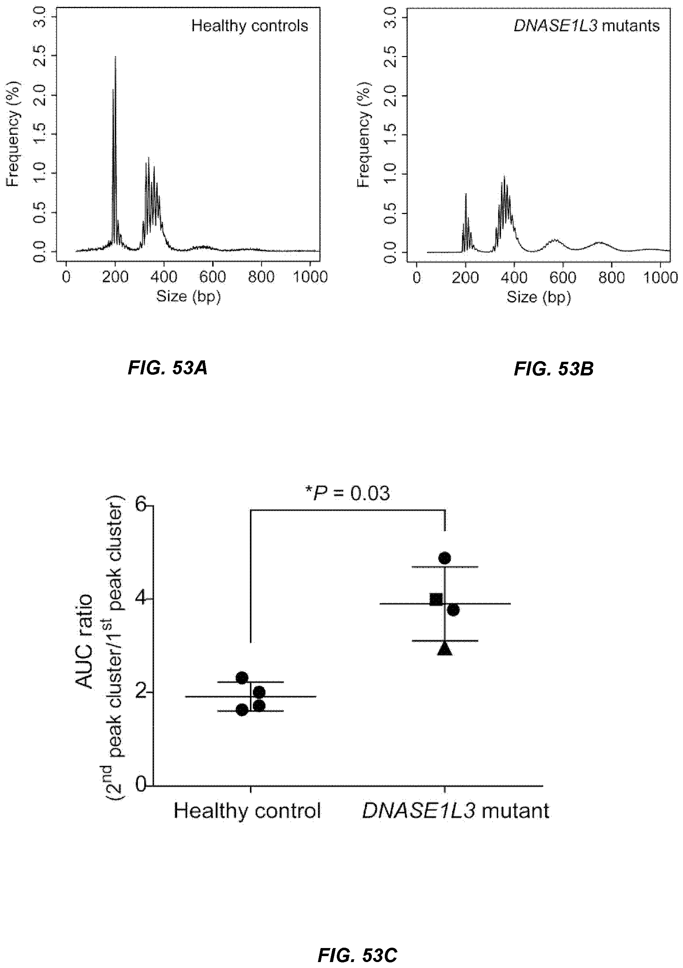

The effects of *DNASE1L3* deficiency on plasma eccDNA were further investigated in patients with *DNASE1L3* loss-of-function mutations (i.e., *DNASE1L3*^{*-*/*-*}). Detailed sample information of these subjects is described in the Materials and Methods. **FIGS. 53A** and **53B** plot the mean size distributions of plasma eccDNA from healthy human subjects and *DNASE1L3*-mutated patients, respectively. The size of the eccDNA fragments is shown on the X-axis, and the frequency is shown on the Y-axis. Size profile of eccDNA pooled from plasma samples collected from 4 healthy human subjects (FIG. 53A). Size profile of eccDNA pooled from 4 plasma samples collected from 3 human subjects with loss-of-function mutations of *DNASE1L3* (FIG. 53B); Two of these samples were collected from the same subject at both pre- and post-hemodialysis timepoints. No difference was seen between the samples taken pre-hemodialysis and those taken post-hemodialysis. When compared to healthy controls, *DNASE1L3* mutants showed a lower 1^{st} peak cluster, reflecting a decrease in frequencies of small eccDNA in these patients. Similar to the results in mice, results in human showed that *DNASE1L3* deficiency in humans results in longer eccDNA in plasma.

**FIG. 53C** compares the AUC ratios between healthy controls and *DNASE1L3*-mutated subjects. The subject group is shown on the X-axis. The AUC ratio is shown on the Y-axis. In FIG. 53C, circular dots denote that samples were collected at single time points from each subject; triangle 8210 and square 8220 denote that samples collected at pre- and post-hemodialysis timepoints from the same patient, respectively. Subjects with *DNASE1L3* mutations exhibited significantly higher AUC ratios than the healthy subjects (P = 0.03, Wilcoxon rank-sum test).

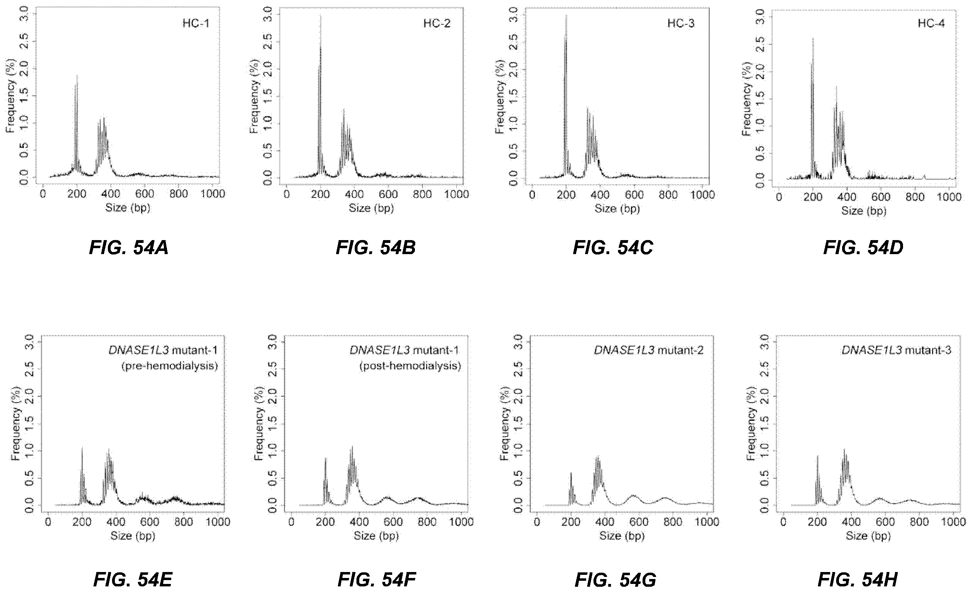

**FIGS. 54A** to **54H** show the individual size profiles of these subjects in FIGS. 53A to 53C. The size of the eccDNA fragments is shown on the X-axis, and the frequency is shown on the Y-axis. For the patient who donated blood samples at two time points, the eccDNA size distributions were quite similar between the pre-hemodialysis timepoint (FIG. 54E) and post-hemodialysis timepoint (FIG. 54F). Interestingly, an enrichment of the 3^{rd} peak cluster (500-650 bp) and the 4^{th} peak cluster (700-800 bp) was also observed in the *DNASE1L3*-mutated subjects (FIGS. 54E-54H), reflecting higher abundance of long eccDNA molecules in the plasma of these patients. Thus, *DNASE1L3* deficiency in human subjects would lead to the lengthening of eccDNA in plasma, which is consistent with our findings in *Dnase1l3* knockout mice (FIGS. 48A-48D).

### B. Size profile changes

Biological properties of eccDNA could be affected by the activity of nucleases. By utilizing knockout mouse models of *Dnase1* and *Dnase1l3,* it is shown herein that the deficiency of *Dnase1l3* would significantly lengthen the plasma eccDNA in mice. However, such effects were not observed in mice with *Dnase1* deficiency. DNASE1L3 may be one of the main contributors affecting the size characteristics of cell-free eccDNA.

Intriguingly, the plasma eccDNA size distributions in wild-type mice (FIG. 48A) were distinct from those in human subjects (FIG. 53A), with the wild-type mice having more enhanced 1st peak clusters and more diminished 2nd peak clusters than wild-type human subjects. On the other hand, the plasma eccDNA size profiles of *Dnase1l3*-deficient mice (FIG. 48C) highly resembled those in humans. Such observations could possibly be attributed to the much higher activity of circulating DNASE1L3 present in mice (M. Napirei, S. Ludwig, J. Mezrhab, T. Klöckl, H. G. Mannherz, Murine serum nucleases - contrasting effects of plasmin and heparin on the activities of DNase1 and DNase1-like 3 (DNase1l3). FEBS J. 276, 1059-1073 (2009)). The shortening effect of DNASE1L3 on eccDNA sizes was further highlighted in our data comparing cell-free eccDNA between healthy human subjects and *DNASE1L3*-mutated patients (FIG. 53C). These lines of evidence consistently suggested that the activity of DNASE1L3 could effectively regulate the biological characteristics of cell-free eccDNA.

Notably, at the intracellular level, neither *Dnase1*^{-/-} mice nor *Dnase1l3*^{-/-} mice showed observable change in eccDNA size profiles (FIGS. 50A-50H). The result suggests that the access of intracellular eccDNA by DNASE1L3 may be limited. Instead, DNASE1L3 could act on eccDNA molecules after they enter the blood circulation. This postulation could in part be supported by the following pieces of evidence pertaining to living cells: (i) DNASE1L3 was detected in the endoplasmic reticulum, but absent from the nucleus (S. D, T. S, Characterization of human DNase I family endonucleases and activation of DNase gamma during apoptosis. Biochemistry 40, 143-152 (2001), M. Napirei, S. Wulf, D. Eulitz, H. G. Mannherz, T. Kloeckl, Comparative characterization of rat deoxyribonuclease 1 (Dnase1) and murine deoxyribonuclease 1-like 3 (Dnase1l3). Biochem. J. 389, 355-64 (2005)); (ii) cellular eccDNA was located inside the cell nucleus (Y. Hotta, A. Bassel, Molecular Size and Circularity of Dna in Cells of Mammals and Higher Plants. Proc. Natl. Acad. Sci. 53, 356-362 (1965), Y. Shibata, et al., Extrachromosomal microDNAs and chromosomal microdeletions in normal tissues. Science 336, 82-6 (2012)), which would thus limit the access of DNASE1L3 to these molecules. However, the release of eccDNA molecules into the circulation would facilitate their access by DNASE1L3, leading to the degradation of these DNA molecules.

The extracellular function of DNASE1L3 on cell-free eccDNA was further evidenced by our findings from the Dnase1l3 pregnancy mouse model. It has previously been established that in *Dnase1l3*-deficient mice pregnant with *Dnase1l3*^{+/-} fetuses, DNASE1L3 released from the fetuses could degrade linear cfDNA molecules in a systemic manner (Serpas et al., PNAS (2019)). Similarly,a partial restoration of eccDNA size profiles towards the wild-type patterns in the maternal plasma under the same pregnancy setting was observed here. This finding suggested that the extracellular DNASE1L3 produced by the fetuses may act on the eccDNA in the maternal blood circulation, mediating the degradation of maternal cell-free eccDNA. As to the local effects of DNASE1L3 (Serpas et al., PNAS (2019)), a shortening of eccDNA derived from the *Dnase1l3*^{+/-} fetuses when compared to their *Dnase1l3*^{-/-} mothers was not observed. We speculated that there might be certain features of cell-free eccDNA that remain to be unveiled, such as whether those eccDNA molecules would be associated with extracellular vesicles or histone proteins.

A biological link between nuclease activity and the properties of cell-free eccDNA is established using mouse and human models with *DNASE1L3* deficiency. Since aberrant expression of *DNASE1L3* has been reported in multiple disorders such as systemic lupus erythematosus (R. W. Y. Chan, et al., Plasma DNA Profile Associated with DNASE1L3 Gene Mutations : Clinical Observations , Relationships to Nuclease Substrate Preference , and In Vivo Correction. Am. J. Hum. Genet., 1-13 (2020), J. Hartl, et al., Autoantibody-mediated impairment of DNASE1L3 activity in sporadic systemic lupus erythematosus. J. Exp. Med. 218 (2021)) and certain types of cancer (S. D, T. S, Characterization of human DNase I family endonucleases and activation of DNase gamma during apoptosis. Biochemistry 40, 143-152 (2001), M. Napirei, S. Wulf, D. Eulitz, H. G. Mannherz, T. Kloeckl, Comparative characterization of rat deoxyribonuclease 1 (Dnase1) and murine deoxyribonuclease 1-like 3 (Dnase1l3). Biochem. J. 389, 355-64 (2005)), size pattern analyses of cell-free eccDNA can be used for biomarker developments for these diseases.

### C. Example methods

The size profile of eccDNA can be used to determine various characteristics of a biological sample or the subject from which the biological sample is obtained. The amounts of certain sizes of eccDNA may be used to compare different size profiles. The raw amounts of certain sizes or the ratios of different sizes can be used. Genetic disorders, including disorders related to under- or over-production of enzymes, may be detected. The activity of nucleases may be monitored. The condition of a subject can be determined based on nuclease activity. The amounts of eccDNA can be used to determine a genetic disorder.

### 1. Determining a genetic disorder associated with a nuclease using eccDNA size distribution

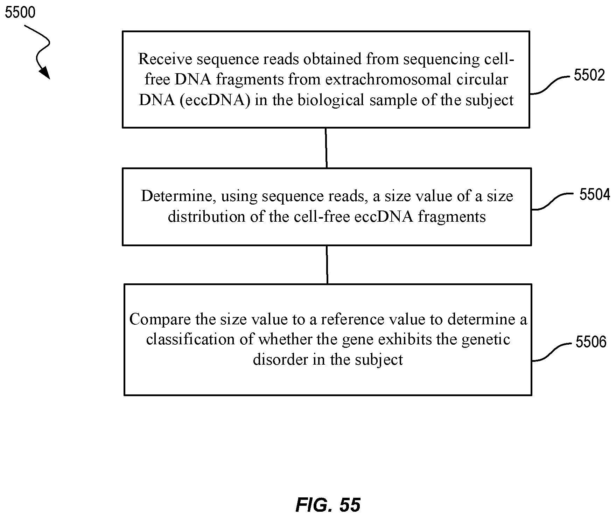

**FIG. 55** is a flowchart illustrating a method 5500 for detecting a genetic disorder for a gene associated with a nuclease using a biological sample of a subject including cell-free eccDNA according to embodiments of the present disclosure. Method 5500 and others method herein can be performed entirely or partially with a computer system. The biological sample can be any cell-free DNA sample, e.g., as described herein. The subject may be pregnant with a fetus.

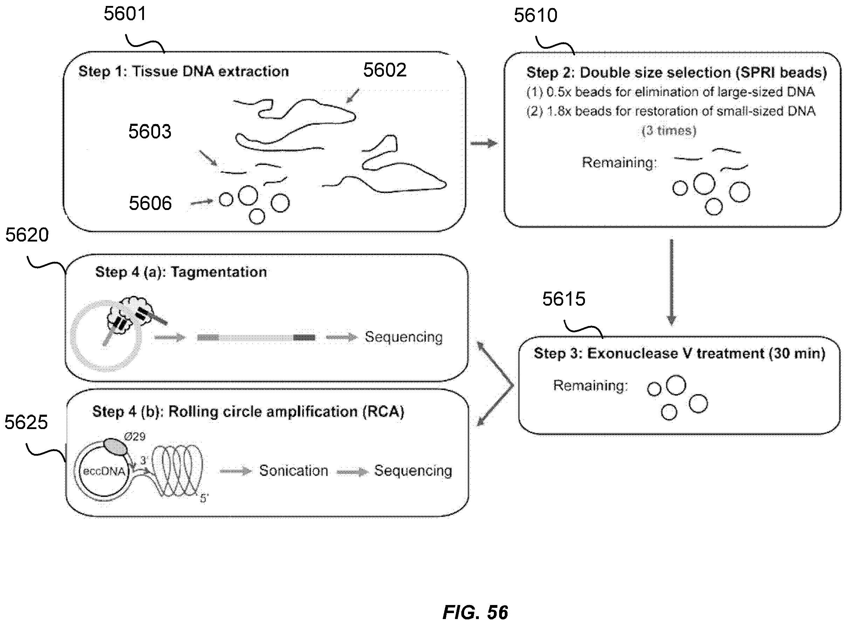

At block 5502, sequence reads obtained from sequencing cell-free DNA fragments from eccDNA in the biological sample of the subject are received. The sequencing may be performed in various ways, e.g., as described herein. The sequencing may be targeted sequencing as described herein. For example, biological sample can be enriched for eccDNA first and/or also enriched for fragments from a particular region or regions within eccDNA. Different methods for tissue eccDNA identification and enrichment are shown in **FIG. 56** and described herein. The enriching may include tagmentation (FIG. 56, step 5620), rolling circle amplification (FIG. 56, step 5625), or enzyme treatment (FIG. 56, step 5615). The enzyme treatment may include exonuclease V treatment. The enzyme treatment may take up to 5 minutes (min), 10 min, 20 min, 30 min, 45 min, 60 min, 90 min, 12 hours (hr), 18 hr, 24 hr, or longer. In some embodiments, the enzyme treatment takes up to about 30 minutes.

At block 5504, the sequence reads are used to determine a size value of a size distribution of the cell-free DNA fragments. A size value may characterize a size distribution. A size value may characterize an amount of cell-free DNA fragments at one or more sizes. In some embodiments, the size distribution is of the cell-free eccDNA.

In some embodiments, the size value is a ratio of a first amount of the cell-free DNA fragments having a first size relative to a second amount of the cell-free DNA fragments having a second size (e.g., FIG. 48E). The first size (e.g., sizes corresponding to FIG. 48E, first peak cluster 7310) may be about 150 base pairs (bp) to about 250 bp. The first size may be from about 50 bp to about 250 bp, about 50 bp to about 100 bp, about 100 bp to about 150 bp, about 150 bp to about 200 bp, or from about 200 bp to about 250 bp. The second size (e.g., sizes corresponding to FIG. 48E, second peak cluster 7320) may be about 300 bp to about 450 bp. The second size may be from about 250 bp to about 500 bp, 250 bp to about 300 bp, 300 bp to about 350 bp, about 350 bp to about 400 bp, about 400 bp to about 450 bp, or about 450 bp to about 500 bp, about 500 bp to about 550 bp, 550 bp to about 600 bp, 600 bp to about 650 bp, 500 to about 650 bp, 650 bp to about 700 bp, 700 bp to about 750 bp, 750 bp to about 800 bp, 700 to 800 bp, or 800 to 850 bp. The first size and the second size (e.g., a median or average size of the first or second size) may be at least about 10 bp, 20 bp, 30 bp, 40 bp, 50 bp, 60 bp, 70 bp, 80 bp, 90 bp, 100 bp, 150 bp, or 200 bp apart from one another. In some cases, the upper limit of the first size may be at least about 10 bp, 20 bp, 30 bp, 40 bp, 50 bp, 60 bp, 70 bp, 80 bp, 90 bp, or 100 bp smaller than the lower limit of the second size. The first size is different from the second size. The one or more sizes may include the first size and the second size. In some embodiments, more than one size value may be used. For example, each size value may be a different ratios involving a different first size and/or a different second size.

In some embodiments, the size value is based on an amount of cell-free DNA fragments in any of the sizes described herein rather than a ratio of amounts of different sizes. For example, the size value may be the amount of cell-free DNA fragments having sizes of 300 bp to 400 bp. The size value may be a frequency (e.g., percentage) or a count of DNA fragments. The frequency may be an area under curve (AUC) of frequency for a size range. For example, the AUC may be the AUC under one of the two peaks in FIGS. 48A-48C. In some embodiments, the size value may be a ratio of AUCs. For example, the AUC ratio may be the AUC under one peak divided by the AUC for another peak (e.g., AUC ratio in FIG. 48D).

At block 5506, the size value from the sample (e.g., from a human subject, or another mammal) are then be compared to a reference size value obtained from one or more reference samples. The samples may be obtained from subjects pregnant with fetus.

The reference samples may comprise a sample obtained from a subject pregnant with a fetus. The reference samples may comprise a sample obtained from a healthy subject, for example a subject that does not have a nuclease activity deficiency, or any genetic disorder for a gene associated with a nuclease. The healthy subject may have normal nuclease activity. The reference samples may comprise a sample from a subject that has a nuclease activity deficiency, or a genetic disorder for a gene associated with a nuclease. The reference sample may be obtained from a tissue or blood (e.g., plasma or serum) or any biological sample described herein. The reference sample may be from subjects at a same or similar gestational age (e.g., same trimester or a gestational age within 1, 2, 3, or 4 weeks of the subject).

The reference value may be determined in the same manner as the size value. The difference between a size value of a sample and a reference value of the reference sample may be used to classify whether a gene exhibits the genetic disorder. The reference value may be a cutoff value that determines a statistically significant difference from the reference samples. For example, the reference value may be one, two, or three standard deviations away from an average of reference subjects with or without the genetic disorder. In some cases, the reference sample is obtained from the subject prior or after a treatment, where the treatment affects the activity of a nuclease. In some embodiments, the treatment is hemodialysis.

In some embodiments, the comparison to the reference can involve a machine learning model, e.g., trained using supervised learning. The size values (and potentially other criteria, such as copy number and methylation levels) and the known conditions of training subjects from whom training samples were obtained can form a training data set. The parameters of the machine learning model can be optimized based on the training set to provide an optimized accuracy in classifying the level of the condition. Example machine learning models include neural networks, decision trees, clustering, and support vector machines. Comparisons may be carried out as described in block 3906 of FIG. 39.

At block 5506, a classification of whether the gene associated with a nuclease exhibits a genetic disorder is determined based on the comparison of the size values. In some embodiments, the subject is pregnant with a fetus. The sample may contain cell-free eccDNA from the subject and the fetus. The size value comparison may then be used to determine a classification of whether the fetus has a nuclease activity deficiency, or a genetic disorder for a gene associated with a nuclease. In some cases, the same sample may be used to determine a classification of whether the pregnant subject has a nuclease activity deficiency, or a genetic disorder for a gene associated with a nuclease based on the comparison. The genetic disorder may be a disorder of the *DNASE1L3* gene. Genetic disorders may include disorders of one or more of the following genes: *DNASE1, DFFB, TREX1* (Three Prime Repair Exonuclease 1), *AEN* (Apoptosis Enhancing Nuclease), *EXO1* (Exonuclease 1), *DNASE2* (Deoxyribonuclease 2), *ENDOG* (Endonuclease G), *APEX1* (Apurinic/Apyrimidinic Endodeoxyribonuclease 1), *FEN1* (Flap Structure-Specific Endonuclease 1), *DNASE1L1* (Deoxyribonuclease 1 Like 1), *DNASE1L2* (Deoxyribonuclease 1 Like 2), and *EXOG* (Exo/Endonuclease G).

In some cases, the sample may be used to detect if a maternal allele, a paternal allele (e.g., a fetal-specific allele), or both alleles of a gene, associated with a nuclease, exhibit a genetic disorder (e.g., FIGS. 52A-52C). For example, two reference values may be used. A first reference value may be determined from one or more first reference subjects with fetuses being homozygous for a first allele at a location. A second reference value may be determined from one or more second reference subjects with fetuses being homozygous for a second allele at the location.

### 2. Determining an efficacy of treatment for a blood disorder

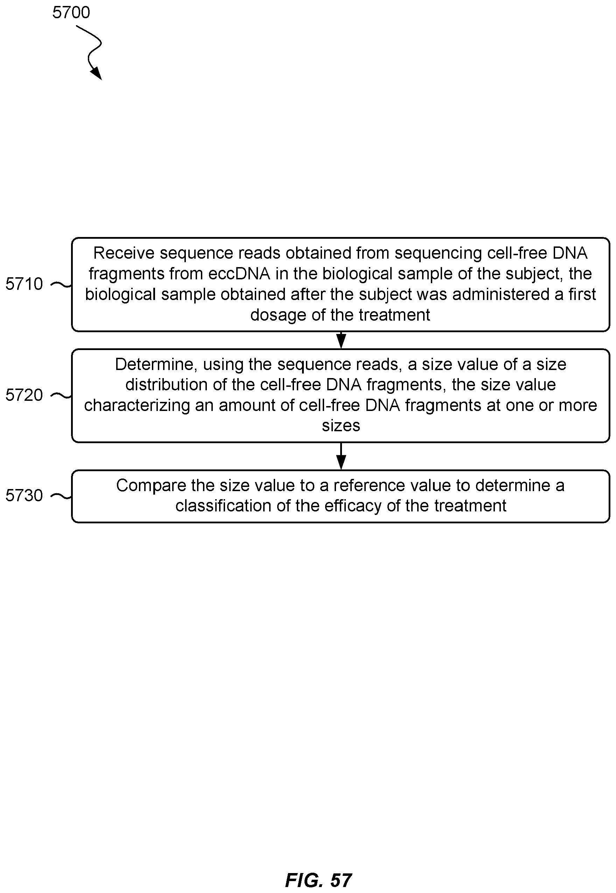

**FIG. 57** shows a flowchart illustrating a method 5700 for determining an efficacy of a treatment of a subject having blood disorder according to embodiments of the present disclosure. Certain blocks of method 5700 can be performed in a similar manner as blocks of method 5500 of FIG. 55.

At block 5710, sequence reads obtained from sequencing cell-free DNA fragments in a blood sample of the subject are received. The blood sample of the subject may be obtained after the subject has undergone a treatment (e.g., a first dosage of a treatment). Treatments may include anticoagulants, hemodialysis, a kidney transplant, or any treatment described herein. The sequence reads may be obtained similar to the manner described with block 4002 of FIG. 40 or in any manner described herein.

At block 5720, a size value of a size distribution of the cell-free DNA fragments is determined. The size value characterizes an amount of cell-free DNA fragments at one or more sizes. Block 5720 may be performed in a similar manner as block 5504.

At block 5730, the size value is compared to a reference value to determine a classification of the efficacy of the treatment. A second dosage of the anticoagulant can be administered to the subject based on the comparison, the second dosage being greater than the first dosage. In other examples, the second dosage can be less than the first dosage, e.g., if the amount overshoots the reference value. Treamtents may be continued, increased, or discontinued based on the comparison.

The reference value can correspond to a measurement previously performed in the subject before administering the treatment. The change in the amount from the previous measurement can indicate an efficacy of the treatment. In another implementation, the reference value can correspond to the amount measured in a healthy subject. An efficacious treatment can be one that brings the amount to within a threshold of the reference value for the healthy subject. In yet another implementation, the reference value can correspond to the amount measured in a subject that has the blood disorder (e.g., as may be previously measured in the subject before administering the treatment). For example, a reference value may comprise a wildtype animal or a healthy human subject. A reference value may comprise a tissue specific sample or a portion of a sample obtained from the same subject (e.g., sequence reads obtained from plasma or buffy coat portion of a sample), as shown, for example, in FIG. 1.

### 3. Monitoring activity of a nuclease using eccDNA

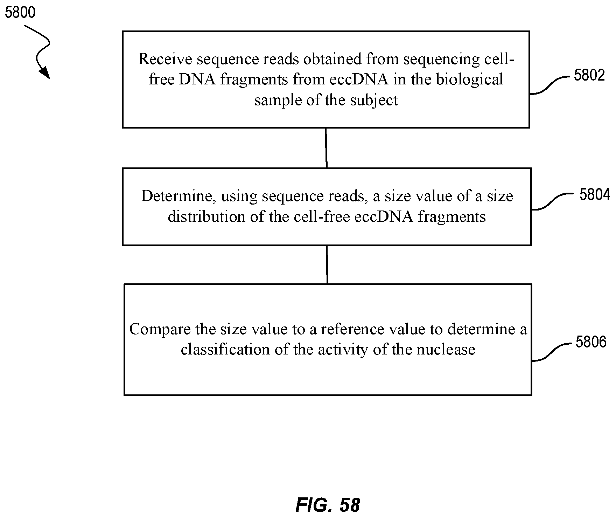

**FIG. 58** is a flowchart illustrating a method 5800 for monitoring an activity of a nuclease using a biological sample of a subject including eccDNA, according to embodiments of the present invention. Certain blocks of method 5800 can be performed in a similar manner as blocks of method 5500.

At block 5802, similar to block 5502, sequence reads obtained from sequencing cell-free DNA fragments from eccDNA in the biological sample of the subject are received.

At block 5804, similar to block 5504, the sequence reads are used to determine a size value of a size distribution of the cell-free DNA fragments.

At block 5806, similar to block 5506, the size value from the sample (e.g., from a human subject, or another mammal) are compared to a reference size value obtained from one or more reference samples. A classification of the activity of the nuclease may then be determined based on the comparison. The nuclease may be DNASE1L3, DNASE1, DFFB, TREX1 (Three Prime Repair Exonuclease 1), AEN (Apoptosis Enhancing Nuclease), EXO1 (Exonuclease 1), DNASE2 (Deoxyribonuclease 2), ENDOG (Endonuclease G), APEX1 (Apurinic/Apyrimidinic Endodeoxyribonuclease 1), FEN1 (Flap Structure-Specific Endonuclease 1), DNASE1L1 (Deoxyribonuclease 1 Like 1), or DNASE1L2 (Deoxyribonuclease 1 Like.

In some cases, the pregnant subject, the fetus, or both have a nuclease activity deficiency, or a genetic disorder for a gene associated with a nuclease. In some other cases, only one of the pregnant subject or the fetus has a nuclease activity deficiency, or a genetic disorder for a gene associated with a nuclease. The gene may be any member of the DNase I family (e.g., in humans). In some embodiments, the gene is *DNASE1* or *DNASE1L3.* A loss of both alleles (i.e., homozygosity for a null allele) or one of the two alleles (i.e., heterozygosity) of the gene may be associated with a disease. For example, homozygosity for a null allele in *DNASE1L3* may be associated with a condition such as systemic lupus erythematosus. In another example, heterozygosity of the *DNASE1L3* may be associated with condition such as rheumatoid arthritis. The size value comparison may be used to determine a nuclease activity deficiency in the subject (i.e., the sample obtained from the subject).

Genotype information of the fetus may be obtained from comparing the size value to the reference value without genotyping the mother. However, in some embodiments, the mother may be genotyped. The mother may be homozygous (e.g., loss of both alleles or wild type for both alleles) or heterozygous. A plurality of reference values may be obtained for groups where (1) the mother is homozygous (wild type) and does not have a deficiency of a gene and the fetus is wild type (e.g., FIG. 52A); (2) the mother is homozygous for a deficiency in a gene and the fetus is homozygous for the deficiency in the gene (FIG. 52B); (3) the mother is homozygous for the deficiency in the gene and the fetus is heterozygous (FIG. 52C); (4) the mother is wild type and the fetus is heterozygous; (5) the mother is heterozygous and the fetus is heterozygous; and (6) the mother is heterozygous and the fetus is wild type. Longer eccDNA fragments may be expected with an organism being homozygous for the deficiency than when the organism is heterozygous for the deficiency than when the organism is wild type. The sizes may also be affected by the fetal fraction of eccDNA in the biological sample. The reference subjects may have a same or similar gestational age as the subject. The genotype of the fetus may be determined by determining the reference value closest to the size value. The fetus may then be determined to have the same genotype as the reference value. The maternal genotype may then also be determined from the reference value.

The classification may be that the nuclease activity is deficient. The size value may indicate longer cell-free DNA fragments than the reference value. For example, the size value may be a ratio of a cluster of longer sizes to shorter sizes, and the ratio may be larger than the reference value.

In some cases, the nuclease activity deficiency is a hallmark of a condition such as a cancer. As such, in some embodiments, the size value comparison described herein is used to classify a subject (i.e., a sample obtained from the subject) as having the condition (e.g., a cancer). The classification may be that the subject has the condition (e.g., cancer) characterized by the deficient nuclease activity. The reference value may be determined from one or more reference subjects having the condition or from one or more reference subjects without the condition.

### 4. Determining a genetic disorder using eccDNA amount

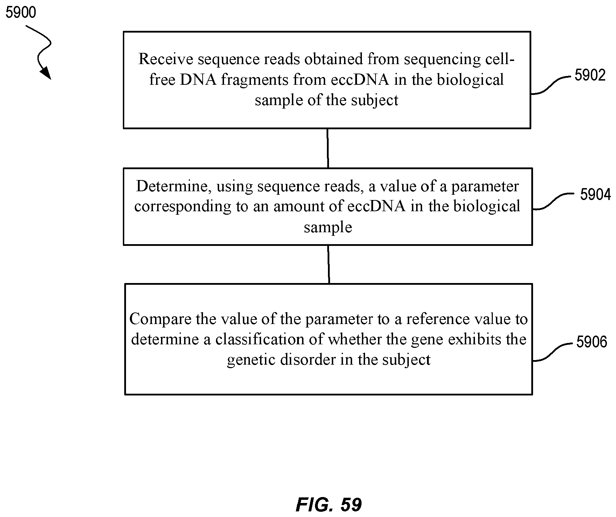

**FIG. 59** is a flowchart illustrating a method for detecting a genetic disorder for a gene associated with a nuclease using a biological sample of a subject including cell-free DNA, according to embodiments of the present invention. Certain blocks of method 5900 can be performed in a similar manner as blocks of methods 5500 or 5800.

At block 5902, similar to blocks 5502 or 5802, sequence reads obtained from sequencing cell-free DNA fragments from eccDNA in the biological sample of the subject are received.

At block 5904, the sequence reads are used to determine a value of a parameter corresponding to an amount of eccDNA in the biological sample. The parameter corresponding to the amount of eccDNA in the biological sample may be, for example, a ratio of the amount of eccDNA to a total amount of mappable sequence reads from the biological sample. For example, the parameter may be the eccDNA per million mappable reads (EPM) described in FIG. 47. The value of the parameter may be, for example, a percentage of abundance of the eccDNA in a sample.

At block 5906, the value of the parameter corresponding to an amount of eccDNA in a sample may be compared to a reference value of the parameter of eccDNA in a reference subject to determine a classification of whether the gene exhibits the genetic disorder in the subject.

The reference subject may be any of the reference subjects described herein (e.g., a subject with the gene associated with a nuclease that does not exhibit a genetic disorder, a subject that does not have a nuclease activity deficiency). The biological sample may be processed to enrich cell-free DNA fragments from eccDNA. The processing may include physical treatment (e.g., filtering, centrifugation, etc.), chemical treatment (e.g., enzymatic digestion), or a combination thereof. In some embodiments, the sample is treated with a nuclease to remove linear DNA before the sequencing of cell-free DNA fragments from eccDNA. The genetic disorder may be any disorder described herein, including a disorder related to *DNASE1L3.* The sample may be further processed by treating the sample with a nuclease followed by sequencing the cell-free DNA fragments to produce the sequence reads. In some cases, the nuclease is exonuclease V.

### 5. Quantifying eccDNA in a sample

The absolute quantity of eccDNA in a sample can be determined by spiking in known quantities of circular DNA. The amount of sequence reads corresponding to the the known quantities of spike-in molecules can then be used to determine the quantity of eccDNA in the sample. A calibration curve relating known quantities of spike-in molecules to amounts of sequence reads may be used to determine the quantity of eccDNA.

Cell-free DNA may be extracted to form the biological sample. The extraction may be similar to step 5601 of FIG. 56. The biological sample may be divided into several equal aliquots or the cell-free DNA may be extracted into several aliquots of equal volume. Different known quanitties of circular DNA can be added to each of the aliquots. For example, various different amounts (e.g., 0.1 ng, 1 ng, 2 ng, 5 ng, 10 ng) of circular DNA of a known size (e.g., 200 bp) can be added (spiked in) to the equal aliquots. The spiked-in molecules may be synthesized circular DNA, plasmid DNA, or other DNA molecules of circular forms.

The mixture of DNA from the biological sample and the spiked-in circular DNA may then be treated with exonuclease V for linear DNA digestion (e.g., step 5615 of FIG. 56). The resultant DNA may then undergo tagmentation for opening of circular DNA (e.g., step 5620 of FIG. 56). The DNA may undergo PCR amplification. The quantity of spike-in circular DNA molecules identified from sequencing results may be correlated with predefined features (e.g., amounts of sequence reads and sizes determined by sequence reads) to establish a standard curve for calibration. The amount of sequence reads of eccDNA from plasma DNA can then be converted into absolute quantities (e.g., mass, concentration) using the calibration curve. In some embodiments, the amount of eccDNA identified from plasma DNA may be normalized using the amounts of sequence reads of known spike-in circular DNA to obtain the relative amount for a eccDNA entity of interest.

A conversion formula derived from the calibration curve can be applied to convert the read counts of eccDNA of various sizes to absolute quantities. For example, if 1 ng of spiked-in circular DNA (e.g., 200 bp) gives 10,000 reads, then 10,000 reads of 400 bp eccDNA of interest would correspond to 2 ng of such molecules in the samples. Such conversion formula might also take into account factors such as, but not limited to, sizes of eccDNA identified, sequencing depth, sequencing length, DNA mappability, and PCR duplication rates. The quantities of eccDNA in a sample may be used to distinguish between healthy control and patient groups using this parameter without considering batch-to-batch variations.

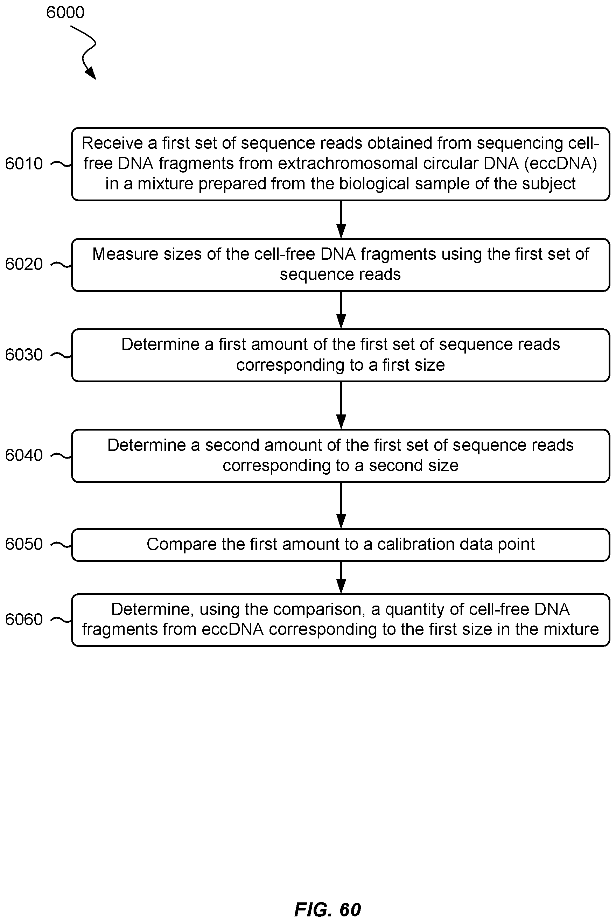

**FIG. 60** is a flowchart of an example process 6000 associated with analyzing a biological sample to quantify the amount of eccDNA. In some implementations, one or more process blocks of FIG. 60 may be performed by a device (e.g., system 6500). In some implementations, one or more process blocks of FIG. 60 may be performed by another device or a group of devices separate from or including the device. Additionally, or alternatively, one or more process blocks of FIG. 60 may be performed by one or more components of system 6500, such as assay device 6510, detector 6520, logic system 6530, local memory 6535, external memory 6540, storage device 6545, processor 6550, and/or treatment device 6560.

At block 6010, a first set of sequence reads obtained from sequencing cell-free DNA fragments from extrachromosomal circular DNA (eccDNA) in a mixture prepared from the biological sample of the subject is received. The first set of sequence reads may be obtained by any method described herein, including for example, block 3902 from FIG. 39. The biological sample may be any biological sample described herein. The prepared mixture may include fragments from a known quantity of circular DNA of a particular known size in addition to the DNA fragments from eccDNA.

The known quantity of circular DNA may be added to the biological sample to obtain and then processed as described with FIG. 56 to obtain the mixture. One or more additional mixtures may also be prepared. The mixture and the one or more additional mixtures each may have the same quantity of eccDNA from the biological sample. The process may include dividing the eccDNA in the biological sample into the mixture and the one or more additional mixtures. For example, the biological sample may be divided into aliquots of equal volume or mass. The known quantity of circular DNA of the second size may be added to a first mixture. The additional known quantities of the circular DNA may be added to the one or more additional mixtures. For example, different known quantities may be added to each mixture. The known quantity may include 0 pg, 1 pg, 2 pg, 3 pg, 4 pg, 5 pg, 6 pg, 7 pg, 8 pg, 9 pg, 10 pg, 50 pg, 0.1 ng, 0.2 ng, 0.3 ng, 0.4 ng, 0.5 ng, 0.6 ng, 0.7 ng, 0.8 ng, 0.9 ng, 1 ng, 2 ng, 3 ng, 4 ng, 5 ng, 6 ng, 7 ng, 8 ng, 9 ng, 10 ng, or a mass within a range specified by within any two of these masses.

The same size of circular DNA may be added to each mixture. The size may be a size including, but not limited to, 100 nt, 200 nt, 500 nt, 1000 nt, 2000 nt, 3000 nt, 4000 nt, 5000 nt, or a size within a range specified by within any two of these sizes.

The mixture and the one or more additional mixtures may be processed as described with FIG. 56. The mixture and the one or more additional mixtures may be treated with an enzyme to remove linear DNA. The enzyme may be a nuclease (e.g., Exonuclease V). The eccDNA and the circular DNA in the mixture and the one or more additional mixtures may be linearized to form linearized eccDNA and linearized circular DNA. The linearizing may be through tagmentation. The linearizing may also be rolling circle amplification followed by sonication. Linearizing may include additional amplification. The linearized eccDNA may be sequenced to obtain the first set of sequence reads and one or more third sets of sequence reads for each of the one or more additional mixtures. The linearized circular DNA may be sequenced to obtain the second set of sequence reads and one or more fourth sets of sequence reads for each of the one or more additional mixtures.

At block 6020, sizes of the cell-free DNA fragments are measured using the first set of sequence reads. These fragments include the fragments from the eccDNA and from the spiked-in circular DNA. The sizes of the cell-free DNA fragments may be measured using alignment of the fragments to a reference genome. The genomic positions of the outermost nucleotides at the ends of a fragment may be determined. The size of the fragment may be calculated using the difference between the genomic positions. In some embodiments, the fragment may be sequence, and the size may be determined by counting the nucleotides in the fragment.

At block 6030, a first amount of the first set of sequence reads corresponding to a first size is determined. The first size may be fragments from the eccDNA and not the circular DNA. The first size may be a specific size or a size range. For example, the size range may be a range from 50 bp to about 250 bp, about 50 bp to about 100 bp, about 100 bp to about 150 bp, about 150 bp to about 200 bp, or from about 200 bp to about 250 bp, about 250 bp to about 300 bp, 300 bp to about 350 bp, about 350 bp to about 400 bp, about 400 bp to about 450 bp, or about 450 bp to about 500 bp, about 500 bp to about 550 bp, 550 bp to about 600 bp, 600 bp to about 650 bp, 500 to about 650 bp, 650 bp to about 700 bp, 700 bp to about 750 bp, 750 bp to about 800 bp, 700 to 800 bp, or 800 to 850 bp The first amount may be a number of fragments or a total length of fragments.

At block 6040, a second amount of the first set of sequence reads corresponding to a second size is determined. The second size may be the particular size of the known quantity of circular DNA in the mixture. The second size may be any size described herein for the sizes of circular DNA. The second size may be a size that is different from sizes resulting from fragments of eccDNA.

At block 6050, the first amount is compared to a calibration data point. The calibration data point may be determined using the second amount of a second set of sequence reads corresponding to the second size. The calibration data point may include a coordinate for the amount as the number of sequence reads and another coordinate for the quantity in the mixture or the biological sample. The calibration data point may be a point of a calibration curve. The calibration curve may be determined using one or more additional amounts of sequence reads corresponding to the second size. Each of the one or more additional amounts may correspond to one or more additional known quantities of the circular DNA in the one or more additional mixtures. The additional known quantities may be different from the known quantity. The known quantity and additional known quantities may be any described herein.

The calibration curve may be a curve determined by a plurality of calibration data points. The plurality of calibration data points may be from a plurality of amounts of sequence reads and a plurality of known quantities of circular DNA. The calibration curve may be a curve that relates the amounts of sequence reads to the known quantities. The calibration curve may be a fit or a regression to a plurality of calibration data points. In some embodiments, the calibration curve may be a function relating amounts of sequence reads to quantities of the circular DNA in a mixture or a biological sample.

At block 6060, a quantity of cell-free DNA fragments from eccDNA corresponding to the first size in the mixture is determined using the comparison. The quantity may be a mass, number of fragments, or length of fragments. The determination of the quantity may include taking the known quantity associated with the calibration data point and adjusting the known quantity by factors including sizes of eccDNA, sequencing depth, sequencing length, DNA mappability, and PCR duplication rates. For example, the known quantity associated with the calibration data point may be multiplied by a ratio of the size of the eccDNA of interest over the size of the circular DNA.

The quantity of sizes of eccDNA other than the first size may be determined. The calibration data point may be a first calibration data point. The quantity may be a first quantity. The known quantity of the first calibration data point may be a first known quantity. A third amount of sequence reads corresponding to a third size of cell-free DNA fragments from eccDNA in the mixture may be determined. The third size may be different from the first size and the second size. The third amount may be compared to a second calibration data point. The second calibration data point may be determined using a fourth amount of a third set of sequence reads corresponding to the second size of the added circular DNA. For example, the second calibration data point may relate the fourth amount with a second known quantity of the second size. The second known quantity and the third amount may be from a second mixture. The third amount may be closer to the fourth amount than the second amount, so the second calibration data point is used in place of or in addition to the first calibration data point. A second quantity of cell-free DNA fragments from eccDNA corresponding to the third size may be determined using the comparison. In some embodiments, the eccDNA of the third size may be in a different mixture than the eccDNA of the first size.

A value of a parameter may be determined using the quantity of cell-free DNA fragments from eccDNA corresponding to the first size in the mixture. The parameter may be a normalized value of the quantity. For example, the parameter may be the quantity divided by the volume or mass of the mixture or the biological sample. The parameter may be a concentration. In some embodiments, the parameter may be determined using one or more physical characteristics of the subject from which the biological sample is obtained. For example, the parameter may use the weight or height of the subject. The value of the parameter may be determined using the second quantity of the cell-free DNA fragments from eccDNA corresponding to the third size. Additional sizes in a size range may be used for the parameter, including any size range described herein. In some embodiments, the parameter may be the quantity of a size or sizes.

The value of the parameter may be compared to a reference value to determine a classification of whether a the gene exhibits the genetic disorder in the subject. The gene and genetic disorder may be any described herein. The reference value may be determined from subjects with the gene not exhibiting a genetic disorder for a gene associated with a nuclease or from subjects exhibiting the genetic order. The reference value may be a cutoff value or a threshold value indicating a statistically different value of the parameter for the reference subjects. The genetic disorder may be characterized by deficiency in the nuclease. The classification may be that the gene exhibits the genetic order if the value of the parameter is greater than the reference value or if the value of the parameter is less than the reference value.

Embodiments may also include treating the genetic disorder. Treatment may include any treatment described herein.

Process 6000 may include additional implementations, such as any single implementation or any combination of implementations described below and/or in connection with one or more other processes described elsewhere herein.

Although FIG. 60 shows example blocks of process 6000, in some implementations, process 6000 may include additional blocks, fewer blocks, different blocks, or differently arranged blocks than those depicted in FIG. 90. Additionally, or alternatively, two or more of the blocks of process 6000 may be performed in parallel.

### D. Materials and methods

Experiments were performed using both mice models and human subjects to study the differences related to differences in the *DNASE1L3* gene.

### 1. Animal models

Mice with deletion of the *Dnase1* gene *(Dnase1*^{*-*/*-*}) were obtained from the Knockout Mouse Project Repository of the University of California at Davis; mice with deletion of the *Dnase1l3* gene (*Dnase1l3*^{-/-}) were obtained from the Jackson Laboratory. Mice were maintained in the Laboratory Animal Center of The Chinese University of Hong Kong (CUHK) with all experimental procedures approved by the Animal Experimentation Ethics committee of CUHK in compliance with the Guide for the Care and Use of Laboratory Animals (8th ed., 2011) established by the National Institutes of Health.

### 2. Human subjects

Four healthy human subjects were recruited with written informed consent. Three human subjects with *DNASE1L3* mutations were recruited from the Istituto Giannina Gaslini (Italy). One of these three *DNASE1L3*-mutated subjects provided blood samples at both pre- and post-hemodialysis timepoints. Thus, four blood samples in total were obtained from this patient cohort.

### 3. Mouse sample collection and processing

Blood samples were collected from 12 wild-type, 11 *Dnase1*^{*-*/*-*} and 11 *Dnase1l3*^{*-*/*-*}mice by cardiac punctures and centrifuged at 1,600 × g for 10 min at 4 °C, followed by another centrifugation step at 16,000 × g for 10 min at 4 °C of the plasma portion to remove cell debris. The buffy coat portion was centrifuged at 5,000 × g for 5 min at room temperature to remove residual plasma. Mouse liver tissues were collected and immediately stored at -80 °C. Plasma DNA was extracted using QIAamp Circulating Nucleic Acid Kits (Qiagen). Buffy coat (6 wild-type, 4 *Dnase1*^{*-*/*-*} and 5 *Dnase1l3*^{*-*/*-*} mice) and liver (5 wild-type, *5 Dnase1*^{*-*/*-*} and 5 *Dnase1l3*^{*-*/*-*}mice) tissue DNA was extracted using QIAamp DNA Mini Kits (Qiagen).

### 4. EccDNA library preparation and sequencing

EccDNA library constructions from plasma samples were performed using the tagmentation-based method as detailed previously (Sin et al., PNAS (2020)). For eccDNA enrichment from tissue DNA of the liver and buffy coat, we employed a dual size selection approach using solid phase reversible immobilization (SPRI) beads (Beckman Coulter).

**FIG. 56** illustrates the workflow of this approach. In step 5601, chromosomal DNA 5602 (large-sized molecules) was first removed using 0.5 × beads; small-sized DNA (linear 5603 and circular 5606) was then collected using 1.8× beads. This approach was performed three times on each sample for optimal selection outcome (step 5610). The size-selected tissue DNA was incubated with exonuclease V (New England Biolabs) in a 50 µL reaction system at 37 °C for 30 min for linear DNA removal (step 5615). The remaining DNA was collected by column purification using MinElute Reaction Cleanup Kit (Qiagen), followed by tagmentation (step 5620) or rolling circle amplification (step 5625) for eccDNA library construction. For tagmentation (step 5620), DNA samples were processed with Nextera XT DNA Library Preparation Kit (Illumina). For rolling circle amplification (step 5625), DNA samples were amplified using NxGen phi29 DNA Polymerase (Lucigen) at 30 °C for 12 hours, followed by sonication to 200 bp and sequencing adapter ligation. DNA libraries were sequenced on Illumina NextSeq 500 or NextSeq 2000 platforms as 2 × 75 bp or 2 × 150 bp paired-end reads.

The following provide more information regarding obtaining circular DNA. Additional information regarding circular DNA can be found in US Patent Publication No. 2020/0407799 A1, filed March 25, 2020.

In step 5615, the workflow first reduces (e.g., to essentially eliminate) linear DNA in the plasma DNA samples by exonuclease digestion (e.g., using exonuclease V). Other techniques can also be used to reduce linear DNA, e.g., cesium chloride-ethidium bromide (CsCl-EB) density gradient centrifugation.

We then followed up this with an approach to open up the circles (e.g., of eccDNA) to form linearized DNA molecules. The linearization of the eccDNA can be performed in various ways. In one example, we utilize restriction enzyme digestion to open up the circles at particular cleavage sites having a cutting sequence motif, which is a type of cutting tag. In another example, we use a transposase (e.g., via tagmentation [step 5620]) for opening up the circles, e.g., to insert a cutting tag that is recognizable like the cutting sequence motif for restriction enzyme digestion. Library preparation and next-generation sequencing of the resultant linearized DNA can then be performed.

Among the various examples using enzyme digestion, one implementation can use the restriction enzyme *Msp*I (cutting of CCGG sequence; methylation-insensitive). In another implementation, we used the restriction enzyme *Hpa*II (cutting of CCGG sequence; methylation-sensitive). In yet another implementation, we combined data generated through the use of *Msp*I and *Hpa*II to arrive at novel insights of eccDNA.

Restriction enzymes other than *Msp*I and *Hpa*II can be used. As an illustration, *Dpn*I and *Dpn*II, both recognize GATC sequence, could also be used. *Dpn*I cleaves only when the recognition site (A base) is methylated. On the other hand, *Dpn*II is not sensitive to methylation status. The number of bases recognized and cut can vary. For example, both *Msp*I and *Hpa*II are 4-base cutters. Restriction enzymes other than a 4-base cutter can be used, such as 6-base cutters.

When compared to rolling circle amplification of eccDNA (Shibata et al. Science. 2012;336:82-86) and shearing (e.g., by a nebulizer) to form linearized DNA, an approach using cutting tags (e.g., restriction enzyme or transposase approach) can provide more stringent criteria in the definition (identification) of eccDNA reads. For example, an eccDNA molecule can be accurately identified using two more anchors comprising the known sequence (cutting tag) where a cut has been made (e.g., CCGG fragment ends) and the absence of a gap between the two end sequences of the sequence read(s). Such a signature anchors can be used to accurately identify eccDNA reads and for determining their location in a reference genome. The absence of a gap can be determined using the reference genome via an alignment procedure, as described in more detail below.

This information from the cutting tag (e.g., CCGG read ends) not only facilitates more accurate identification of eccDNA, the complementing information provided by the number of eccDNA detected from methylation-insensitive and methylation-sensitive restriction enzymes also allows one to deduce the methylation levels of the eccDNA. Such information was not available through previously documented approaches. Moreover, the inexistence of CCGG fragment ends in the eccDNA fragments (or other recognition sequences specific for other types of restriction enzymes, i.e., other types of cutting tags) can provide insights of the pre-existence of eccDNA damage, which refers to linearization of eccDNA prior to restriction enzyme cutting. Such linearization might result from mechanical shearing during DNA processing, nuclease attacks in blood stream, etc. Such eccDNA molecules, although detected with junctional sites, often lack restriction enzyme cutting motifs at one or both ends of the fragment. Such cases can be referred to as "pre-existent eccDNA damage." Such information was also not obtainable by previously documented approaches. Such information could provide valuable knowledge for the biological mechanisms of eccDNA generation and processing *in vivo.*

The use of restriction enzyme digestion has been used in the creation of recombinant plasmids for molecular cloning. However, there are clear differences between such an application and the present disclosure. Firstly, eccDNA molecules are generated from the genome of organisms with clear start and end positions when mapped to the genome, whereas such concepts do not exist in a bacterial plasmid. Secondly, the restriction enzyme approaches for eccDNA study can provide insights of the host genome sequences. But for the bacterial plasmid DNA, restriction enzyme digestion approaches only allow one to peek into the plasmid DNA information and not the host genome itself (Shintani et al. Front Microbiol. 2015;31;6:242).

The restriction enzyme approach uses the presence of specific recognition sites on the eccDNA in order for its digestion and linearization. A tagmentation approach, which makes use of random cutting of DNA by a transposase, does not require specific DNA sequences. Therefore, the tagmentation approach could potentially provide a higher number of linearized eccDNA for library construction and sequencing. In a previous report, the use of tagmentation for eccDNA analysis in tissues was described (Shoura et al. G3 (Bethesda). 2017;7(10):3295-3303). Shoura et al used cesium chloride-ethidium bromide density gradient centrifugation to enrich eccDNA from tissue genomic DNA. In contrast, such a step does not need to be performed. Therefore, a tagmentation approach of the present disclosure can be more suitable for plasma DNA and other bodily fluids or stool that include circulating DNA.

### a) Principle and bioinformatics approach for eccDNA identification

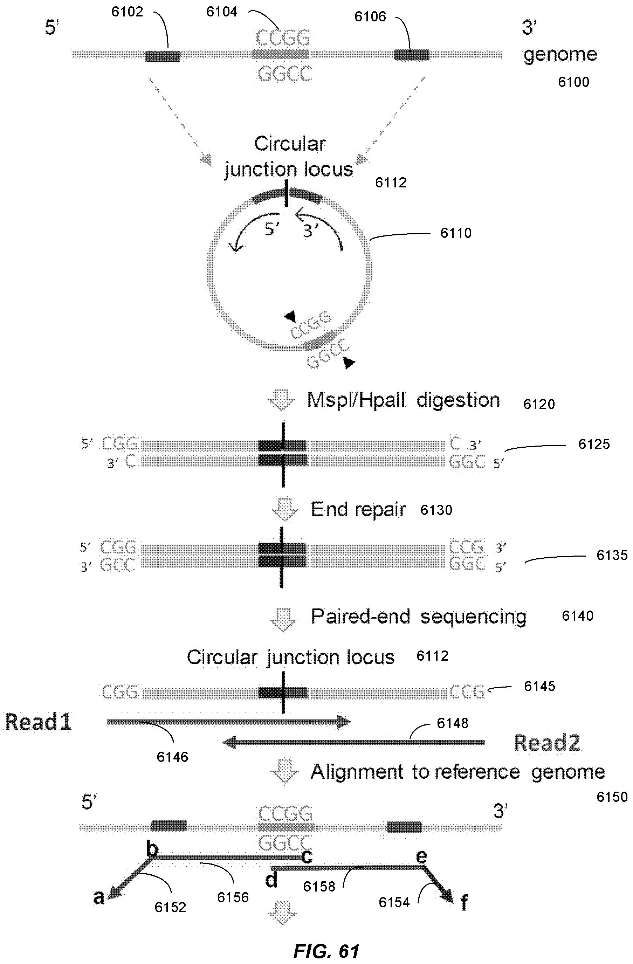

**FIG. 61** shows an example technique for eccDNA identification according to embodiments of the present disclosure. The "blue" region 6102 and the "red" region 6106 in genome 6100 indicate two regions that are assumed to be joined together to form extrachromosomal circular DNA (eccDNA). The "cyan" bar indicates a restriction enzyme recognition site 6104, which act as cutting tags. For example, the *Msp*I restriction enzyme could recognize and cleave CCGG sites. Such specific cutting would linearize the original circular DNA molecules. The resulting linearized molecules would carry staggered ends, which can be repaired though the end-repair step to form blunt-end molecules. Such blunt DNA ends would carry the cutting tag (i.e., 5' CGG and 3' CGG motifs). Subsequently, the blunt-end DNA could be sequenced using different sequencing technologies including, but not limited to, the Illumina platform, Ion Torrent sequencing, etc.

An eccDNA 6110 is shown having a circular junction locus 6112 that includes the two regions 6102 and 6106 from genome 6100. The ends of region 6102 and 6106 include nucleotides at two separated genomic locations that are immediately adjacent to one another in eccDNA 6110 to form circular junction locus 6112. At step 6120, digestion is performed at site 6104 to generate linearized DNA molecule 6125. At step 6130, end repair is performed, e.g., as described above, to generate end-repaired DNA molecule 6135. At step 6140, sequencing (e.g., paired-end sequencing or single molecule sequencing) is performed to obtain sequence 6145, which includes circular junction locus 6112. As shown, sequence 6145 can include read1 and read2.

If we sequenced read1 and read2 with a sufficient read length, there is a high likelihood to have sequence reads across the circular junction locus 6112 (indicated by the chimeric arrows) in the step of paired-end sequencing. Read1 extends from the left end of linearized DNA molecule 6125, where read1 is blue on the left side of circular junction locus 6112 and red to the right of circular junction locus 6112. Read2 extends from the right end of linearized DNA molecule 6125, where read2 is red on the right side of circular junction locus 6112 and blue to the left of circular junction locus 6112.

At step 6150, alignment is performed to the reference genome. When read1 and/or read2 cover the circular junction locus 6112, in the alignment results, we would observe read1 and read2 sequences of linearized molecules (e.g., cutting by *Msp*I) mapping to a reference genome in unique mapping directionalities. For illustration purpose, we define an unmapped segment 6152 (red arrow after the alignment step, "b→a" segment) in read1, which would correspond the sequence across the junction derived from the other genomic region being joint to form a circular DNA. Similarly, we define an unmapped segment 6154 (blue arrow after the alignment step, "e→f" segment) in read2, which would correspond the sequence across the junction derived from the other genomic region being joint to form a circular DNA molecule.

Such unique mapping directionalities are covered by the below two scenarios that involve a reversed direction between the read and the reference genome:
a. Read1 would be aligned in a reversed strand and read2 would be aligned in a forward strand when read1 smallest mapping coordinate of segment "b→c" (i.e., b) is equal to or smaller than read2 smallest mapping coordinate of segment "d→e" (i.e., d).
b. Read1 would be aligned in a forward strand and read2 would be aligned in a reversed strand when read2 smallest mapping coordinate is equal to or smaller than read1 smallest mapping coordinate (not shown in FIG. 61).

Such unique mapping directionalities were different from conventional mapping directions for a pair of paired-end reads originating from an initially linear DNA. Thus, such criteria can be used to identify a circular molecule. For example, read1 is fully aligned in a forward strand and read2 is fully aligned in a reversed strand when read1 smallest mapping coordinate is equal to or smaller than read2 smallest mapping coordinates; or read1 is fully aligned in a reversed strand and read2 is fully aligned in a forward strand when read2 smallest mapping coordinates are equal to or smaller than read1 smallest mapping coordinates. Bioinformatically, searching the mapping sites in the reference genome of the unmapped segments present in read1 and/or read2 would allow for delineating the junctions. The distance between junction sites deduced from the unmapped segments from a fragment would indicate the size of a circular DNA. For example, the distance between region 6102 and site 6104 provide the size of the circular DNA.

Another feature is that there were two nucleotides overlapped between the mapped read1 and read2 if a circular DNA was cut only once. Such two nucleotides overlapped sequence between read1 and read2 was introduced by the staggered ends (i.e. jagged end) created by *Msp*I or *Hpa*II, or other digestion enzyme. *Msp*I or *Hpa*II would make two staggered single-stranded breaks and the distance between two breaks would be 2 bp. Such 5' protruding 2-nt single-stranded ends (complementary to each other) would be filled to form blunt ends during the end-repair step. Therefore, the resultant DNA sequences would carry 2 bp overlap between ends of read1 and read2 sequences. In other words, during the library preparation step, there will be an "end repair" step, which will complete the jagged ends into blunt ends by adding two nucleotides to each end. Therefore, the resultant DNA sequences will have two blunt ends instead of two jagged ends. When the two sequencing reads are aligned to the genome, the two nucleotides added during the end repair steps will appear as two extra base pairs that overlap between two reads, which can be used in addition or alternatively to identify a circular NDA molecule.

Taken together, in an example eccDNA identification approach, there can be four "diagnostic features", including:
a. Circular DNA specific mapping directions (directionality), as provided in (a) and (b) above;
b. Junction-aware reads (only a portion of an ending sequence mapping to the reference genome);
c. Restriction enzyme cutting tags;
d. Two overlapped bases in 5' ends of read1 and read2 sequences.

Such diagnostic features can greatly improve the specificity in identifying the genome-wide eccDNA molecules in plasma DNA. In some implementations, sequencing reads fulfilling at least one of these "diagnostic features" can be defined as a candidate circular DNA. For a circular DNA being cut multiple times by a restriction enzyme, read1 and read2 would not bear repeated sequences (overlapped bases) between each other. In other implementations, only one read from a pair might cross the junction site and the other would not carry the junction. As another example, both reads from a pair would not carry a junction, but show unique mapping directions implying a circular DNA. In yet another example, even though one could not directly observe the complete restriction enzyme cutting tags in the sequencing reads, one could retrieve the reference sequence from the reference genome between these deduced junction sites of one circular DNA. Then one could bioinformatically investigate if any restriction enzyme cutting tags (motifs) exists in such a retrieved reference sequence. Such inferred restriction enzyme cutting motifs would increase the confidence that the identification of a circular DNA species was indeed correct.

Accordingly, a method can use a restriction enzyme as part of analyzing eccDNA. Such a technique can be used in combination with other methods described herein, e.g., for analysis of eccDNA as well as mtDNA. Downstream analysis can include measurement of properties of the sample using the detection of the circular DNA.

In a first step, a biological sample of an organism can be received. Examples of biological samples are provided herein, such as plasma and serum. The biological sample includes a plurality of extrachromosomal circular DNA (eccDNA) molecules. The eccDNA may be from any number of chromosomes, including the autosomes and/or sex chromosomes. Each of the plurality of eccDNA molecules includes a junction at which nucleotides at two separated genomic locations are immediately adjacent to one another. Circular junction locus 6112 is an example of such a junction with regions 6102 and 6106 including such two separated genomic locations that are immediately adjacent to one another.

In a second step (e.g., step 6120), digestion is performed using a restriction enzyme. In some implementations, more than one type of restriction enzyme can be used. Digesting the plurality of eccDNA molecules can form a set of linearized DNA molecules that each includes the junction. Each restriction enzyme can cut at a different motif, with the resulting linearized DNA fragments having a different cutting tag. The term "linearized DNA fragments" differs from a "linear DNA fragment," which was already linear before any digestion.

In a third step (e.g., step 6140), for each of the linearized DNA molecules, sequencing of at least both ends of the linearized DNA molecules can be performed to obtain one or more sequence reads. The one or more sequence reads may or may not include the junction. If a read does not include the junction, an eccDNA molecule can still be identified using the directionality of the mapping. In some embodiments, two sequence reads (one for each end) can be obtained. In other embodiments, a single sequence read of the entire linearized DNA molecule can include both ends, as is described herein.

After the sequence reads are obtained, the sequence reads can be mapped (aligned) to a reference genome, e.g., to see if they map in a reverse orientation. If they do map in a reverse orientation (example criterion), then the correspond linearized DNA molecule can be identified as originally being circular. Accordingly, for each of the linearized DNA molecules, a pair of end sequences for the linearized DNA molecule from the one or more sequence reads can be selected. The pair of end sequences do not include the junction. An example of such end sequences are end sequence 6146 and end sequence 6148 in FIG. 61. A direction of each of the pair of end sequences is reversed to obtain a pair of reversed end sequences. An example of such reversed end sequences are reversed end sequence 6156 and reversed end sequence 6158. The pair of reversed end sequences can then be mapped to a reference genome.

The mapped reversed end sequences can be analyzed to measure a property of the biological sample. Examples of such measurements are provided herein. Such analysis can use a collective value (e.g., count, size, or methylation) of the detected eccDNA. Accordingly, the method can further include identifying the linearized DNA molecule as originating from an eccDNA molecule based on the pair of reversed end sequences mapping to the reference genome, and determining a collective value of the identified eccDNA molecules, wherein analyzing the mapped reversed end sequences to measure the property of the biological sample uses the collective value.

### b) Identification technique

As explained above, various criteria can be used to identify the circular DNA molecules. Additionally, various procedures may be used in the analysis of the raw sequence reads (e.g., read1 and read2 from FIG. 61) to identify one or more of the properties of circular DNA.

The raw sequence reads can be pre-processed. For example, the duplicated reads, sequencing adapters, and low-quality bases on the 3' end of a sequencing read can be removed. Further, a specified number of bases of paired-end reads (or from the ends of a single-molecule read) can be selected for alignment.

### (1) Putative eccDNA identification

The bioinformatically truncated read1 and read2 consisting of the first 50 bp of read1 and read2 in pre-processed paired-end reads can be used for alignment to a human reference genome using an alignment procedure, e.g., Bowtie 2 (Langmead et al. Nat Methods. 2012;9:357-9) in a paired-end mode. Other alignment techniques can also be used. Other lengths of each read may be used besides 50 bp, e.g., at least 20, 25, 30, 35, 40, or 45 bp. A first pass at alignment can try a standard orientation, e.g., read1 is aligned with the left end at a lower genomic position than the last based in the read. For those paired-end reads that are aligned normally (i.e., in a forward direction), the mapping directionality regarding read1 and read2 would be determined in a first pass. In contrast to conventionally properly mapped paired ends, if a fragment's read1 and read2 corresponded to circular DNA, the forward orientation would not provide proper alignment of the pair, as such reads have circular DNA specific mapping directions (FIG. 61).

If the pair of reads are not aligned with a forward orientation, a reverse orientation can be tried in a second alignment pass. As shown in FIG. 61, read1 and read2 are reversed. If the truncated reads can be aligned in a reverse orientation, then the corresponding reads before truncating can be re-aligned to the reference genome. The non-truncated reads may be needed so that they cover the junction. If the read does cover a junction, then it would not fully align to the reference genome, even in a reverse direction, e.g., as shown in FIG. 61. The paired-end reads with at least one read which was not able to be aligned to the reference genome in its full length can be used for the downstream detailed analysis of "diagnostic features" (e.g., 4 above) for eccDNA because such a read that was not able to align the reference genome in an end-to-end mode suggests a junction. These paired-end reads can be deemed as putative reads originating from circular DNA molecules.

### (2) Probing the junctions of eccDNA molecules

To accurately locate the genomic location of an eccDNA with single base resolution, some implementations fine-tuned the realignment for putative read, separately. Taking read1 as an example, the first 20 bp and the last 20 bp from read1 sequences were used as seeds (seed A and seed B, respectively) to determine the candidate genomic regions perhaps carrying a junction. The shortened reads used for searching candidate locations helped to minimize the likelihood a read contained a junctions, which would affect the alignment accuracy and the precise determination of a junction site. In this step, multiple hits (e.g., no more than 10 hits for each seed) may be allowed, so as to maximize the sensitivity to detect the junctions. If seed B sequence was not placed in the downstream of seed A mapping position in the same direction, it would suggest that such read1 would carry a junction.

Next, we used a searching approach to probe the junction in a single base resolution for the read1 that was identified as potentially carrying a junction.

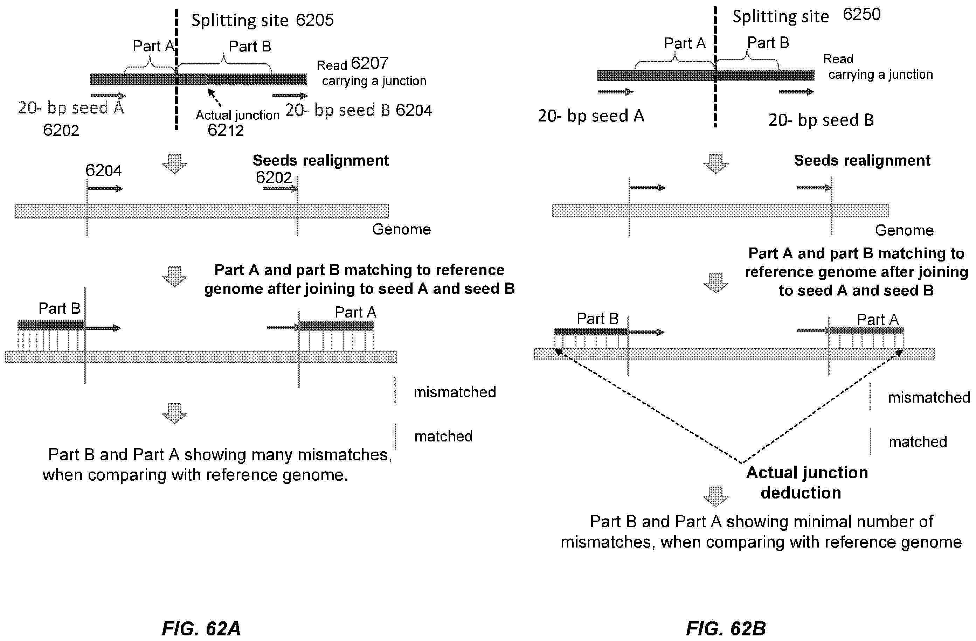

**FIGS. 62A** and **62B** show a schematic approach for junction searching approach according to embodiments of the present disclosure. The search is performed within a read after alignment to the reference genome, e.g., as shown after step 6150 in FIG. 61. The read 6207 carrying the junction contains two segments (red and blue) of opposite mapping directions, e.g., as shown in FIG. 61.

In FIGS. 62A and 62B, the searching was conducted in a "splitting and matching" manner. We used "splitting site" 6205 (as indicated by black dash line) to divide the original read1 sequence into two parts, namely, part A and part B. We iteratively slid the "splitting site" 6205 along the whole read except for seed regions 6202 and 6204 (e.g., of length 20 bp), so as to exhaust all combinations of part A and part B. The sequence to the left of "splitting site" 6205, but not including the seed region 6202, is part A. The sequence to the right of "splitting site" 6205, but not including the seed region 6204, is part B. The minimum length for each of part A and part B can be constrained, e.g., not less than 18 bp.

FIG. 62A shows an example where "splitting site" 6205 does not overlap with the actual junction 6212. After splitting the read, the seed regions 6202 and 6204 can be realigned, as shown. Then, the part A and part B can respectively be joined, as shown. When the "splitting site" 6205 did not overlap with the actual junction 6212, part A and part B would show many mismatches if we compared part A and part B to a reference genome after part A and part B were pasted to seed A and seed B, respectively.

FIG. 62B shows an example when the "splitting site" did exactly overlap with the actual junction 6212. Part A and part B would show zero mismatch in theory if we compared part A and part B to a reference genome after part A and part B were pasted to seed A and seed B, respectively. Therefore, the "splitting site" 6250 in read1 sequence giving a minimum of mismatch among all combinations of part A and part B was identified as a junction. Such a minimum can satisfy a mismatch condition. In other implementations, a seed can be extended until a specified number (e.g., two or more) consecutive positions mismatch with the reference.

Such searching was also applied to read2 sequences independently. The read2 sequence would be used for further improving the specificity. For example, the read2 sequence would have two scenarios: (1) read2 sequence carried a junction as read1. Such junction information should be compatible with the results deduced from read1 sequence. (2) read2 sequence did not carry a junction. In this case, read2 sequence should be fully aligned within the regions demarcated by the sequences at either end of the junction site, which was deduced from the read1 sequences (i.e., part A and part B). The processing orders for read1 and read2 would be exchangeable. In yet another embodiment, the total number of mismatches along the whole read carrying the deduced junction was required to be no more than a specified number (e.g., 2).

### 5. EccDNA identification and size profiling

Details of the bioinformatics principles for mouse eccDNA identification and size profiling were modified from a previous study (Sin et al., PNAS (2020)) with minor adjustments, including the fact that mouse genomes were used as reference genomes. For the mouse pregnancy model, mating pairs were set up as follows: female mice of the C57BL/6 genomic background (wild-type or *Dnase1l3-*/*-*) were crossed with male mice from either the BALB/c (wild-type) or the C57BL/6 (*Dnase1l3-*/*-*) genomic background (FIG. 51). Sequencing data of eccDNA libraries from the pregnant mice were first mapped against the C57BL/6 reference genome (NCBI build 38/UCSCmm10) for candidate eccDNA identification. The resultant candidate eccDNA reads were subsequently mapped against the BALB/c genome. Only the candidate reads identified as eccDNA under both C57BL/6 and BALB/c genomes were selected for downstream analyses. A database containing 4,576,884 SNPs that differ between the C57BL/6 and the BALB/c genomes was obtained from the Mouse Genomes Project (https://www.sanger.ac.uk/science/data/mouse-genomes-project). Because all female mice were from the C57BL/6 strain, any eccDNA harboring BALB/c-specific alleles would be designated as fetal-specific molecules. The remaining molecules covering the same allele with shared SNPs would be assigned as shared molecules, which would predominantly be of maternal origin.

### 6. Statistical analysis

Kruskal-Wallis test followed by Dunn's Multiple Comparison Test was applied to compare three groups of data. Wilcoxon rank-sum test was applied to compare two groups of data. These statistical tests were performed using GraphPad Prism 8.0 (GraphPad Software). Statistical significance was defined as P < 0.05.

### III. TREATMENT

Embodiments may further include treating the genetic disorder or low nuclease activity (e.g., lower than a threshold) in the patient after determining a classification for the subject. The classification for the subject after treatment may or may not involve adding anticoagulants in vivo or in vitro to enhance the cfDNA end profile. Further, the treatment can be determined as an alternative to a current treatment (e.g., an anticoagulant) when the current dosage has low efficacy, e.g., an increase in dosage or a different anticoagulant can be used. Treatment can be provided according to a determined level of a disorder, any identified mutations, and/or a tissue of origin. For example, an identified mutation (e.g., for polymorphic implementations) can be targeted with a particular drug or chemotherapy. The tissue of origin can be used to guide a surgery or any other form of treatment. And, the level of a disorder can be used to determine how aggressive to be with any type of treatment, which may also be determined based on the level of disorder. A disorder (e.g., cancer) may be treated by chemotherapy, drugs, diet, therapy, and/or surgery. In some embodiments, the more the value of a parameter (e.g., amount or size) exceeds the reference value, the more aggressive the treatment may be.

Treatment may include chemotherapy, which is the use of drugs to destroy cancer cells, usually by keeping the cancer cells from growing and dividing. The drugs may involve, for example but are not limited to, mitomycin-C (available as a generic drug), gemcitabine (Gemzar), and thiotepa (Tepadina) for intravesical chemotherapy. The systemic chemotherapy may involve, for example but not limited to, cisplatin gemcitabine, methotrexate (Rheumatrex, Trexall), vinblastine (Velban), doxorubicin, and cisplatin.

In some embodiments, treatment may include immunotherapy. Immunotherapy may include immune checkpoint inhibitors that block a protein called PD-1. Inhibitors may include but are not limited to atezolizumab (Tecentriq), nivolumab (Opdivo), avelumab (Bavencio), durvalumab (Imfinzi), and pembrolizumab (Keytruda).

Treatment embodiments may also include targeted therapy. Targeted therapy is a treatment that targets the cancer's specific genes and/or proteins that contributes to cancer growth and survival. For example, erdafitinib is a drug given orally that is approved to treat people with locally advanced or metastatic urothelial carcinoma with FGFR3 or FGFR2 genetic mutations that has continued to grow or spread of cancer cells.

Some treatments may include radiation therapy. Radiation therapy is the use of high-energy x-rays or other particles to destroy cancer cells. In addition to each individual treatment, combinations of these treatments described herein may be used. In some embodiments, when the value of the parameter exceeds a threshold value, which itself exceeds a reference value, a combination of the treatments may be used.

### IV. EXAMPLE IMPLEMENTATION DETAILS

Experimental techniques used in studying the nuclease effect on linear cfDNA and eccDNA are descirbed. These techniques can be applied to any method described herein.

In example murine models, mice with a CRISPR/Cas9-targeted deletion of exon 5 in *Dnase1l3* (mm9 Chr14: 8,809,531 - 8,810,216) on a C57BL/6NJ background were generated by The Jackson Laboratory. Mice carrying a targeted allele of *Dnase1* [Dnaseltm1.1(KOMP)Vlcg] on B6 background and WT control mice on B6 background were obtained from the Knockout Mouse Project Repository of the University of California at Davis. All experimental procedures were approved by the Animal Experimentation Ethics committee of The Chinese University of Hong Kong (CUHK) and performed in compliance with "Guide for the Care and Use of Laboratory Animals" (8th edition, 2011) established by the National Institutes of Health. The mice were maintained in the Laboratory Animal Center of CUHK. Male and female mice aged 14-20 weeks were used for experiments. An analysis on the influence of sex and gender on the results was not done since their blood samples were pooled together.

In example murine sample collection, mice were euthanized and exsanguinated by cardiac puncture. Whole blood was placed into EDTA-containing tubes (1.3 mL K3E microtubes from Sarstedt) and immediately separated by a double centrifugation protocol (1,600 x *g* for 10 minutes at 4 °C, then recentrifugation of the plasma at 16,000 x *g* for 10 minutes at 4 °C) (Chiu et al., 2001). Plasma from 3-4 mice were collected into each pool, yielding 1.1-1.9 mL plasma per pool. In total, we created 6 pools of WT from plasma of 20 WT mice, 6 pools of *Dnase1l3*^{*-*/*-*}from plasma of 20 *Dnase1l3*^{*-*/*-*} mice, and 2 pools of *Dnase1*^{*-*/*-*} from plasma of 8 *Dnase1*^{*-*/*-*}mice.

In example human subjects, 3 subjects (H2, H4, and V11) were recruited with *DNASE1L3* deficiency and 1 heterozygous parent (H1) from the Istituto Giannina Gaslinin (Italy) and The Hospital for Sick Children (SickKids) (Canada) with written informed consent. The 3 *DNASE1L3-*deficient subjects (H2, H4, and V11) have homozygous frameshift c.290_291delCA (p.Thr97Ilefs*2) mutation, and H1 is the heterozygous parent of H2 and H4. Plasma data of 8 healthy individuals from a previously published dataset were used as controls (Chan et al., 2013). Plasma was collected for all human subjects, but paired buffy coat was available only for H1, H2, and H4. The study was approved by the Joint Chinese University of Hong Kong-Hospital Authority New Territories East Cluster Clinical Research Ethics Committee, the ethics committee of the Istituto Giannina Gaslini (approval BIOL 6/5/04), and the SickKids Research Ethics Board.

### A. DNA extraction and bisulfite DNA sequencing

In an example, plasma DNA was extracted with the QIAamp Circulating Nucleic Acid Kit (Qiagen), and buffy coat DNA was extracted with the QIAamp DNA Blood Mini Kit (Qiagen) then sonicated to a median size of 350 bp (Covaris). Indexed DNA libraries were constructed using the TruSeq DNA Nano Library Prep Kit (Illumina) with bisulfite modification using the EpiTect Bisulfite Kit (Qiagen). The bisulfite-converted DNA libraries were enriched with 12 cycles of PCR and analyzed on Agilent 4200 TapeStation (Agilent Technologies) using the High Sensitivity D1000 ScreenTape System (Agilent Technologies) for quality control and gel-based size determination. Libraries were quantified by the Qubit dsDNA high sensitivity assay kit (Thermo Fisher Scientific) before sequencing. 2 x 75 bp paired-end sequencing was performed on the HiSeq 4000 platform (Illumina) for the plasma libraries and on the NextSeq 500 platform (Illumina) for the buffy coat libraries.

### B. Quality control, trimming, and alignment of bisulfite sequencing data

In an example, sequences were assigned to their corresponding samples based on their six-base index sequence. The adapter sequences were removed and low quality bases with Phred score below 20 were trimmed from the paired-end bisulfite sequencing reads. Cleaned reads were aligned to the reference genome (mouse: NCBI MGSCv37/UCSC mm9; human: NCBI GRCh37/USCS hg19; non-repeat-masked) with a maximum of two mismatches. Paired-end reads sharing the same start and end genomic coordinates were deemed PCR duplicates and were discarded from downstream analysis. The methylation densities of all CpG sites across the genome were generated by Methy-Pipe (Jiang et al., 2010).

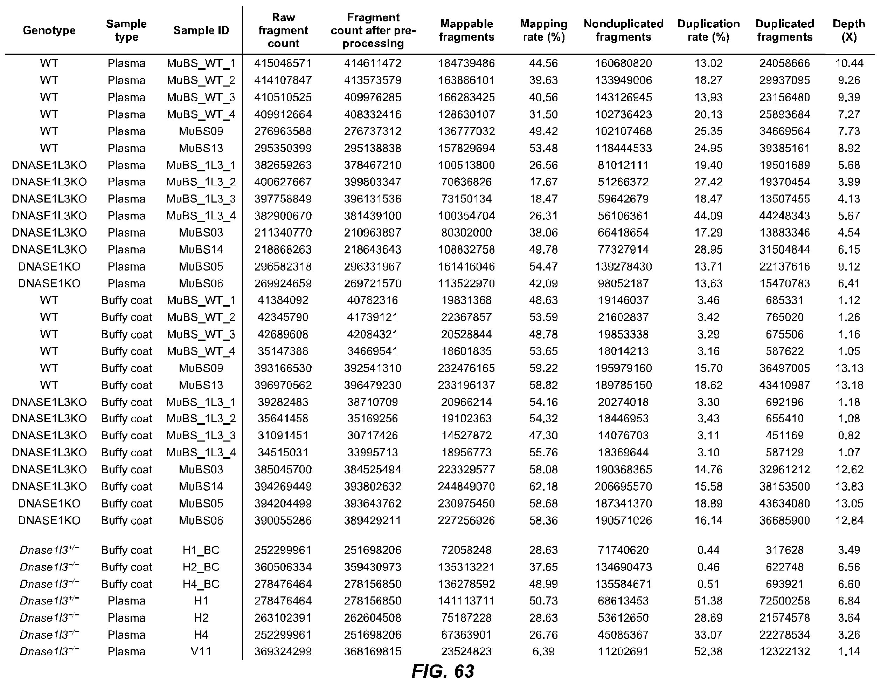

**FIG. 63** summarizes the number of unique fragments obtained for each condition. The first column lists the genotype. The second column lists the sample type. The third column is the sample identification. The fourth column is the raw fragment count. The fifth column is the fragment count after pre-processing. The sixth column is the number of mappable fragments. The seventh column is the mapping rate (mappable fragments divided by fragment count after pre-processing). The eighth column is the number of non duplicated fragments. The ninth column is the duplication rate. The 10^{th} column is the number of duplicated fragments. The 11^{th} column is the sequencing depth.

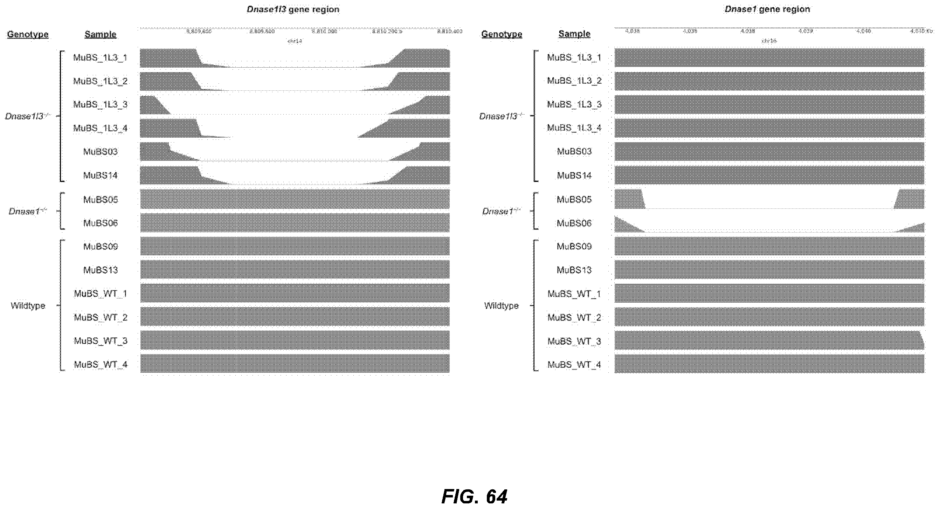

**FIG. 64** shows that the the deletions of the *Dnase1* and *Dnase1l3* genes in the mouse data were confirmed in the aligned data. The left panel shows the *Dnase1l3* gene region. The right panel shows the *Dnase1* gene region. The different samples are listed in the different rows. The colored bar in each row represents the presence of fragments aligning to that region. The left panel shows that the samples from the *Dnase1l3*^{*-*/*-*} genotype have deleted regions, unlike samples from the *Dnase1*^{*-*/*-*} and wildtype genotypes. The right panel shows that samples from *Dnase1*^{*-*/*-*}genotype have deleted regions, unlike samples from *Dnase1l3*^{*-*/*-*} and wildtype genotypes.

### C. Calculation of end density and methylation level around different regions

In an example, RNA polymerase II (Pol II), H3K4me3, H3K27ac regions were downloaded from the Human and Mouse ENCODE project (Shen et al., 2012; Dunham et al., 2012). The transcriptional start sites (TSSs) of all genes and the CpG islands (CGI) were downloaded from UCSC. 10,000 random non-overlapping regions of 10,000 bp length were randomly selected across the whole genome by BEDTools (v2.27.1) (Quinlan and Hall 2010). Using a visualization window size of ± 1000 bp, the fragment end counts was normalized by the median end counts in the ± 3000 bp region to obtain the normalized end density. The methylation level of these regions were calculated from the CpG sites in the corresponding regions. The respective sample medians were calculated and plotted.

### D. cfDNA size of 0% and 100% methylated fragments

In an example, the genome coordinates of the aligned ends were used to deduce the size of the whole fragment of the sequenced cfDNA. To identify 0% and 100% methylated fragments, fragments with three or more CpG sites were used to calculate the methylation percentage. Those with zero out of at least three CpGs methylated were labelled as a 0% methylated fragment, and those with all out of at least three CpGs methylated were labelled as 100% methylated fragments. The median size of each genotype in these fragment types was plotted.

### E. OCR and CGI fragment analysis

In an example, the region ± 500 bp around the center of TSS, PoI II, H3K4me3 and H3K27ac regions were merged with CGI regions. Fragments were considered within these regions if at least one base overlapped with these regions. The fragment percentage and the size profile of the fragments within these regions were calculated, and the methylation level and size profile was recalculated after masking these regions. For the circos plot, the reference genome was split into 1 Mb bins, and each dot in the circos plot represents the methylation level of each bin deduced from all the CpG sites within the 1 Mb bin.

### F. Analysis of putatively methylated and unmethylated CpGs

In example murine models, whole-genome bisulfite sequencing (WGBS) data for 8 mouse tissues with 2 biological replicates were obtained from the ENCODE portal (https://www.encodeproject.org/) using the following identifiers: ENCFF874IPH, ENCFF249MKR, ENCFF916JME, ENCFF012ENO, ENCFF283GDL,ENCFF348XNA, ENCFF978EJO, ENCFF282MIR, ENCFF779LLA, ENCFF060ISR, ENCFF853NGK, ENCFF373MDU, ENCFF306KYH, ENCFF663AVX, ENCFF6781ZX, ENCFF918TYN, ENCFF098RUM, ENCFF585VLM, ENCFF847MPY, ENCFF980YJZ, ENCFF0730SB, ENCFF804QBF, ENCFF192LZC, ENCFF442AJP, ENCFF541AEY, ENCFF753BBR, ENCFF798LHE, ENCFF082ZSO, ENCFF623FPU, ENCFF422TOH, ENCFF240XBY, ENCFF566GDN, ENCFF340YVI, ENCFF703DEV, ENCFF802SFU, ENCFF306ZPW. WGBS data for 9 human tissues were obtained from the Roadmap Epigenomics Project using the following identifiers: GSM1010983, GSM1010981, GSM983648, GSM983649, GSM1010984, GSM983650, GSM916049, GSM983647, GSM983651, GSM1010987, GSM983645, GSM983646, GSM983652, GSM1120324, GSM1010978, GSM1058027, GSM1059433, GSM1120321. Alignment and methylation analysis of these dataset was performed by Bismark with the ENCODE WGBS single-end pipeline (Krueger and Andrews 2011).

Putatively unmethylated and methylated CpG sites were identified from these datasets with methylation level cutoffs at ≤ 20% and ≥ 90%, respectively. From the mouse dataset, 545,720 putatively methylated CpGs, and 7,140 putatively unmethylated CpGs were identified. From the human dataset, 439,114 putatively methylated CpGs were identified.

For the end density analysis, the respective CpG sites were aggregated and the normalized end density within ± 1000 bp and a ± 20 bp window is shown. The normalized end density is the end count divided by the median end counts of the ± 1000 bp region. Fragments with any of its bases covering either the C or G of the identified CpGs were used in the calculation of the CpG methylation at these putatively unmethylated or methylated CpG sites.

### G. Statistical analysis

Analysis was performed by in-house bioinformatics programs, which were written in Perl and R languages. A *P* value of less than 0.05 was considered statistically significant and all probabilities were two-tailed.

### V. EXAMPLE SYSTEMS

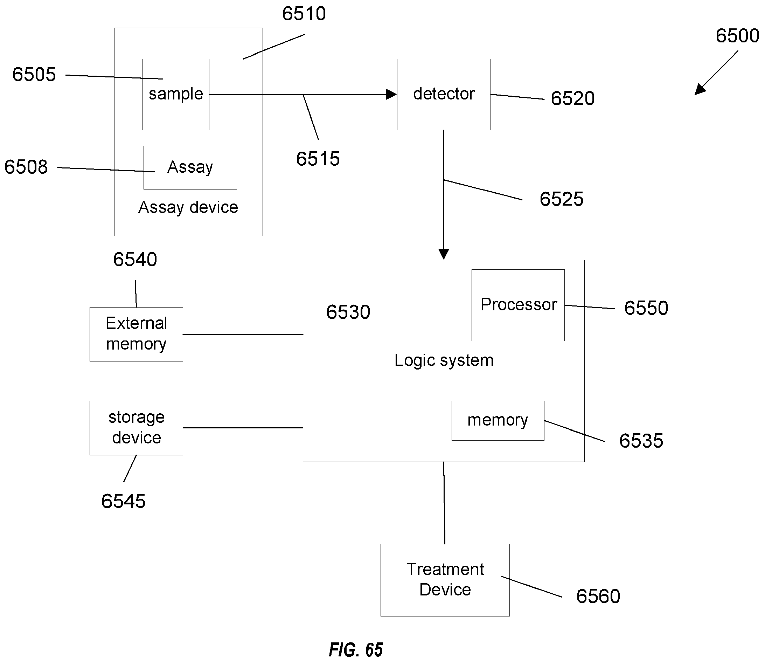

**FIG. 65** illustrates a measurement system 6500 according to an embodiment of the present disclosure. The system as shown includes a sample 6505, such as cell-free DNA molecules within an assay device 6510, where an assay 6508 can be performed on sample 6505. For example, sample 6505 can be contacted with reagents of assay 6508 to provide a signal of a physical characteristic 6515. An example of an assay device can be a flow cell that includes probes and/or primers of an assay or a tube through which a droplet moves (with the droplet including the assay). Physical characteristic 6515 (e.g., a fluorescence intensity, a voltage, or a current), from the sample is detected by detector 6520. Detector 6520 can take a measurement at intervals (e.g., periodic intervals) to obtain data points that make up a data signal. In one embodiment, an analog-to-digital converter converts an analog signal from the detector into digital form at a plurality of times. Assay device 6510 and detector 6520 can form an assay system, e.g., a sequencing system that performs sequencing according to embodiments described herein. A data signal 6525 is sent from detector 6520 to logic system 6530. As an example, data signal 6525 can be used to determine sequences and/or locations in a reference genome of DNA molecules. Data signal 6525 can include various measurements made at a same time, e.g., different colors of fluorescent dyes or different electrical signals for different molecule of sample 6505, and thus data signal 6525 can correspond to multiple signals. Data signal 6525 may be stored in a local memory 6535, an external memory 6540, or a storage device 6545.

Logic system 6530 may be, or may include, a computer system, ASIC, microprocessor, graphics processing unit (GPU), etc. It may also include or be coupled with a display (e.g., monitor, LED display, etc.) and a user input device (e.g., mouse, keyboard, buttons, etc.). Logic system 6530 and the other components may be part of a stand-alone or network connected computer system, or they may be directly attached to or incorporated in a device (e.g., a sequencing device) that includes detector 6520 and/or assay device 6510. Logic system 6530 may also include software that executes in a processor 6550. Logic system 6530 may include a computer readable medium storing instructions for controlling measurement system 6500 to perform any of the methods described herein. For example, logic system 6530 can provide commands to a system that includes assay device 6510 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

System 6500 may also include a treatment device 6560, which can provide a treatment to the subject. Treatment device 6560 can determine a treatment and/or be used to perform a treatment. Examples of such treatment can include surgery, radiation therapy, chemotherapy, immunotherapy, targeted therapy, hormone therapy, and stem cell transplant. Logic system 6530 may be connected to treatment device 6560, e.g., to provide results of a method described herein. The treatment device may receive inputs from other devices, such as an imaging device and user inputs (e.g., to control the treatment, such as controls over a robotic system).

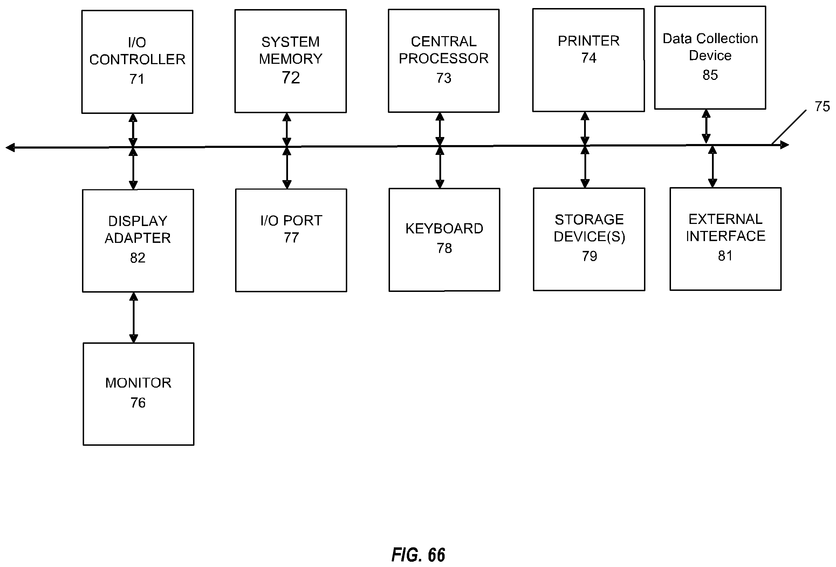

Any of the computer systems mentioned herein may utilize any suitable number of subsystems. Examples of such subsystems are shown in **FIG. 66** in computer system 10. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 66 are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire^{®}). For example, I/O port 77 or external interface 81 (e.g. Ethernet, Wi-Fi, etc.) can be used to connect computer system 10 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software stored in a memory with a generally programmable processor in a modular or integrated manner, and thus a processor can include memory storing software instructions that configure hardware circuitry, as well as an FPGA with configuration instructions or an ASIC. As used herein, a processor can include a single-core processor, multicore processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present disclosure using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk) or Blu-ray disk, flash memory, and the like. The computer readable medium may be any combination of such devices. In addition, the order of operations may be re-arranged. A process can be terminated when its operations are completed, but could have additional steps not included in a figure. A process may correspond to a method, a function, a procedure, a subroutine, a subprogram, etc. When a process corresponds to a function, its termination may correspond to a return of the function to the calling function or the main function

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

## Claims

1. A computer-implemented method for analyzing a biological sample of a subject including cell-free DNA, the method comprising:
identifying a first set of CpG sites that are all hypomethylated or all hypermethylated in a reference genome;
receiving sequence reads obtained from sequencing cell-free DNA fragments in the biological sample of the subject;
aligning the sequence reads to the reference genome to determine genomic positions in the reference genome corresponding to the cell-free DNA fragments;
determining, using the aligned sequence reads, a relative abundance of the cell-free DNA fragments covering the first set of CpG sites; and
comparing the relative abundance to a reference value to determine a level of a condition of the subject, wherein:
the level of the condition includes a nuclease activity;
the level of the condition is whether a gene associated with a nuclease exhibits a genetic disorder in the subject, or
the condition is cancer or an auto-immune disease.

2. The computer-implemented method of claim 1, wherein the level of the condition is whether a gene exhibits a genetic disorder in the subject, and wherein the gene is associated with a nuclease.

3. The computer-implemented method of claim 1, wherein the condition is cancer.

4. The computer-implemented method of claim 1, wherein the condition is an auto-immune disease.

5. The computer-implemented method of claim 1, wherein the level of the condition includes a nuclease activity.

6. The computer-implemented method of any one of claims 1 to 5, wherein the relative abundance of the cell-free DNA fragments is relative to a second set of CpG sites.

7. The computer-implemented method of claim 6, wherein the relative abundance of the cell-free DNA fragments is determined for the cell-free DNA fragments having a specified size.

8. The computer-implemented method of claim 7, wherein the specified size is a size range.

9. The computer-implemented method of any one of claims 7 to 8, wherein the relative abundance is an end density of sequence reads covering the first set of CpG sites.

10. The computer-implemented method of any one of claims 1 to 4, wherein the relative abundance of the cell-free DNA fragments is a statistical value of a size distribution of the cell-free DNA fragments covering the first set of CpG sites.

11. The computer-implemented method of claim 10, wherein the statistical value is a size ratio of a first amount of the cell-free DNA fragments covering the first set of CpG sites having a first size relative to a second amount of the cell-free DNA fragments covering the first set of CpG sites having a second size.

12. The computer-implemented method of any one of claims 1 to 11, wherein a site is determined to be hypomethylated in the reference genome by:
comparing a methylation level in the reference genome at the site to a threshold, and
determining the methylation level in the reference genome is below the threshold.

13. The computer-implemented method of any one of claims 1 to 11, wherein a site is determined to be hypermethylated in the reference genome by:
comparing a methylation level in the reference genome at the site to a threshold, and
determining the methylation level in the reference genome is above the threshold.

14. The computer-implemented method of any one of claims 1 to 5, wherein the relative abundance comprises a percentage of fragments covering the first set of CpG sites.

15. A computer-implemented computer program comprising a plurality of instructions that when executed cause a computer system to perform the method of any one of the preceding claims.

## Patentansprüche

1. Computerimplementiertes Verfahren zum Analysieren einer biologischen Probe eines Subjekts, die zellfreie DNA beinhaltet, wobei das Verfahren umfasst:
Identifizieren einer ersten Gruppe von CpG-Stellen, die in einem Referenzgenom entweder alle hypomethyliert oder alle hypermethyliert sind;
Empfangen von Sequenz-Reads, die durch Sequenzierung von zellfreien DNA-Fragmenten in der biologischen Probe des Subjekts erhalten wurden;
Abgleichen der Sequenz-Reads mit dem Referenzgenom, um genomische Positionen in dem Referenzgenom zu bestimmen, die den zellfreien DNA-Fragmenten entsprechen;
Bestimmen einer relativen Häufigkeit der zellfreien DNA-Fragmente, die den ersten Satz von CpG-Stellen abdecken, unter Verwendung der abgeglichenen Sequenz-Reads; und
Vergleichen der relativen Häufigkeit mit einem Referenzwert, um das Niveau eines Zustands des Subjekts zu bestimmen, wobei:
das Niveau des Zustands eine Nuklease-Aktivität beinhaltet;
das Niveau des Zustands beinhaltet, ob ein mit einer Nuklease assoziiertes Gen eine genetische Erkrankung bei dem Subjekt aufweist, oder
der Zustand Krebs oder eine Autoimmunerkrankung ist.

2. Computerimplementiertes Verfahren nach Anspruch 1, wobei das Niveau des Zustands darin besteht, ob ein Gen bei dem Subjekt eine genetische Erkrankung aufweist, und wobei das Gen mit einer Nuklease assoziiert ist.

3. Computerimplementiertes Verfahren nach Anspruch 1, wobei der Zustand Krebs ist.

4. Computerimplementiertes Verfahren nach Anspruch 1, wobei der Zustand eine Autoimmunerkrankung ist.

5. Computerimplementiertes Verfahren nach Anspruch 1, wobei das Niveau des Zustands eine Nuklease-Aktivität beinhaltet.

6. Computerimplementiertes Verfahren nach einem der Ansprüche 1 bis 5, wobei die relative Häufigkeit der zellfreien DNA-Fragmente relativ zu einem zweiten Satz von CpG-Stellen steht.

7. Computerimplementiertes Verfahren nach Anspruch 6, wobei die relative Häufigkeit der zellfreien DNA-Fragmente für die zellfreien DNA-Fragmente mit einer spezifizierten Größe bestimmt wird.

8. Computerimplementiertes Verfahren nach Anspruch 7, wobei die spezifizierte Größe ein Größenbereich ist.

9. Computerimplementiertes Verfahren nach einem der Ansprüche 7 bis 8, wobei die relative Häufigkeit eine Enddichte von Sequenz-Reads ist, die den ersten Satz von CpG-Stellen abdecken.

10. Computerimplementiertes Verfahren nach einem der Ansprüche 1 bis 4, wobei die relative Häufigkeit der zellfreien DNA-Fragmente ein statistischer Wert einer Größenverteilung der zellfreien DNA-Fragmente ist, die den ersten Satz von CpG-Stellen abdecken.

11. Computerimplementiertes Verfahren nach Anspruch 10, wobei der statistische Wert ein Größenverhältnis von einer ersten Menge der zellfreien DNA-Fragmente, die den ersten Satz von CpG-Stellen abdecken und eine erste Größe aufweisen, relativ zu einer zweiten Menge der zellfreien DNA-Fragmente, die den ersten Satz von CpG-Stellen abdecken und eine zweite Größe aufweisen.

12. Computerimplementiertes Verfahren nach einem der Ansprüche 1 bis 11, wobei bestimmt wird, dass eine Stelle in dem Referenzgenom hypomethyliert ist, indem:
ein Methylierungsniveau in dem Referenzgenom an der Stelle mit einem Schwellenwert verglichen wird, und
bestimmt wird, dass das Methylierungsniveau in dem Referenzgenom unterhalb des Schwellenwerts liegt.

13. Computerimplementiertes Verfahren nach einem der Ansprüche 1 bis 11, wobei bestimmt wird, dass eine Stelle in dem Referenzgenom hypermethyliert ist, indem:
ein Methylierungsniveau in dem Referenzgenom an der Stelle mit einem Schwellenwert verglichen wird, und
bestimmt wird, dass das Methylierungsniveau in dem Referenzgenom oberhalb des Schwellenwerts liegt.

14. Computerimplementiertes Verfahren nach einem der Ansprüche 1 bis 5, wobei die relative Häufigkeit einen Prozentsatz der Fragmente umfasst, die den ersten Satz von CpG-Stellen abdecken.

15. Computerimplementiertes Computerprogramm, das eine Mehrzahl von Anweisungen umfasst, die bei Ausführung bewirken, dass ein Computersystem das Verfahren nach einem der vorstehenden Ansprüche durchführt.

## Revendications

1. Procédé mis en œuvre par ordinateur pour analyser un échantillon biologique d'un sujet comprenant de l'ADN acellulaire, le procédé comprenant :
l'identification d'un premier ensemble de sites CpG qui sont tous hypométhylés ou tous hyperméthylés dans un génome de référence,
la réception de lectures de séquences obtenues à partir du séquençage de fragments d'ADN acellulaire présents dans l'échantillon biologique du sujet,
l'alignement des lectures de séquences sur le génome de référence afin de déterminer des positions génomiques dans le génome de référence correspondant aux fragments d'ADN acellulaire,
la détermination, au moyen des lectures de séquences alignées, d'une abondance relative des fragments d'ADN acellulaire couvrant le premier ensemble de sites CpG, et
la comparaison de l'abondance relative à une valeur de référence afin de déterminer le niveau d'une affection chez le sujet, étant entendu que :
le niveau de l'affection comprend une activité nucléasique,
le niveau de l'affection est relatif au fait qu'un gène associé à une nucléase présente ou non un trouble génétique chez le sujet, ou
l'affection est un cancer ou une maladie auto-immune.

2. Procédé mis en œuvre par ordinateur selon la revendication 1, dans lequel le niveau de l'affection est relatif au fait qu'un gène présente ou non un trouble génétique chez le sujet, et dans lequel le gène est associé à une nucléase.

3. Procédé mis en œuvre par ordinateur selon la revendication 1, dans lequel l'affection est un cancer.

4. Procédé mis en œuvre par ordinateur selon la revendication 1, dans lequel l'affection est une maladie auto-immune.

5. Procédé mis en œuvre par ordinateur selon la revendication 1, dans lequel le niveau de l'affection comprend une activité nucléasique.

6. Procédé mis en œuvre par ordinateur selon l'une quelconque des revendications 1 à 5, dans lequel l'abondance relative des fragments d'ADN acellulaire est déterminée par rapport à un deuxième ensemble de sites CpG.

7. Procédé mis en œuvre par ordinateur selon la revendication 6, dans lequel l'abondance relative des fragments d'ADN acellulaire est déterminée pour les fragments d'ADN acellulaire présentant une taille spécifiée.

8. Procédé mis en œuvre par ordinateur selon la revendication 7, dans lequel la taille spécifiée correspond à une plage de tailles.

9. Procédé mis en œuvre par ordinateur selon l'une quelconque des revendications 7 et 8, dans lequel l'abondance relative est une densité finale de lectures de séquences couvrant le premier ensemble de sites CpG.

10. Procédé mis en œuvre par ordinateur selon l'une quelconque des revendications 1 à 4, dans lequel l'abondance relative des fragments d'ADN acellulaire est une valeur statistique d'une distribution de tailles des fragments d'ADN acellulaire couvrant le premier ensemble de sites CpG.

11. Procédé mis en œuvre par ordinateur selon la revendication 10, dans lequel la valeur statistique est un rapport de taille entre une première quantité de fragments d'ADN acellulaire couvrant le premier ensemble de sites CpG présentant une première taille et une deuxième quantité de fragments d'ADN acellulaire couvrant le premier ensemble de sites CpG présentant une deuxième taille.

12. Procédé mis en œuvre par ordinateur selon l'une quelconque des revendications 1 à 11, dans lequel un site est déterminé comme étant hypométhylé dans le génome de référence par :
comparaison d'un niveau de méthylation dans le génome de référence au niveau du site à un seuil, et
détermination que le niveau de méthylation dans le génome de référence est inférieur au seuil.

13. Procédé mis en œuvre par ordinateur selon l'une quelconque des revendications 1 à 11, dans lequel un site est déterminé comme étant hyperméthylé dans le génome de référence par :
comparaison d'un niveau de méthylation dans le génome de référence au niveau du site à un seuil, et
détermination que le niveau de méthylation dans le génome de référence est supérieur au seuil.

14. Procédé mis en œuvre par ordinateur selon l'une quelconque des revendications 1 à 5, dans lequel l'abondance relative comprend un pourcentage de fragments couvrant le premier ensemble de sites CpG.

15. Programme informatique mis en œuvre par ordinateur, comprenant une pluralité d'instructions qui, lorsqu'elles sont exécutées, amènent un système informatique à réaliser le procédé selon l'une quelconque des revendications précédentes.
